# EUROPÄISCHE PATENTSCHRIFT

(11) **EP 1 527 052 B1**
(45) Veröffentlichungstag und Bekanntmachung des Hinweises auf die Patenterteilung: **31.05.2006**
(21) Anmeldenummer: 03765096.7
(22) Anmeldetag: 22.07.2003
(51) Int. Cl.: C07D 239/54, C07D 401/12, C07D 403/12, A01N 43/54, A01N 43/84

(54) **3-HETEROCYCLYL-SUBSTITUIERTE BENZOESÄUREDERIVATE**
3-HETEROCYCLYL SUBSTITUTED BENZOIC ACID DERIVATIVES
DERIVES D'ACIDE BENZOIQUE SUBSTITUES PAR UN 3-HETEROCYLYLE

(30) Priorität: 23.07.2002 DE 10233402
(43) Veröffentlichungstag der Anmeldung: 04.05.2005
(73) Patentinhaber: BASF Aktiengesellschaft, 67056 Ludwigshafen (DE)
(72) Erfinder: PUHL, Michael, 68623 Lampertheim (DE); HAMPRECHT, Gerhard, 69469 Weinheim (DE); REINHARD, Robert, 67065 Ludwigshafen (DE); SAGASSER, Ingo, 67125 Dannstadt-Schauernheim (DE); SEITZ, Werner, 68167 Plankstadt (DE); ZAGAR, Cyrill, 68167 Mannheim (DE); WITSCHEL, Matthias, 67098 Bad Dürkheim (DE); LANDES, Andreas, 67354 Römerberg-Heiligenstein (DE)
(74) Vertreter: Reitstötter - Kinzebach
(86) Internationale Anmeldenummer: PCT/EP2003/008013
(87) Internationale Veröffentlichungsnummer: WO 2004/009561

(56) Entgegenhaltungen:
- WO-A-01/83459
- WO-A-92/06962
- WO-A-98/28280
- WO-A-03/029226

## Beschreibung

Die vorliegende Erfindung betrifft 3-Heterocyclyl substituierte Benzoesäure-Derivate und deren landwirtschaftlich brauchbaren Salze, Mittel die derartige Verbindungen enthalten sowie die Verwendung der 3-Heterocyclyl substituierten Benzoesäure-Derivate, ihrer Salze oder Mittel, die diese enthalten, als Herbizide, Desikkantien oder Defoliantien.

Verschiedentlich wurden Uracil substituierte Benzoesäure-Derivate als herbizid wirksame Verbindungen beschrieben. So beschreiben z. B. die WO 88/10254, WO 89/03825 und die WO 91/00278 die Ester von 2-Halogen-5-(substituiertes Uracil)-benzoesäuren und die Ester von 2-Cyano-5-(substituiertes Uracil)-benzoesäuren, die gegebenenfalls in 4-Stellung mit Halogen substituiert sein können. Die WO 89/02891 und die WO 93/06090 beschreiben die Amide von 2-Halogen-5-(substituiertes Uracil)-benzoesäuren und die Amide von 2-Cyano-5-(substituiertes Uracil)-benzoesäuren, die gegebenenfalls in 4-Stellung mit Halogen substituiert sein können, als herbizid wirksame Substanzen. WO 98/28280 beschreibt ebenfalls herbizid wirksame substituierte 3-Sulfonaminophenyl-Uracilverbindungen.

Weiterhin sind aus der WO 01/83459 herbizid wirksame 3-Heterocyclyl substituierte Phenylsulfamoylcarboxamide der Formel A bekannt, 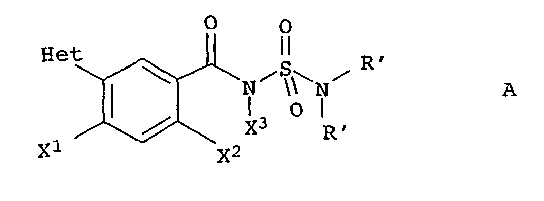 worin Het u.a. für einen ungesättigten, fünf- oder sechsgliedrigen, heterocyclischen Rest, der über ein Stickstoffatom an den Phenylring gebunden ist, steht, X¹ für Wasserstoff, Halogen oder C₁-C₄-Alkyl steht, X² für Wasserstoff, Cyano, Thiocarbamoyl, Halogen, C₁-C₄-Alkyl oder C₁-C₄-Halogenalkyl steht, X³ u. a. für Wasserstoff, C₁-C₆-Alkyl, C₁-C₆-Alkoxyalkyl steht, R' jeweils unabhängig voneinander u. a. für Wasserstoff, Alkoxy, C₁-C₁₀-Alkyl, C₂-C₁₀-Alkenyl, C₃-C₁₀-Alkinyl, C₃-C₇-Cycloalkyl stehen oder die beiden Reste R' bilden gemeinsam mit dem Stickstoff-Atom, an das sie gebunden sind, einen 3- bis 7-gliedrigen heterocyclischen Ring.

WO 92/06962 offenbart herbizid wirksame Arylhaloalkylpyrazol-Verbindungen. WO 03/029226 beschreibt herbizid wirksame heterocyclyl-Substituierte Phenoxyalkyl-, Phenylthioalkyl-, Phenylaminoalkyl- und Phenylalkyl-Sulfamoylcarboxamide.

Der vorliegenden Erfindung liegt die Aufgabe zugrunde, neue herbizid wirksame Verbindungen bereitzustellen, mit denen sich unerwünschte Pflanzen besser als mit den bekannten Herbiziden gezielt bekämpfen lassen. Die neuen Herbizide sollen vorteilhafterweise eine hohe Aktivität gegenüber Schadpflanzen aufweisen. Au-βerdem ist eine hohe Kulturpflanzenverträglichkeit erwünscht. Die Aufgabe erstreckte sich auch auf die Bereitstellung neuer desikkant/defoliant wirksamer Verbindungen.

Es wurde nun überraschenderweise gefunden, dass diese Aufgabe durch 3-Heterocyclyl substituierte Benzoesäure-Derivate der nachstehend definierten allgemeinen Formel I gelöst wird: 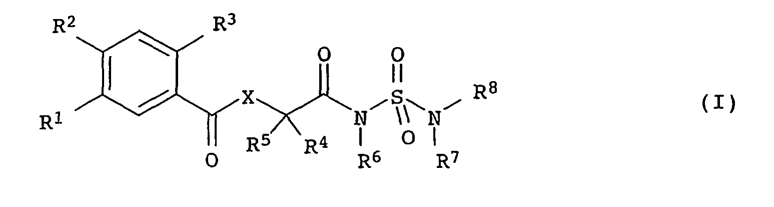 worin die Variablen die folgenden Bedeutungen haben:
- X: Sauerstoff oder NR⁹,
- R¹: heterocylischer Rest der allgemeinen Formeln II-A bis II-H, 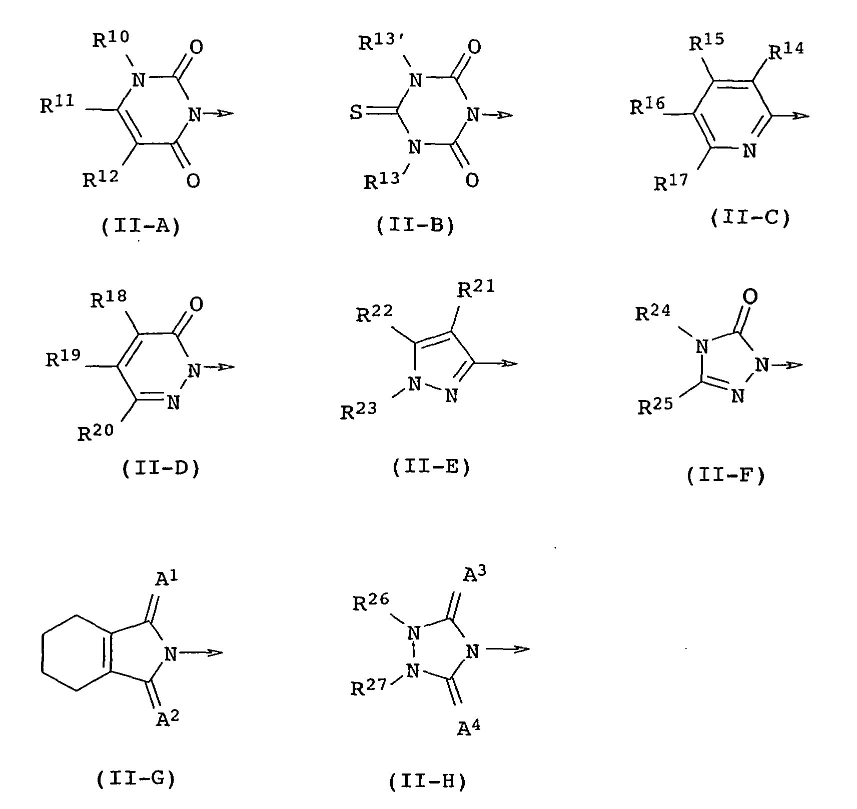
- R²: Wasserstoff oder Halogen,
- R³: Halogen oder Cyano,
- R⁴, R⁵: unabhängig voneinander Wasserstoff, C₁-C₄-Alkyl oder C₁-C₄-Alkoxy, oder R⁴ und R⁵ stehen gemeinsam für eine Gruppe =CH₂,
- R⁶: Wasserstoff, C₁-C₄-Alkyl oder C₁-C₄-Alkoxy,
- R⁷, R⁸: unabhängig voneinander Wasserstoff, C₁-C₆-Alkyl, C₃-C₆-Alkenyl, C₃-C₆-Alkinyl, C₁-C₄-Halogenalkyl, C₁-C₄-Alkoxy-C₁-C₄-alkyl, C₁-C₄-Alkylthio-C₁-C₄-alkyl, C₁-C₄-Alkylsulfinyl-C₁-C₄-alkyl, C₁-C₄-Alkylsulfonyl-C₁-C₄-alkyl, Cyano-C₁-C₄-alkyl, C₁-C₄-Alkoxycarbonyl-C₁-C₄-alkyl, Amino-C₁-C₄-alkyl, C₁-C₄-Alkylamino-C₁-C₄-alkyl, Di(C₁-C₄-alkyl)amino-C₁-C₄-alkyl, Aminocarbonyl-C₁-C₄-alkyl, (C₁-C₄-Alkylamino)carbonyl-C₁-C₄-alkyl, Di(C₁-C₄-alkyl)aminocarbonyl-C₁-C₄-alkyl, Phenyl oder C₁-C₄-Alkylphenyl oder
- R⁷ und R⁸: bilden gemeinsam mit dem Stickstoff-Atom, an das sie gebunden sind, einen gesättigten oder ungesättigten 3-, 4-, 5-, 6- oder 7-gliedrigen Stickstoffheterocyclus, der gegebenenfalls ein oder zwei weitere Heteroatome, ausgewählt unter Stickstoff, Schwefel und Sauerstoff, als Ringglieder enthalten kann, der 1 oder 2 Carbonyl- und/oder Thiocarbonylgruppen als Ringglied enthalten kann, und/oder durch ein, zwei oder drei Substituenten, ausgewählt unter C₁-C₄-Alkyl und Halogen, substituiert sein kann,
- R⁹: Wasserstoff, Hydroxy, C₁-C₄-Alkyl, C₁-C₄-Alkoxy, Phenyl, Phenyl-C₁-C₄-alkyl, C₃-C₆-Alkenyl oder C₃-C₆-Alkinyl,
- R¹⁰: Wasserstoff, C₁-C₄-Alkyl oder Amino,
- R¹¹: C₁-C₄-Alkyl oder C₁-C₄-Halogenalkyl,
- R¹²: Wasserstoff oder C₁-C₄-Alkyl,
- R¹³, R^{13'}: unabhängig voneinander Wasserstoff oder C₁-C₄-Alkyl,
- R¹⁴: Halogen,
- R¹⁵: Wasserstoff oder C₁-C₄-Alkyl,
- R¹⁶: C₁-C₄-Halogenalkyl, C₁-C₄-Alkylthio, C₁-C₄-Alkylsulfonyl oder C₁-C₄-Alkylsulfonyloxy,
- R¹⁷: Wasserstoff oder C₁-C₄-Alkyl,
- R¹⁸: Wasserstoff, C₁-C₄-Alkyl oder Amino,
- R¹⁹: C₁-C₄-Halogenalkyl, C₁-C₄-Alkylthio oder C₁-C₄-Alkylsulfonyl,
- R²⁰: Wasserstoff oder C₁-C₄-Alkyl,
- R²¹: Wasserstoff, Halogen oder C₁-C₄-Alkyl,
- R²²: C₁-C₄-Alkyl, C₁-C₄-Halogenalkyl, C₁-C₄-Halogenalkoxy, C₁-C₄-Alkylthio oder C₁-C₄-Alkylsulfonyl,
- R²³: Wasserstoff oder C₁-C₄-Alkyl,
oder
- R²² und R²³: bilden gemeinsam mit den Atomen, an die sie gebunden sind, einen 5-, 6- oder 7-gliedrigen, gesättigten oder ungesättigten Ring, der ein Heteroatom, das ausgewählt ist unter Sauerstoff und Stickstoff, als ringbildendes Atom enthalten kann, und/oder der durch ein, zwei oder drei Reste, ausgewählt unter C₁-C₄-Alkyl und Halogen, substituiert sein kann,
- R²⁴: Wasserstoff, C₁-C₄-Alkyl oder C₁-C₄-Halogenalkyl,
- R²⁵: C₁-C₄-Alkyl oder C₁-C₄-Halogenalkyl,
oder
- R²⁴ und R²⁵: bilden gemeinsam mit den Atomen, an die sie gebunden sind, einen 5-, 6- oder 7-gliedrigen, gesättigten oder ungesättigten Ring, der gegebenenfalls ein Sauerstoffatom als ringbildendes Atom enthält, und/oder der durch ein, zwei oder drei Reste, ausgewählt unter C₁-C₄-Alkyl und Halogen, substituiert sein kann,
- R²⁶: Wasserstoff, C₁-C₄-Alkyl oder C₁-C₄-Halogenalkyl,
- R²⁷: Wasserstoff, C₁-C₄-Alkyl oder C₁-C₄-Halogenalkyl,
oder
- R²⁶ und R²⁷: bilden gemeinsam mit den Atomen, an die sie gebunden sind, einen 5-, 6- oder 7-gliedrigen, gesättigten oder ungesättigten Ring, der gegebenenfalls ein Sauerstoffatom als ringbildendes Atom enthält, und/oder der durch ein, zwei oder drei Reste, ausgewählt unter C₁-C₄-Alkyl und Halogen, substituiert sein kann,
- A¹, A², A³, A⁴: jeweils unabhängig voneinander Sauerstoff oder Schwefel.

Die vorliegende Erfindung betrifft demnach 3-Heterocyclyl substituierte Benzoesäure-Derivate der allgemeinen Formel I sowie deren landwirtschaftlich brauchbare Salze.

Die Erfindung betrifft auch die Tautomere der Verbindungen I, z. B. solche Verbindungen I, in denen R¹ für einen heterocyclischen Rest der Formel II-A, II-B, II-F oder II-H steht.

Außerdem betrifft die Erfindung
- die Verwendung von Verbindungen I und/oder ihre Salze als Herbizide oder zur Desikkation/Defoliation von Pflanzen
- herbizide Mittel, welche die Verbindungen I und/oder ihre Salze als wirksame Substanzen und mindestens einen inerten flüssigen und/oder festen Trägerstoff sowie gewünschtenfalls mindestens einen oberflächenaktiven Stoff enthalten,
- Mittel zur Desikkation/Defoliation von Pflanzen, welche die Verbindungen I und/oder ihre Salze als wirksame Substanzen und mindestens einen inerten flüssigen und/oder festen Trägerstoff sowie gewünschtenfalls mindestens einen oberflächenaktiven Stoff enthalten, sowie
- Verfahren zur Bekämpfung von unerwünschtem Pflanzenwuchs oder zur Desikkation/Defoliation von Pflanzen mit den Verbindungen I und/oder ihren Salzen, wobei man diese auf Pflanzen bzw. auf deren Lebensraum und/oder auf Saatgut einwirken läβt.

Die Verbindungen der Formel I können je nach Substitutionsmuster ein oder mehrere Chiralitätszentren aufweisen und liegen dann als Enantiomeren- oder Diastereomerengemische vor. Gegenstand der Erfindung sind sowohl die reinen Enantiomere oder Diastereomere als auch deren Gemische.

Unter landwirtschaftlich brauchbaren Salzen kommen vor allem die Salze derjenigen Kationen oder die Säureadditionssalze derjenigen Säuren in Betracht, deren Kationen beziehungsweise Anionen die herbizide Wirkung und/oder desikkante/defoliante Wirkung der Verbindungen I nicht negativ beeinträchtigen. So kommen als Kationen insbesondere die Ionen der Alkalimetalle, vorzugsweise Natrium und Kalium, der Erdalkalimetalle, vorzugsweise Calcium, Magnesium und Barium, und der Übergangsmetalle, vorzugsweise Mangan, Kupfer, Zink und Eisen, sowie das Ammoniumion, das gewünschtenfalls ein bis vier C₁-C₄-Alkylsubstituenten und/oder einen Phenyl- oder Benzylsubstituenten tragen kann, vorzugsweise Diisopropylammonium, Tetramethylammonium, Tetrabutylammonium, Trimethylbenzylammonium, des weiteren Phosphoniumionen, Sulfoniumionen, vorzugsweise Tri(C₁-C₄-alkyl)sulfonium und Sulfoxoniumionen, vorzugsweise Tri(C₁-C₄-alkyl)sulfoxonium, in Betracht.

Anionen von brauchbaren Säureadditionssalzen sind in erster Linie Chlorid, Bromid, Fluorid, Hydrogensulfat, Sulfat, Dihydrogenphosphat, Hydrogenphosphat, Phosphat, Nitrat, Hydrogencarbonat, Carbonat, Hexafluorosilikat, Hexafluorophosphat, Benzoat, sowie die Anionen von C₁-C₄-Alkansäuren, vorzugsweise Formiat, Acetat, Propionat und Butyrat. Sie können durch Reaktion von I mit einer Säure des entsprechenden Anions, vorzugsweise der Chlorwasserstoffsäure, Bromwasserstoffsäure, Schwefelsäure, Phosphorsäure oder Salpetersäure, gebildet werden.

Die bei der Definition der Substituenten R¹ bis R²⁷ oder als Reste an heterocyclischen Ringen genannten organischen Molekülteile stellen - wie die Bedeutung Halogen - Sammelbegriffe für individuelle Aufzählungen der einzelnen Gruppenmitglieder dar. Sämtliche Kohlenstoffketten, also alle Alkyl-, Halogenalkyl-, Cyanoalkyl-, Aminoalkyl-, Aminocarbonylalkyl-, Alkoxy-, Halogenalkoxy-, Alkylthio-, Halogenalkylthio-, Alkylsulfinyl-, Alkylsulfonyl-, Alkylsulfonyloxy-, Alkenyl-Teile können geradkettig oder verzweigt sein. Halogenierte Substituenten tragen vorzugsweise ein bis fünf gleiche oder verschiedene Halogenatome. Die Bedeutung Halogen steht jeweils für Fluor, Chlor, Brom oder Iod.

Ferner stehen beispielsweise:
- C₁-C₄-Alkyl für: z. B. Methyl, Ethyl, Propyl, 1-Methylethyl, Butyl, 1-Methylpropyl, 2-Methylpropyl oder 1,1-Dimethylethyl;
- C₁-C₆-Alkyl für: C₁-C₄-Alkyl, wie voranstehend genannt, sowie z. B. n-Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1-Ethyl-1-methylpropyl oder 1-Ethyl-3-methylpropyl;
- C₁-C₄-Halogenalkyl für: einen C₁-C₄-Alkylrest, wie vorstehend genannt, der partiell oder vollständig durch Fluor, Chlor, Brom und/oder Iod substituiert ist, also z. B. Chlormethyl, Dichlormethyl, Trichlormethyl, Fluormethyl, Difluormethyl, Trifluormethyl, Chlorfluormethyl, Dichlorfluormethyl, Chlordifluormethyl, 2-Fluorethyl, 2-Chlorethyl, 2-Bromethyl, 2-Iodethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl, 2-Fluorpropyl, 3-Fluorpropyl, 2,2-Difluorpropyl, 2,3-Difluorpropyl, 2-Chlorpropyl, 3-Chlorpropyl, 2,3-Dichlorpropyl, 2-Brompropyl, 3-Brompropyl, 3,3,3-Trifluorpropyl, 3,3,3-Trichlorpropyl, 2,2,3,3,3-Pentafluorpropyl, Heptafluorpropyl, 1-(Fluormethyl)-2-fluorethyl, 1-(Chlormethyl)-2-chlorethyl, 1-(Brommethyl)-2-bromethyl, 4-Fluorbutyl, 4-Chlorbutyl, 4-Brombutyl oder Nonafluorbutyl; insbesondere für Difluormethyl, Trifluormethyl;
- C₁-C₄-Alkoxy für: z. B. Methoxy, Ethoxy, n-Propoxy, 1-Methylethoxy, Butoxy, 1-Methylpropoxy, 2-Methylpropoxy oder 1,1-Dimethylethoxy;
- C₁-C₄-Halogenalkoxy für: einen C₁-C₄-Alkoxyrest wie vorstehend genannt, der partiell oder vollständig durch Fluor, Chlor, Brom und/oder Iod substituiert ist, also z.B. OCH₂F, OCHF₂, OCF₃, OCH₂Cl, OCH(Cl)₂, OC(Cl)₃, Chlorfluormethoxy, Dichlorfluormethoxy, Chlordifluormethoxy, 2-Fluorethoxy, 2-Chlorethoxy, 2-Bromethoxy, 2-Iodethoxy, 2,2-Difluorethoxy, 2,2,2-Trifluorethoxy, 2-Chlor-2-fluorethoxy, 2-Chlor-2,2-difluorethoxy, 2,2-Dichlor-2-fluorethoxy, 2,2,2-Trichlorethoxy, OC₂F₅, 2-Fluorpropoxy, 3-Fluorpropoxy, 2,2-Difluorpropoxy, 2,3-Difluorpropoxy, 2-Chlorpropoxy, 3-Chlorpropoxy, 2,3-Dichlorpropoxy, 2-Brompropoxy, 3-Brompropoxy, 3,3,3-Trifluorpropoxy, 3,3,3-Trichlorpropoxy, OCH₂-C₂F₅, OCF₂-C₂F₅, 1-(CH₂F)-2-fluorethoxy, 1-(CH₂Cl)-2-chlorethoxy, 1-(CH₂Br)-2-bromethoxy, 4-Fluorbutoxy, 4-Chlorbutoxy, 4-Brombutoxy oder Nonafluorbutoxy, vorzugsweise für OCHF₂;
- C₁-C₄-Alkoxy-C₁-C₄-alkyl für: durch C₁-C₄-Alkoxy - wie vorstehend genannt - substituiertes C₁-C₄-Alkyl, also z.B. für CH₂-OCH₃, CH₂-OC₂H₅, n-Propoxymethyl, CH₂-OCH(CH₃)₂, n-Butoxymethyl, (1-Methylpropoxy)methyl, (2-Methylpropoxy)methyl, CH₂-OC(CH₃)₃, 2-(Methoxy)ethyl, 2-(Ethoxy)ethyl, 2-(n-Propoxy)ethyl, 2-(1-Methylethoxy)ethyl, 2-(n-Butoxy)ethyl, 2-(1-Methylpropoxy)ethyl, 2-(2-Methylpropoxy)ethyl, 2-(1,1-Dimethylethoxy)ethyl, 2-(Methoxy)propyl, 2-(Ethoxy)propyl, 2-(n-Propoxy)propyl, 2-(1-Methylethoxy)propyl, 2-(n-Butoxy)propyl, 2-(1-Methylpropoxy)propyl, 2-(2-Methylpropoxy)propyl, 2-(1,1-Dimethylethoxy)propyl, 3-(Methoxy)propyl, 3-(Ethoxy)propyl, 3-(n-Propoxy)propyl, 3-(1-Methylethoxy)propyl, 3-(n-Butoxy)propyl, 3-(1-Methylpropoxy)propyl, 3-(2-Methylpropoxy)propyl, 3-(1,1-Dimethylethoxy)propyl, 2-(Methoxy)butyl, 2-(Ethoxy)butyl, 2-(n-Propoxy)butyl, 2-(1-Methylethoxy)butyl, 2-(n-Butoxy)butyl, 2-(1-Methylpropoxy)butyl, 2-(2-Methylpropoxy)butyl, 2-(1,1-Dimethylethoxy)butyl, 3-(Methoxy)butyl, 3-(Ethoxy)butyl, 3-(n-Propoxy)butyl, 3-(1-Methylethoxy)butyl, 3-(n-Butoxy)butyl, 3-(1-Methylpropoxy)butyl, 3-(2-Methylpropoxy)butyl, 3-(1,1-Dimethylethoxy)butyl, 4-(Methoxy)butyl, 4-(Ethoxy)butyl, 4-(n-Propoxy)butyl, 4-(1-Methylethoxy)butyl, 4-(n-Butoxy)butyl, 4-(1-Methylpropoxy)butyl, 4-(2-Methylpropoxy)butyl oder 4-(1,1-Dimethylethoxy)butyl;
- C₁-C₄-Alkylthio für: z. B. SCH₃, SC₂H₅, SCH₂-C₂H₅, SCH(CH₃)₂, n-Butylthio, SCH(CH₃)-C₂H₅, SCH₂-CH(CH₃)₂ oder SC(CH₃)₃;
- C₁-C₄-Alkylthio-C₁-C₄-alkyl für: durch C₁-C₄-Alkylthio - wie vorstehend genannt - substituiertes C₁-C₄-Alkyl, also z.B. für CH₂-SCH₃, CH₂-SC₂H₅, n-Propylthiomethyl, CH₂-SCH(CH₃)₂, n-Butylthiomethyl, (1-Methylpropylthio)methyl, (2-Methylpropylthio)methyl, CH₂-SC(CH₃)₃, 2-(Methylthio)ethyl, 2-(Ethylthio)ethyl, 2-(n-Propylthio)ethyl, 2-(1-Methylethylthio)ethyl, 2-(n-Butylthio)ethyl, 2-(1-Methylpropylthio)ethyl, 2-(2-Methylpropylthio)ethyl, 2-(1,1-Dimethylethylthio)ethyl, 2-(Methylthio)propyl, 2-(Ethylthio)propyl, 2-(n-Propylthio)propyl, 2-(1-Methylethylthio)propyl, 2-(n-Butylthio)propyl, 2-(1-Methylpropylthio)propyl, 2-(2-Methylpropylthio)propyl, 2-(1,1-Dimethylethylthio)propyl, 3-(Methylthio)propyl, 3-(Ethylthio)propyl, 3-(n-Propylthio)propyl, 3-(1-Methylethylthio)propyl, 3-(n-Butylthio)propyl, 3-(1-Methylpropylthio)propyl, 3-(2-Methylpropylthio)propyl, 3-(1,1-Dimethylethylthio)propyl, 2-(Methylthio)butyl, 2-(Ethylthio)butyl, 2-(n-Propylthio)butyl, 2-(1-Methylethylthio)butyl, 2-(n-Butylthio)butyl, 2-(1-Methylpropylthio)butyl, 2-(2-Methylpropylthio)butyl, 2-(1,1-Dimethylethylthio)butyl, 3-(Methylthio)butyl, 3-(Ethylthio)butyl, 3-(n-Propylthio)butyl, 3-(1-Methylethylthio)butyl, 3-(n-Butylthio)butyl, 3-(1-Methylpropylthio)butyl, 3-(2-Methylpropylthio)butyl, 3-(1,1-Dimethylethylthio)butyl, 4-(Methylthio)butyl, 4-(Ethylthio)butyl, 4-(n-Propylthio)butyl, 4-(1-Methylethylthio)butyl, 4-(n-Butylthio)butyl, 4-(1-Methylpropylthio)butyl, 4-(2-Methylpropylthio)butyl oder 4-(1,1-Dimethylethylthio)butyl;
- C₁-C₄ Alkylsulfinyl (C₁-C₄-Alkyl-S(=O)-) sowie die Alkylsulfinylteile von C₁-C₄-Alkylsulfinyl-C₁-C₄-Alkyl für: z.B. Methylsulfinyl, Ethylsulfinyl, Propylsulfinyl, 1-Methylethylsulfinyl, Butylsulfinyl, 1-Methylpropylsulfinyl, 2-Methylpropylsulfinyl oder 1,1-Dimethylethylsulfinyl;
- C₁-C₄-Alkylsulfonyl (C₁-C₄-Alkyl-S(=O)₂-) sowie die Alkylsulfonylteile von C₁-C₄-Alkylsulfonyl-C₁-C₄-Alkyl für: z. B. Methylsulfonyl, Ethylsulfonyl, Propylsulfonyl, 1-Methylethylsulfonyl, Butylsulfonyl, 1-Methylpropylsulfonyl, 2-Methylpropylsulfonyl oder 1,1-Dimethylethylsulfonyl, vorzugsweise Methylsulfonyl;
- C₁-C₄-Alkylsulfonyloxy für: z. B. Methylsulfonyloxy, Ethylsulfonyloxy, n-Propylsulfonyloxy, 1-Methylethylsulfonyloxy, Butylsulfonyloxy, 1-Methylpropylsulfonyloxy, 2-Methylpropylsulfonyloxy oder 1,1-Dimethylethylsulfonyloxy, vorzugsweise Methylsulfonyloxy;
- Cyano-C₁-C₄-alkyl für: z. B. CH₂CN, 1-Cyanoethyl, 2-Cyanoethyl, 1-Cyanoprop-1-yl, 2-Cyanoprop-1-yl, 3-Cyanoprop-1-yl, 1-Cyanobut-1-yl, 2-Cyanobut-1-yl, 3-Cyanobut-1-yl, 4-Cyanobut-1-yl, 1-Cyanobut-2-yl, 2-Cyanobut-2-yl, 3-Cyanobut-2-yl, 4-Cyanobut-2-yl, 1-(CH₂CN)eth-1-yl, 1-(CH₂CN)-1-(CH₃)eth-1-yl oder 1-(CH₂CN)prop-1-yl;
- Phenyl-C₁-C₄-alkyl für: z. B. Benzyl, 1-Phenylethyl, 2-Phenylethyl, 1-Phenylprop-1-yl, 2-Phenylprop-1-yl, 3-Phenylprop-1-yl, 1-Phenylbut-1-yl, 2-Phenylbut-1-yl, 3-Phenylbut-1-yl, 4-Phenylbut-1-yl, 1-Phenylbut-2-yl, 2-Phenylbut-2-yl, 3-Phenylbut-2-yl, 4-Phenylbut-2-yl, 1-(Benzyl)eth-1-yl, 1-(Benzyl)-1-(methyl)eth-1-yl oder 1-(Benzyl)prop-1-yl;
- C₁-C₄ Alkoxycarbonyl für: z.B. Methoxycarbonyl, Ethoxycarbonyl, Propoxycarbonyl, 1-Methylethoxycarbonyl, Butoxycarbonyl, 1-Methylpropoxycarbonyl, 2-Methylpropoxycarbonyl oder 1,1-Dimethylethoxycarbonyl;
- (C₁-C₄-Alkoxy)carbonyl-C₁-C₄-alkyl für: durch (C₁-C₄-Alkoxy)-carbonyl - wie vorstehend genannt - substituiertes C₁-C₄-Alkyl, also z.B. für CH₂-CO-OCH₃, CH₂-CO-OC₂H₅, CH₂-CO-OCH₂-C₂H₅, CH₂-CO-OCH(CH₃)₂, n-Butoxycarbonylmethyl, CH₂-CO-OCH(CH₃)-C₂H₅, CH₂-CO-OCH₂-CH(CH₃)₂, CH₂-CO-OC(CH₃)₃, 1-(CO-OCH₃)ethyl, 1-(CO-OC₂H₅)ethyl, 1-(CO-OCH₂-C₂H₅)ethyl, 1-[CH(CH₃)₂]ethyl, 1-(n-Butoxycarbonyl)ethyl, 1-[1-Methylpropoxycarbonyl]ethyl, 1-[2-Methylpropoxycarbonyl]ethyl, 2-(CO-OCH₃)ethyl, 2-(CO-OC₂H₅)ethyl, 2-(CO-OCH₂-C₂H₅)ethyl, 2-[CO-OCH(CH₃)₂]ethyl, 2-(n-Butoxycarbonyl)ethyl, 2-[1-Methylpropoxycarbonyl]ethyl, 2-[2-Methylpropoxycarbonyl]ethyl, 2-[CO-OC(CH₃)₃]ethyl, 2-(CO-OCH₃)propyl, 2-(CO-OC₂H₅)propyl, 2-(CO-OCH₂-C₂H₅)propyl, 2-[CO-OCH(CH₃)₂]propyl, 2-(n-Butoxycarbonyl)propyl, 2-[1-Methylpropoxycarbonyl]propyl, 2-[2-Methylpropoxycarbonyl]propyl, 2-[CO-OC(CH₃)₃]propyl, 3-(CO-OCH₃)propyl, 3-(CO-OC₂H₅)propyl, 3-(CO-OCH₂-C₂H₅)propyl, 3-[CO-OCH(CH₃)₂]propyl, 3-(n-Butoxycarbonyl)propyl, 3-[1-Methylpropoxycarbonyl]propyl, 3-[2-Methylpropoxycarbonyl]propyl, 3-[CO-OC(CH₃)₃]propyl, 2-(CO-OCH₃)butyl, 2-(CO-OC₂H₅)butyl, 2-(CO-OCH₂-C₂H₅)butyl, 2-[CO-OCH(CH₃)₂]butyl, 2-(n-Butoxycarbonyl)butyl, 2-[1-Methylpropoxycarbonyl]butyl, 2-[2-Methylpropoxycarbonyl]butyl, 2-[CO-OC(CH₃)₃]butyl, 3-(CO-OCH₃)butyl, 3-(CO-OC₂H₅)butyl, 3-(CO-OCH₂-C₂H₅)butyl, 3-[CO-OCH(CH₃)₂]butyl, 3-(n-Butoxycarbonyl)butyl, 3-[1-Methylpropoxycarbonyl]butyl, 3-[2-Methylpropoxycarbonyl]butyl, 3-[CO-OC(CH₃)₃]butyl, 4-(CO-OCH₃)butyl, 4-(CO-OC₂H₅)butyl, 4-(CO-OCH₂-C₂H₅)butyl, 4-[CO-OCH(CH₃)₂]butyl, 4-(n-Butoxycarbonyl)butyl, 4-[1-Methylpropoxycarbonyl]butyl, 4-[2-Methylpropoxycarbonyl]butyl oder 4-[CO-OC(CH₃)₃]butyl, vorzugsweise für CH₂-CO-OCH₃, CH₂-CO-OC₂H₅, 1-(CO-OCH₃)ethyl oder 1-(CO-OC₂H₅)ethyl;
- Amino-C₁-C₄-alkyl für: z. B. CH₂NH₂, 1-Aminoethyl, 2-Aminoethyl, 1-Aminoprop-1-yl, 2-Aminoprop-1-yl, 3-Aminoprop-1-yl, 1-Aminobut-1-yl, 2-Aminobut-1-yl, 3-Aminobut-1-yl, 4-Aminobut-1-yl, 1-Aminobut-2-yl, 2-Aminobut-2-yl, 3-Aminobut-2-yl, 4-Aminobut-2-yl, 1-(CH₂NH₂)eth-1-yl, 1-(CH₂NH₂)-1-(CH₃)eth-1-yl oder 1-(CH₂NH₂)prop-1-yl;
- C₁-C₄-Alkylamino für: z. B. H₃C-NH-, H₅C₂-NH-, n-Propyl-NH-, 1-Methylethyl-NH-, n-Butyl-NH-, 1-Methylpropyl-NH-, 2-Methylpropyl-NH- oder 1,1-Dimethylethyl-NH-;
- C₁-C₄-Alkylamino-C₁-C₄-alkyl für: durch C₁-C₄-Alkylamino wie vorstehend definiert, substituiertes C₁-C₄-Alkyl, also beispielsweise für CH₂CH₂-NH-CH₃, CH₂CH₂-N(CH₃)₂, CH₂CH₂-NH-C₂H₅ oder CH₂CH₂-N(C₂H₅)₂;
- Di(C₁-C₄-alkyl)amino für: N(CH₃)₂, N(C₂H₅)₂, N,N-Dipropylamino, N,N-Di-(1-methylethyl)amino, N,N-Dibutylamino, N,N-Di-(1-methylpropyl)amino, N,N-Di-(2-methylpropyl)amino, N,N-Di-(1,1-dimethylethyl)amino, N-Ethyl-N-methylamino, N-Methyl-N-propylamino, N-Methyl-N-(1-methylethyl)amino, N-Butyl-N-methylamino, N-Methyl-N-(1-methylpropyl)amino, N-Methyl-N-(2-methylpropyl)amino, N-(1,1-Dimethylethyl)-N-methylamino, N-Ethyl-N-propylamino, N-Ethyl-N-(1-methylethyl)amino, N-Butyl-N-ethylamino, N-Ethyl-N-(1-methylpropyl)amino, N-Ethyl-N-(2-methylpropyl)amino, N-Ethyl-N-(1,1-dimethylethyl)amino, N-(1-Methylethyl)-N-propylamino, N-Butyl-N-propylamino, N-(1-Methylpropyl)-N-propylamino, N-(2-Methylpropyl)-N-propylamino, N-(1,1-Dimethylethyl)-N-propylamino, N-Butyl-N-(1-methylethyl)amino, N-(1-Methylethyl)-N-(1-methylpropyl)amino, N-(1-Methylethyl)-N-(2-methylpropyl)amino, N-(1,1-Dimethylethyl)-N-(1-methylethyl)amino, N-Butyl-N-(1-methylpropyl)amino, N-Butyl-N-(2-methylpropyl)amino, N-Butyl-N-(1,1-dimethylethyl)-amino, N-(1-Methylpropyl)-N-(2-methylpropyl)amino, N-(1,1-Dimethylethyl)-N-(1-methylpropyl)amino oder N-(1,1-Dimethylethyl)-N-(2-methylpropyl)amino;
- Di(C₁-C₄-alkyl)amino-C₁-C₄-alkyl für: durch Di(C₁-C₄-alkyl)amino wie vorstehend genannt substituiertes C₁-C₄-Alkyl, also z.B. für CH₂N(CH₃)₂, CH₂N(C₂H₅)₂, N,N-Dipropylaminomethyl, N,N-Di[CH(CH₃)₂]aminomethyl, N,N-Dibutylaminomethyl, N,N-Di-(1-methylpropyl)aminomethyl, N,N-Di(2-methylpropyl)aminomethyl, N,N-Di[C(CH₃)₃]aminomethyl, N-Ethyl-N-methylaminomethyl, N-Methyl-N-propylaminomethyl, N-Methyl-N-[CH(CH₃)₂]aminomethyl, N-Butyl-N-methylaminomethyl, N-Methyl-N-(1-methylpropyl)aminomethyl, N-Methyl-N-(2-methylpropyl)aminomethyl, N-[C(CH₃)₃]-N-methylaminomethyl, N-Ethyl-N-propylaminomethyl, N-Ethyl-N-[CH(CH₃)₂]aminomethyl, N-Butyl-N-ethylaminomethyl, N-Ethyl-N-(1-methylpropyl)aminomethyl, N-Ethyl-N-(2-methylpropyl)aminomethyl, N-Ethyl-N-[C(CH₃)₃]aminomethyl, N-[CH(CH₃)₂]-N-propylaminomethyl, N-Butyl-N-propylaminomethyl, N-(1-Methylpropyl)-N-propylaminomethyl, N-(2-Methylpropyl)-N-propylaminomethyl, N-[C(CH₃)₃]-N-propylaminomethyl, N-Butyl-N-(1-methylethyl)-aminomethyl, N-[CH(CH₃)₂]-N-(1-methylpropyl)aminomethyl, N-[CH(CH₃)₂]-N-(2-methylpropyl)aminomethyl, N-[C(CH₃)₃]-N-[CH(CH₃)₂]aminomethyl, N-Butyl-N-(1-methylpropyl)aminomethyl, N-Butyl-N-(2-methylpropyl)aminomethyl, N-Butyl-N-[C(CH₃)₃]aminomethyl, N-(1-Methylpropyl)-N-(2-methylpropyl)aminomethyl, N-[C(CH₃)₃]-N-(1-methylpropyl)aminomethyl, N-[C(CH₃)₃]-N-(2-methylpropyl)aminomethyl, N,N-Dimethylaminoethyl, N,N-Diethylaminoethyl, N,N-Di(n-propyl)aminoethyl, N,N-Di-[CH(CH₃)₂]-aminoethyl, N,N-Dibutylaminoethyl, N,N-Di(1-methylpropyl)aminoethyl, N,N-Di(2-methylpropyl)aminoethyl, N,N-Di-[C(CH₃)₃]aminoethyl, N-Ethyl-N-methylaminoethyl, N-Methyl-N-propylaminoethyl, N-Methyl-N-[CH(CH₃)₂]aminoethyl, N-Butyl-N-methylaminoethyl, N-Methyl-N-(1-methylpropyl)aminoethyl, N-Methyl-N-(2-methylpropyl)aminoethyl, N-[C(CH₃)₃]-N-methylaminoethyl, N-Ethyl-N-propylaminoethyl, N-Ethyl-N-[CH(CH₃)_{2]}aminoethyl, N-Butyl-N-ethylaminoethyl, N-Ethyl-N-(1-methylpropyl)aminoethyl, N-Ethyl-N-(2-methylpropyl)aminoethyl, N-Ethyl-N-[C(CH₃)₃]aminoethyl, N-[CH(CH₃)₂]-N-propylaminoethyl, N-Butyl-N-propylaminoethyl, N-(1-Methylpropyl)-N-propylaminoethyl, N-(2-Methylpropyl)-N-propylaminoethyl, N-[C(CH₃)₃]-N-propylaminoethyl, N-Butyl-N-[CH(CH₃)₂]aminoethyl, N-[CH(CH₃)₂]-N-(1-methylpropyl)aminoethyl, N-[CH(CH₃)₂]-N-(2-methylpropyl)aminoethyl, N-[C(CH₃)₃]-N-[CH(CH₃)₂]aminoethyl, N-Butyl-N-(1-methylpropyl)aminoethyl, N-Butyl-N-(2-methylpropyl)aminoethyl, N-Butyl-N-[C(CH₃)₃]aminoethyl, N-(1-Methylpropyl)-N-(2-methylpropyl)aminoethyl, N-[C(CH₃)₃]-N-(1-methylpropyl)aminoethyl oder N-[C(CH₃)₃]-N-(2-methylpropyl)aminoethyl;
- Aminocarbonyl-C₁-C₄-alkyl für: z. B. CH₂CONH₂, 1-(CONH₂)ethyl, 2-(CONH₂)ethyl, 1-(CONH₂)prop-1-yl, 2-(CONH₂)prop-1-yl, 3-(CONH₂)prop-1-yl, 1-(CONH₂)but-1-yl, 2-(CONH₂)but-1-yl, 3-(CONH₂)but-1-yl, 4-(CONH₂)but-1-yl, 1-(CONH₂)but-2-yl, 2-(CONH₂)but-2-yl, 3-(CONH₂)but-2-yl, 4-(CONH₂)but-2-yl, 1-(CH₂CONH₂)eth-1-yl, 1-(CH₂CONH₂)-1-(CH₃)-eth-1-yl oder 1-(CH₂CONH₂)prop-1-yl;
- (C₁-C₄-Alkylamino)carbonyl-C₁-C₄-alkyl für: durch (C₁-C₄-Alkylamino)carbonyl wie vorstehend genannt, substituiertes C₁-C₄-Alkyl, also z.B. für CH₂-CO-NH-CH₃, CH₂-CO-NH-C₂H₅, CH₂-CO-NH-CH₂-C₂H₅, CH₂-CO-NH-CH(CH₃)₂, CH₂-CO-NH-CH₂CH₂-C₂H₅, CH₂-CO-NH-CH(CH₃)-C₂H₅, CH₂-CO-NH-CH₂-CH(CH₃)₂, CH₂-CO-NH-C(CH₃)₃, CH(CH₃)-CO-NH-CH₃, CH(CH₃)-CO-NH-C₂H₅, 2-(CO-NH-CH₃)ethyl, 2-(CO-NH-C₂H₅)ethyl, 2-(CO-NH-CH₂-C₂H₅)ethyl, 2-[CH₂-CO-NH-CH(CH₃)₂]ethyl, 2-(CO-NH-CH₂CH₂-C₂H₅)ethyl, 2-[CO-NH-CH(CH₃)-C₂H₅]ethyl, 2-[CO-NH-CH₂-CH(CH₃)₂]ethyl, 2-[CO-NH-C(CH₃)₃]ethyl, 2-(CO-NH-CH₃)propyl, 2-(CO-NH-C₂H₅)propyl, 2-(CO-NH-CH₂-C₂H₅)propyl, 2-[CH₂-CO-NH-CH(CH₃)₂]propyl, 2-(CO-NH-CH₂CH₂-C₂H₅)propyl, 2-[CO-NH-CH(CH₃)-C₂H₅]propyl, 2-[CO-NH-CH₂-CH(CH₃)₂]propyl, 2-[CO-NH-C(CH₃)₃]propyl, 3-(CO-NH-CH₃)propyl, 3-(CO-NH-C₂H₅)propyl, 3-(CO-NH-CH₂-C₂H₅)propyl, 3-[CH₂-CO-NH-CH(CH₃)₂]propyl, 3-(CO-NH-CH₂CH₂-C₂H₅)propyl, 3-[CO-NH-CH(CH₃)-C₂H₅]propyl, 3-[CO-NH-CH₂-CH(CH₃)₂]propyl, 3-[CO-NH-C(CH₃)₃]propyl, 2-(CO-NH-CH₃)butyl, 2-(CO-NH-C₂H₅)butyl, 2-(CO-NH-CH₂-C₂H₅)butyl, 2-[CH₂-CO-NH-CH(CH₃)₂]butyl, 2-(CO-NH-CH₂CH₂-C₂H₅)butyl, 2-[CO-NH-CH(CH₃)-C₂H₅]butyl, 2-[CO-NH-CH₂-CH(CH₃)₂]butyl, 2-[CO-NH-C(CH₃)₃]butyl, 3-(CO-NH-CH₃)butyl, 3-(CO-NH-C₂H₅)butyl, 3-(CO-NH-CH₂-C₂H₅)butyl, 3-[CH₂-CO-NH-CH(CH₃)₂]butyl, 3-(CO-NE-CH₂CH₂-C₂H₅)butyl, 3-[CO-NH-CH(CH₃)-C₂H₅]butyl, 3-[CO-NH-CH₂-CH(CH₃)₂]butyl, 3-[CO-NR-C(CH₃)₃]butyl, 4-(CO-NH-CH₃)butyl, 4-(CO-NH-C₂H₅)butyl, 4-(CO-NH-CH₂-C₂H₅)butyl, 4-[CH₂-CO-NH-CH(CH₃)₂]butyl, 4-(CO-NH-CH₂CH₂-C₂H₅)butyl, 4-[CO-NH-CH(CH₃)-C₂H₅]butyl, 4-[CO-NH-CH₂-CH(CH₃)₂]butyl oder 4-[CO-NE-C(CH₃)₃]butyl;
- Di-(C₁-C₄-alkyl)aminocarbonyl-C₁-C₄-alkyl für: durch Di(C₁-C₄-alkyl)aminocarbonyl - wie vorstehend genannt -substituiertes C₁-C₄-Alkyl, also z.B. Di-(C₁-C₄-alkyl)aminocarbonylmethyl, 1- oder 2-Di-(C₁-C₄-alkyl)aminocarbonylethyl, 1-, 2- oder 3-Di-(C₁-C₄-alkyl)aminocarbonylpropyl;
- C₁-C₄-Alkylphenyl für: durch C₁-C₄-Alkyl - wie vorstehend genannt - substituiertes Phenyl, also z. B. für 2-Tolyl, 3-Tolyl, 4-Tolyl, 2-Ethylphenyl, 3-Ethylphenyl, 4-Ethylphenyl, 2-(n-Propyl)phenyl, 3-(n-Propyl)phenyl, 4-(n-Propyl)phenyl, 2-(1-Methylethyl)phenyl, 3-(1-Methylethyl)phenyl, 4-(1-Methylethyl)phenyl, 2-(n-Butyl)phenyl, 3-(n-Butyl)phenyl, 4-(n-Butyl)phenyl, 2-(1-Methylpropyl)phenyl, 3-(1-Methylpropyl)phenyl, 4-(1-Methylpropyl)phenyl, 2-(2-Methylpropyl)phenyl, 3-(2-Methylpropyl)phenyl, 4-(2-Methylpropyl)phenyl, 2-(1,1-Dimethyl)phenyl, 3-(1,1-Dimethyl)phenyl, 4-(1,1-Dimethyl)phenyl;
- C₃-C₆-Alkenyl für: einen einfach ungesättigten aliphatischen Kohlenwasserstoffrest mit 3 bis 6 Kohlenstoffatomen, der vorzugsweise nicht über ein olefinisches C-Atom gebunden ist, z. B. Prop-1-en-1-yl, Prop-2-en-1-yl, 1-Methylethenyl, Buten-1-yl, Buten-2-yl, Buten-3-yl, 1-Methyl-prop-1-en-1-yl, 2-Methyl-prop-1-en-1-yl, 1-Methyl-prop-2-en-1-yl, 2-Methyl-prop-2-en-1-yl, Penten-1-yl, Penten-2-yl, Penten-3-yl, Penten-4-yl, 1-Methyl-but-1-en-1-yl, 2-Methyl-but-1-en-1-yl, 3-Methyl-but-1-en-1-yl, 1-Methyl-but-2-en-1-yl, 2-Methyl-but-2-en-1-yl, 3-Methyl-but-2-en-1-yl, 1-Methyl-but-3-en-1-yl, 2-Methyl-but-3-en-1-yl, 3-Methyl-but-3-en-1-yl, 1,1-Dimethyl-prop-2-en-1-yl, 1,2-Dimethyl-prop-1-en-1-yl, 1,2-Dimethyl-prop-2-en-1-yl, 1-Ethyl-prop-1-en-2-yl, 1-Ethyl-prop-2-en-1-yl, Hex-1-en-1-yl, Hex-2-en-1-yl, Hex-3-en-1-yl, Hex-4-en-1-yl, Hex-5-en-1-yl, 1-Methyl-pent-1-en-1-yl, 2-Methyl-pent-1-en-1-yl, 3-Methyl-pent-1-en-1-yl, 4-Methyl-pent-1-en-1-yl, 1-Methyl-pent-2-en-1-yl, 2-Methyl-pent-2-en-1-yl, 3-Methyl-pent-2-en-1-yl, 4-Methyl-pent-2-en-1-yl, 1-Methyl-pent-3-en-1-yl, 2-Methyl-pent-3-en-1-yl, 3-Methyl-pent-3-en-1-yl, 4-Methyl-pent-3-en-1-yl, 1-Methyl-pent-4-en-1-yl, 2-Methyl-pent-4-en-1-yl, 3-Methyl-pent-4-en-1-yl, 4-Methyl-pent-4-en-1-yl, 1,1-Dimethyl-but-2-en-1-yl, 1,1-Dimethyl-but-3-en-1-yl, 1,2-Dimethyl-but-1-en-1-yl, 1,2-Dimethyl-but-2-en-1-yl, 1,2-Dimethyl-but-3-en-1-yl, 1,3-Dimethyl-but-1-en-1-yl, 1,3-Dimethyl-but-2-en-1-yl, 1,3-Dimethyl-but-3-en-1-yl, 2,2-Dimethyl-but-3-en-1-yl, 2,3-Dimethyl-but-1-en-1-yl, 2,3-Dimethyl-but-2-en-1-yl, 2,3-Dimethyl-but-3-en-1-yl, 3,3-Dimethyl-but-1-en-1-yl, 3,3-Dimethyl-but-2-en-1-yl, 1-Ethyl-but-1-en-1-yl, 1-Ethyl-but-2-en-1-yl, 1-Ethyl-but-3-en-1-yl, 2-Ethyl-but-1-en-1-yl, 2-Ethyl-but-2-en-1-yl, 2-Ethyl-but-3-en-1-yl, 1,1,2-Trimethyl-prop-2-en-1-yl, 1-Ethyl-1-methyl-prop-2-en-1-yl, 1-Ethyl-2-methyl-prop-1-en-1-yl oder 1-Ethyl-2-methyl-prop-2-en-1-yl;
- C₃-C₆-Alkinyl für: einen eine Dreifachbindung enthaltenden aliphatischen Kohlenwasserstoffrest mit 3 bis 6 Kohlenstoffatomen, der vorzugsweise nicht über ein C-Atom der Dreifachbindung gebunden ist, z. B. Propargyl (2-Propinyl), 1-Propinyl, But-1-in-3-yl, But-1-in-4-yl, But-2-in-1-yl, Pent-1-in-3-yl, Pent-1-in-4-yl, Pent-1-in-5-yl, Pent-2-in-1-yl, Pent-2-in-4-yl, Pent-2-in-5-yl, 3-Methyl-but-1-in-3-yl, 3-Methyl-but-1-in-4-yl, Hex-1-in-3-yl, Hex-1-in-4-yl, Hex-1-in-5-yl, Hex-1-in-6-yl, Hex-2-in-1-yl, Hex-2-in-4-yl, Hex-2-in-5-yl, Hex-2-in-6-yl, Hex-3-in-1-yl, Hex-3-in-2-yl, 3-Methyl-pent-1-in-3-yl, 3-Methyl-pent-1-in-4-yl, 3-Methyl-pent-1-in-5-yl, 4-Methyl-pent-2-in-4-yl oder 4-Methyl-pent-2-in-5-yl.

Im Hinblick auf die Verwendung der erfindungsgemäßen Verbindungen der Formel I als Herbizide und/oder als desikkant/defoliant wirksame Verbindungen haben die Variablen X, R² bis R⁶ vorzugsweise die folgenden Bedeutungen, und zwar unabhängig voneinander und insbesondere in Kombination:
- X: Sauerstoff,
- R²: Wasserstoff, Fluor oder Chlor,
- R³: Chlor oder Cyano, insbesondere Chlor,
- R⁴, R⁵: unabhängig voneinander Wasserstoff oder C₁-C₄-Alkyl, insbesondere Wasserstoff oder Methyl,
- R⁶: Wasserstoff oder C₁-C₄-Alkyl, insbesondere Wasserstoff oder Methyl, besondess bevorzugt Wasserstoff.

In einer bevorzugten Ausführungsform der Erfindung steht R⁴ oder R⁵ für Wasserstoff und der andere Rest R⁴ oder R⁵ für C₁-C₄-Alkyl, insbesondere Methyl oder R⁴, R⁵ stehen jeweils für Methyl.

Ganz besonders bevorzugt sind die Verbindungen der Formel I, worin die Variablen
- R²: für Wasserstoff, Chlor oder Fluor,
- R³: für Chlor oder Cyano,
- R⁶: für Wasserstoff und
- X: für Sauerstoff
stehen.

Folgende Ausführungsformen der 3-Heterocyclyl substituierten Benzoesäure-Derivate der allgemeinen Formel I sind hervorzuheben:
1. In einer besonders bevorzugten Ausführungsform steht R¹ in Formel I für einen heterocyclischen Rest der Formel II-A. Derartige Verbindungen werden im Folgenden auch als Verbindungen I-A bezeichnet. In den Verbindungen I-A haben X, R² bis R⁸ vorzugsweise die als bevorzugt und insbesondere die als besonders bevorzugt angegebenen Bedeutungen. Insbesondere stehen in Formel II-A:
   - R¹⁰: für C₁-C₄-Alkyl oder Amino, insbesondere für Methyl oder Amino,
   - R¹¹: für C₁-C₄-Halogenalkyl, insbesondere für Trifluormethyl, und
   - R¹²: für Wasserstoff
2. In einer weiteren bevorzugten Ausführungsform steht R¹ in Formel I für einen heterocyclischen Rest der Formel II-B. Derartige Verbindungen werden im Folgenden auch als Verbindungen I-B bezeichnet. In den Verbindungen I-B haben X, R² bis R⁸ vorzugsweise die als bevorzugt und insbesondere die als besonders bevorzugt angegebenen Bedeutungen. Insbesondere stehen in Formel II-B
   - R¹³, R^{13'}: jeweils unabhängig voneinander für C₁-C₄-Alkyl, insbesondere für Methyl.
3. In einer weiteren bevorzugten Ausführungsform steht R¹ in Formel I für einen heterocyclischen Rest der Formel II-C. Derartige Verbindungen werden im Folgenden auch als Verbindungen I-C bezeichnet. In den Verbindungen I-C haben X, R² bis R⁸ vorzugsweise die als bevorzugt und insbesondere die als besonders bevorzugt angegebenen Bedeutungen. Insbesondere stehen in Formel II-C:
   - R¹⁴: für Fluor oder Chlor, insbesondere für Chlor,
   - R¹⁵: für Wasserstoff oder C₁-C₄-Alkyl, insbesondere für Wasserstoff,
   - R¹⁶: für C₁-C₄-Halogenalkyl, C₁-C₄-Alkylsulfonyl oder C₁-C₄-Alkylsulfonyloxy, insbesondere für Trifluormethyl, Methylsulfonyl oder Methylsulfonyloxy.
4. In einer weiteren bevorzugten Ausführungsform steht R¹ in Formel I für einen heterocyclischen Rest der Formel II-D. Derartige Verbindungen werden im Folgenden auch als Verbindungen I-D bezeichnet. In den Verbindungen I-D haben X, R² bis R⁸ vorzugsweise die als bevorzugt und insbesondere die als besonders bevorzugt angegebenen Bedeutungen. Insbesondere stehen in Formel II-D:
   - R¹⁸: für Wasserstoff, Methyl oder Amino,
   - R¹⁹: für C₁-C₄-Halogenalkyl oder C₁-C₄-Alkylsulfonyl, insbesondere für Trifluormethyl oder Methylsulfonyl,
   - R²⁰: für Wasserstoff.
5. In einer weiteren bevorzugten Ausführungsform steht R¹ in Formel I für einen heterocyclischen Rest der Formel II-E. Derartige Verbindungen werden im Folgenden auch als Verbindungen I-E bezeichnet. In den Verbindungen I-E haben X, R² bis R⁸ vorzugsweise die als bevorzugt und insbesondere die als besonders bevorzugt angegebenen Bedeutungen. Insbesondere stehen in Formel II-E:
   - R²¹: für Halogen oder C₁-C₄-Alkyl, insbesondere für Chlor oder Brom,
   - R²²: für C₁-C₄-Halogenalkyl, C₁-C₄-Halogenalkoxy oder C₁-C₄-Alkylsulfonyl, insbesondere für Trifluormethyl, Difluormethyloxy oder Methylsulfonyl,
   - R²³: für C₁-C₄-Alkyl, insbesondere für Methyl.
6. In einer weiteren bevorzugten Ausführungsform steht R¹ in Formel I für einen heterocyclischen Rest der Formel II-F. Derartige Verbindungen werden im Folgenden auch als Verbindungen I-F bezeichnet. In den Verbindungen I-F haben X, R² bis R⁸ vorzugsweise die als bevorzugt und insbesondere die als besonders bevorzugt angegebenen Bedeutungen. Insbesondere stehen in Formel II-F:
   - R²⁴: für Wasserstoff, Methyl, Difluormethyl oder Trifluormethyl,
   - R²⁵: für Methyl oder Trifluormethyl,
   oder
   - R²⁴ und R²⁵: zusammen für eine Kette der Formel -(CH₂)₄-.
7. In einer weiteren bevorzugten Ausführungsform steht R¹ in Formel I für einen heterocyclischen Rest der Formel II-G. Derartige Verbindungen werden im Folgenden auch als Verbindungen I-G bezeichnet. In den Verbindungen I-G haben X, R² bis R⁸ vorzugsweise die als bevorzugt und insbesondere die als besonders bevorzugt angegebenen Bedeutungen. Insbesondere stehen in Formel II-G:
   - A¹, A²: jeweils für Sauerstoff.
8. In einer weiteren bevorzugten Ausführungsform steht R¹ in Formel I für einen heterocyclischen Rest der Formel II-H. Derartige Verbindungen werden im Folgenden auch als Verbindungen I-H bezeichnet. In den Verbindungen I-H haben X, R² bis R⁸ vorzugsweise die als bevorzugt und insbesondere die als besonders bevorzugt angegebenen Bedeutungen. Insbesondere stehen in Formel II-H:
   - A³, A⁴: wie vorgenannt, vorzugsweise jeweils für Sauerstoff,
   - R²⁶, R²⁷: jeweils unabhängig voneinander für C₁-C₄-Alkyl oder C₁-C₄-Halogenalkyl, insbesondere für Methyl oder Difluormethyl oder Trifluormethyl oder
   - R²⁶ und R²⁷: zusammen für eine Kette der Formel -CH₂-O-(CH₂)₂- oder -(CH₂)₄ .

Ganz besonders bevorzugt sind die Verbindungen der Formel I, worin die Variablen
- R¹: für II-A mit R¹⁰ = CH₃ oder Amino, R¹¹ = CF₃ und R¹² = Wasserstoff,
- R²: für Wasserstoff oder Fluor,
- R³: für Chlor oder Cyano,
- R⁶: für Wasserstoff und
- X: für Sauerstoff
stehen.

Ganz besonders bevorzugt sind die 3-Heterocyclyl substituierten Benzoesäure-Derivate der Formel I-Aa (≡ I mit R¹ = II-A, R¹⁰ = Methyl, R¹¹ = Trifluormethyl und R¹² = Wasserstoff, R² = F; R³ = Cl; R⁶ = H, X = O), worin R⁴, R⁵, R⁷ und R⁸ die oben genannten Bedeutungen, insbesondere die als bevorzugt genannten Bedeutungen, aufweisen. Beispiele für derartige Verbindungen sind die Verbindungen I-Aa.1 bis I-Aa.600, in denen die Variablen R⁴, R⁵, R⁷ und R⁸ gemeinsam die in einer Zeile der Tabelle 1 angegebenen Bedeutungen aufweisen.

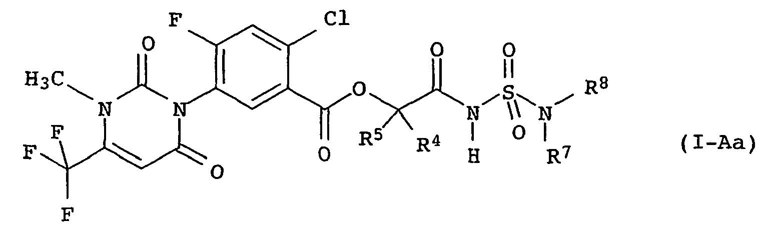

**Tabelle 1:**

| Nr. | R⁴ | R⁵ | R⁷ | R⁸ |
|---|---|---|---|---|
| 1 | H | H | H | H |
| 2 | H | H | H | CH₃ |
| 3 | H | H | H | CH₂CH₃ |
| 4 | H | H | H | CH₂CH₂CH₃ |
| 5 | H | H | H | CH₂CH₂CH₂CH₃ |
| 6 | H | H | H | CH(CH₃)₂ |
| 7 | H | H | H | CH₂CH(CH₃)₂ |
| 8 | H | H | H | CH(CH₃)CH₂CH₃ |
| 9 | H | H | H | C(CH₃)₃ |
| 10 | H | H | H | CH₂OCH₃ |
| 11 | H | H | H | CH₂CH₂OCH₃ |
| 12 | H | H | H | CH₂OCH₂CH₃ |
| 13 | H | H | H | CH₂CH₂OCH₂CH₃ |
| 14 | H | H | H | CH(CH₃)CH₂OCH₃ |
| 15 | H | H | H | CH₂CH₂Cl |
| 16 | H | H | H | CH₂CH₂SCH₃ |
| 17 | H | H | H | CH₂CH₂S(O)CH₃ |
| 18 | H | H | H | CH₂CH₂S(O)₂CH₃ |
| 19 | H | H | H | CH₂CH₂CN |
| 20 | H | H | H | CH₂CH₂CO₂CH₃ |
| 21 | H | H | H | CH₂CH₂CO₂CH₂CH₃ |
| 22 | H | H | H | CH₂CH₂NH₂ |
| 23 | H | H | H | CH₂CH₂N(CH₃)₂ |
| 24 | H | H | H | CH₂CH₂N(CH₂CH₃)₂ |
| 25 | H | H | H | CH₂CH=CH₂ |
| 26 | H | H | H | C(CH₃)=CH₂ |
| 27 | H | H | H | CH₂CH=CHCH₃ |
| 28 | H | H | H | C(CH₃)CH=CHCH₃ |
| 29 | H | H | H | CH₂C≡CH |
| 30 | H | H | H | CH(CH₃)C≡CH |
| 31 | H | H | H | CH₂C≡CHCH₃ |
| 32 | H | H | H | Ph |
| 33 | H | H | -(CH₂)₄- | |
| 34 | H | H | -(CH₂)₅- | |
| 35 | H | H | -(CH₂)₂NH(CH₂)₂- | |
| 36 | H | H | -(CH₂)₂NCH₃(CH₂)₂- | |
| 37 | H | H | -(CH₂)₂O(CH₂)₂- | |
| 38 | H | H | -CH₂CH=CHCH₂- | |
| 39 | H | H | -CH₂CH=CHCH₂CH₂- | |
| 40 | H | H | -CH=CHCH₂CH₂CH₂- | |
| 41 | H | H | CH₃ | H |
| 42 | H | H | CH₃ | CH₃ |
| 43 | H | H | CH₃ | CH₂CH₃ |
| 44 | H | H | CH₃ | CH₂CH₂CH₃ |
| 45 | H | H | CH₃ | CH₂CH₂CH₂CH₃ |
| 46 | H | H | CH₃ | CH(CH₃)₂ |
| 47 | H | H | CH₃ | CH₂CH(CH₃)₂ |
| 48 | H | H | CH₃ | CH(CH₃)CH₂CH₃ |
| 49 | H | H | CH₃ | C(CH₃)₃ |
| 50 | H | H | CH₃ | CH₂OCH₃ |
| 51 | H | H | CH₃ | CH₂CH₂OCH₃ |
| 52 | H | H | CH₃ | CH₂OCH₂CH₃ |
| 53 | H | H | CH₃ | CH₂CH₂OCH₂CH₃ |
| 54 | H | H | CH₃ | CH(CH₃)CH₂OCH₃ |
| 55 | H | H | CH₃ | CH₂CH₂Cl |
| 56 | H | H | CH₃ | CH₂CH₂SCH₃ |
| 57 | H | H | CH₃ | CH₂CH₂S(O)CH₃ |
| 58 | H | H | CH₃ | CH₂CH₂S(O)₂CH₃ |
| 59 | H | H | CH₃ | CH₂CH₂CN |
| 60 | H | H | CH₃ | CH₂CH₂CO₂CH₃ |
| 61 | H | H | CH₃ | CH₂CH₂CO₂CH₂CH₃ |
| 62 | H | H | CH₃ | CH₂CH₂NH₂ |
| 63 | H | H | CH₃ | CH₂CH₂N(CH₃)₂ |
| 64 | H | H | CH₃ | CH₂CH₂N(CH₂CH₃)₂ |
| 65 | H | H | CH₃ | CH₂CH=CH₂ |
| 66 | H | H | CH₃ | C(CH₃)=CH₂ |
| 67 | H | H | CH₃ | CH₂CH=CHCH₃ |
| 68 | H | H | CH₃ | C(CH₃)CH=CHCH₃ |
| 69 | H | H | CH₃ | CH₂C≡CH |
| 70 | H | H | CH₃ | CH(CH₃)C≡CH |
| 71 | H | H | CH₃ | CH₂C≡CHCH₃ |
| 72 | H | H | CH₃ | Ph |
| 73 | H | H | CH₂CH₃ | H |
| 74 | H | H | CH₂CH₃ | CH₃ |
| 75 | H | H | CH₂CH₃ | CH₂CH₃ |
| 76 | H | H | CH₂CH₃ | CH₂CH₂CH₃ |
| 77 | H | H | CH₂CH₃ | CH₂CH₂CH₂CH₃ |
| 78 | H | H | CH₂CH₃ | CH(CH₃)₂ |
| 79 | H | H | CH₂CH₃ | CH₂CH(CH₃)₂ |
| 80 | H | H | CH₂CH₃ | CH(CH₃)CH₂CH₃ |
| 81 | H | H | CH₂CH₃ | C(CH₃)₃ |
| 82 | H | H | CH₂CH₃ | CH₂OCH₃ |
| 83 | H | H | CH₂CH₃ | CH₂CH₂OCH₃ |
| 84 | H | H | CH₂CH₃ | CH₂OCH₂CH₃ |
| 85 | H | H | CH₂CH₃ | CH₂CH₂OCH₂CH₃ |
| 86 | H | H | CH₂CH₃ | CH(CH₃)CH₂OCH₃ |
| 87 | H | H | CH₂CH₃ | CH₂CH₂Cl |
| 88 | H | H | CH₂CH₃ | CH₂CH₂SCH₃ |
| 89 | H | H | CH₂CH₃ | CH₂CH₂S(O)CH₃ |
| 90 | H | H | CH₂CH₃ | CH₂CH₂S(O)₂CH₃ |
| 91 | H | H | CH₂CH₃ | CH₂CH₂CN |
| 92 | H | H | CH₂CH₃ | CH₂CH₂CO₂CH₃ |
| 93 | H | H | CH₂CH₃ | CH₂CH₂CO₂CH₂CH₃ |
| 94 | H | H | CH₂CH₃ | CH₂CH₂NH₂ |
| 95 | H | H | CH₂CH₃ | CH₂CH₂N(CH₃)₂ |
| 96 | H | H | CH₂CH₃ | CH₂CH₂N(CH₂CH₃)₂ |
| 97 | H | H | CH₂CH₃ | CH₂CH=CH₂ |
| 98 | H | H | CH₂CH₃ | C(CH₃)=CH₂ |
| 99 | H | H | CH₂CH₃ | CH₂CH=CHCH₃ |
| 100 | H | H | CH₂CH₃ | C(CH₃)CH=CHCH₃ |
| 101 | H | H | CH₂CH₃ | CH₂C≡CH |
| 102 | H | H | CH₂CH₃ | CH(CH₃)C≡CH |
| 103 | H | H | CH₂CH₃ | CH₂C≡CHCH₃ |
| 104 | H | H | CH₂CH₃ | Ph |
| 105 | H | H | CH₂CH₂CH₃ | H |
| 106 | H | H | CH₂CH₂CH₃ | CH₃ |
| 107 | H | H | CH₂CH₂CH₃ | CH₂CH₃ |
| 108 | H | H | CH₂CH₂CH₃ | CH₂CH₂CH₃ |
| 109 | H | H | CH₂CH₂CH₃ | CH₂CH₂CH₂CH₃ |
| 110 | H | H | CH₂CH₂CH₃ | CH(CH₃)₂ |
| 111 | H | H | CH₂CH₂CH₃ | CH₂CH(CH₃)₂ |
| 112 | H | H | CH₂CH₂CH₃ | CH(CH₃)CH₂CH₃ |
| 113 | H | H | CH₂CH₂CH₃ | C(CH₃)₃ |
| 114 | H | H | CH₂CH₂CH₃ | CH₂OCH₃ |
| 115 | H | H | CH₂CH₂CH₃ | CH₂CH₂OCH₃ |
| 116 | H | H | CH₂CH₂CH₃ | CH₂OCH₂CH₃ |
| 117 | H | H | CH₂CH₂CH₃ | CH₂CH₂OCH₂CH₃ |
| 118 | H | H | CH₂CH₂CH₃ | CH(CH₃)CH₂OCH₃ |
| 119 | H | H | CH₂CH₂CH₃ | CH₂CH₂Cl |
| 120 | H | H | CH₂CH₂CH₃ | CH₂CH₂SCH₃ |
| 121 | H | H | CH₂CH₂CH₃ | CH₂CH₂S(O)CH₃ |
| 122 | H | H | CH₂CH₂CH₃ | CH₂CH₂S(O)₂CH₃ |
| 123 | H | H | CH₂CH₂CH₃ | CH₂CH₂CN |
| 124 | H | H | CH₂CH₂CH₃ | CH₂CH₂CO₂CH₃ |
| 125 | H | H | CH₂CH₂CH₃ | CH₂CH₂CO₂CH₂CH₃ |
| 126 | H | H | CH₂CH₂CH₃ | CH₂CH₂NH₂ |
| 127 | H | H | CH₂CH₂CH₃ | CH₂CH₂N(CH₃)₂ |
| 128 | H | H | CH₂CH₂CH₃ | CH₂CH₂N(CH₂CH₃)₂ |
| 129 | H | H | CH₂CH₂CH₃ | CH₂CH=CH₂ |
| 130 | H | H | CH₂CH₂CH₃ | C(CH₃)=CH₂ |
| 131 | H | H | CH₂CH₂CH₃ | CH₂CH=CHCH₃ |
| 132 | H | H | CH₂CH₂CH₃ | C(CH₃)CH=CHCH₃ |
| 133 | H | H | CH₂CH₂CH₃ | CH₂C≡CH |
| 134 | H | H | CH₂CH₂CH₃ | CH(CH₃)C≡CH |
| 135 | H | H | CH₂CH₂CH₃ | CH₂C≡CHCH₃ |
| 136 | H | H | CH₂CH₂CH₃ | Ph |
| 137 | H | H | CH(CH₃)₂ | H |
| 138 | H | H | CH(CH₃)₂ | CH₃ |
| 139 | H | H | CH(CH₃)₂ | CH₂CH₃ |
| 140 | H | H | CH(CH₃)₂ | CH₂CH₂CH₃ |
| 141 | H | H | CH(CH₃)₂ | CH₂CH₂CH₂CH₃ |
| 142 | H | H | CH(CH₃)₂ | CH(CH₃)₂ |
| 143 | H | H | CH(CH₃)₂ | CH₂CH(CH₃)₂ |
| 144 | H | H | CH(CH₃)₂ | CH(CH₃)CH₂CH₃ |
| 145 | H | H | CH(CH₃)₂ | C(CH₃)₃ |
| 146 | H | H | CH(CH₃)₂ | CH₂OCH₃ |
| 147 | H | H | CH(CH₃)₂ | CH₂CH₂OCH₃ |
| 148 | H | H | CH(CH₃)₂ | CH₂OCH₂CH₃ |
| 149 | H | H | CH(CH₃)₂ | CH₂CH₂OCH₂CH₃ |
| 150 | H | H | CH(CH₃)₂ | CH(CH₃)CH₂OCH₃ |
| 151 | H | H | CH(CH₃)₂ | CH₂CH₂Cl |
| 152 | H | H | CH(CH₃)₂ | CH₂CH₂SCH₃ |
| 153 | H | H | CH(CH₃)₂ | CH₂CH₂S(O)CH₃ |
| 154 | H | H | CH(CH₃)₂ | CH₂CH₂S(O)₂CH₃ |
| 155 | H | H | CH(CH₃)₂ | CH₂CH₂CN |
| 156 | H | H | CH(CH₃)₂ | CH₂CH₂CO₂CH₃ |
| 157 | H | H | CH(CH₃)₂ | CH₂CH₂CO₂CH₂CH₃ |
| 158 | H | H | CH(CH₃)₂ | CH₂CH₂NH₂ |
| 159 | H | H | CH(CH₃)₂ | CH₂CH₂N(CH₃)₂ |
| 160 | H | H | CH(CH₃)₂ | CH₂CH₂N(CH₂CH₃)₂ |
| 161 | H | H | CH(CH₃)₂ | CH₂CH=CH₂ |
| 162 | H | H | CH(CH₃)₂ | C(CH₃)=CH₂ |
| 163 | H | H | CH(CH₃)₂ | CH₂CH=CHCH₃ |
| 164 | H | H | CH(CH₃)₂ | C(CH₃)CH=CHCH₃ |
| 165 | H | H | CH(CH₃)₂ | CH₂C≡CH |
| 166 | H | H | CH(CH₃)₂ | CH(CH₃)C≡CH |
| 167 | H | H | CH(CH₃)₂ | CH₂C≡CHCH₃ |
| 168 | H | H | CH(CH₃)₂ | Ph |
| 169 | H | H | CH₂CH=CH₂ | H |
| 170 | H | H | CH₂CH=CH₂ | CH₃ |
| 171 | H | H | CH₂CH=CH₂ | CH₂CH₃ |
| 172 | H | H | CH₂CH=CH₂ | CH₂CH₂CH₃ |
| 173 | H | H | CH₂CH=CH₂ | CH₂CH₂CH₂CH₃ |
| 174 | H | H | CH₂CH=CH₂ | CH(CH₃)₂ |
| 175 | H | H | CH₂CH=CH₂ | CH₂CH(CH₃)₂ |
| 176 | H | H | CH₂CH=CH₂ | CH(CH₃)CH₂CH₃ |
| 177 | H | H | CH₂CH=CH₂ | C(CH₃)₃ |
| 178 | H | H | CH₂CH=CH₂ | CH₂OCH₃ |
| 179 | H | H | CH₂CH=CH₂ | CH₂CH₂OCH₃ |
| 180 | H | H | CH₂CH=CH₂ | CH₂OCH₂CH₃ |
| 181 | H | H | CH₂CH=CH₂ | CH₂CH₂OCH₂CH₃ |
| 182 | H | H | CH₂CH=CH₂ | CH(CH₃)CH₂OCH₃ |
| 183 | H | H | CH₂CH=CH₂ | CH₂CH₂Cl |
| 184 | H | H | CH₂CH=CH₂ | CH₂CH₂SCH₃ |
| 185 | H | H | CH₂CH=CH₂ | CH₂CH₂S(O)CH₃ |
| 186 | H | H | CH₂CH=CH₂ | CH₂CH₂S(O)₂CH₃ |
| 187 | H | H | CH₂CH=CH₂ | CH₂CH₂CN |
| 188 | H | H | CH₂CH=CH₂ | CH₂CH₂CO₂CH₃ |
| 189 | H | H | CH₂CH=CH₂ | CH₂CH₂CO₂CH₂CH₃ |
| 190 | H | H | CH₂CH=CH₂ | CH₂CH₂NH₂ |
| 191 | H | H | CH₂CH=CH₂ | CH₂CH₂N(CH₃)₂ |
| 192 | H | H | CH₂CH=CH₂ | CH₂CH₂N(CH₂CH₃)₂ |
| 193 | H | H | CH₂CH=CH₂ | CH₂CH=CH₂ |
| 194 | H | H | CH₂CH=CH₂ | C(CH₃)=CH₂ |
| 195 | H | H | CH₂CH=CH₂ | CH₂CH=CHCH₃ |
| 196 | H | H | CH₂CH=CH₂ | C(CH₃)CH=CHCH₃ |
| 197 | H | H | CH₂CH=CH₂ | CH₂C≡CH |
| 198 | H | H | CH₂CH=CH₂ | CH(CH₃)C≡CH |
| 199 | H | H | CH₂CH=CH₂ | CH₂C≡CHCH₃ |
| 200 | H | H | CH₂CH=CH₂ | Ph |
| 201 | CH₃ | H | H | H |
| 202 | CH₃ | H | H | CH₃ |
| 203 | CH₃ | H | H | CH₂CH₃ |
| 204 | CH₃ | H | H | CH₂CH₂CH₃ |
| 205 | CH₃ | H | H | CH₂CH₂CH₂CH₃ |
| 206 | CH₃ | H | H | CH(CH₃)₂ |
| 207 | CH₃ | H | H | CH₂CH(CH₃)₂ |
| 208 | CH₃ | H | H | CH(CH₃)CH₂CH₃ |
| 209 | CH₃ | H | H | C(CH₃)₃ |
| 210 | CH₃ | H | H | CH₂OCH₃ |
| 211 | CH₃ | H | H | CH₂CH₂OCH₃ |
| 212 | CH₃ | H | H | CH₂OCH₂CH₃ |
| 213 | CH₃ | H | H | CH₂CH₂OCH₂CH₃ |
| 214 | CH₃ | H | H | CH(CH₃)CH₂OCH₃ |
| 215 | CH₃ | H | H | CH₂CH₂Cl |
| 216 | CH₃ | H | H | CH₂CH₂SCH₃ |
| 217 | CH₃ | H | H | CH₂CH₂S(O)CH₃ |
| 218 | CH₃ | H | H | CH₂CH₂S(O)₂CH₃ |
| 219 | CH₃ | H | H | CH₂CH₂CN |
| 220 | CH₃ | H | H | CH₂CH₂CO₂CH₃ |
| 221 | CH₃ | H | H | CH₂CH₂CO₂CH₂CH₃ |
| 222 | CH₃ | H | H | CH₂CH₂NH₂ |
| 223 | CH₃ | H | H | CH₂CH₂N(CH₃)₂ |
| 224 | CH₃ | H | H | CH₂CH₂N(CH₂CH₃)₂ |
| 225 | CH₃ | H | H | CH₂CH=CH₂ |
| 226 | CH₃ | H | H | C(CH₃)=CH₂ |
| 227 | CH₃ | H | H | CH₂CH=CHCH₃ |
| 228 | CH₃ | H | H | C(CH₃)CH=CHCH₃ |
| 229 | CH₃ | H | H | CH₂C≡CH |
| 230 | CH₃ | H | H | CH(CH₃)C≡CH |
| 231 | CH₃ | H | H | CH₂C≡CHCH₃ |
| 232 | CH₃ | H | H | Ph |
| 233 | CH₃ | H | -(CH₂)₄- | |
| 234 | CH₃ | H | -(CH₂)₅- | |
| 235 | CH₃ | H | -(CH₂)₂NH(CH₂)₂- | |
| 236 | CH₃ | H | -(CH₂)₂NCH₃(CH₂)₂- | |
| 237 | CH₃ | H | -(CH₂)₂O(CH₂)₂- | |
| 238 | CH₃ | H | -CH₂CH=CHCH₂- | |
| 239 | CH₃ | H | -CH₂CH=CHCH₂CH₂- | |
| 240 | CH₃ | H | -CH=CHCH₂CH₂CH₂- | |
| 241 | CH₃ | H | CH₃ | H |
| 242 | CH₃ | H | CH₃ | CH₃ |
| 243 | CH₃ | H | CH₃ | CH₂CH₃ |
| 244 | CH₃ | H | CH₃ | CH₂CH₂CH₃ |
| 245 | CH₃ | H | CH₃ | CH₂CH₂CH₂CH₃ |
| 246 | CH₃ | H | CH₃ | CH(CH₃)₂ |
| 247 | CH₃ | H | CH₃ | CH₂CH(CH₃)₂ |
| 248 | CH₃ | H | CH₃ | CH(CH₃)CH₂CH₃ |
| 249 | CH₃ | H | CH₃ | C(CH₃)₃ |
| 250 | CH₃ | H | CH₃ | CH₂OCH₃ |
| 251 | CH₃ | H | CH₃ | CH₂CH₂OCH₃ |
| 252 | CH₃ | H | CH₃ | CH₂OCH₂CH₃ |
| 253 | CH₃ | H | CH₃ | CH₂CH₂OCH₂CH₃ |
| 254 | CH₃ | H | CH₃ | CH(CH₃)CH₂OCH₃ |
| 255 | CH₃ | H | CH₃ | CH₂CH₂Cl |
| 256 | CH₃ | H | CH₃ | CH₂CH₂SCH₃ |
| 257 | CH₃ | H | CH₃ | CH₂CH₂S(O)CH₃ |
| 258 | CH₃ | H | CH₃ | CH₂CH₂S(O)₂CH₃ |
| 259 | CH₃ | H | CH₃ | CH₂CH₂CN |
| 260 | CH₃ | H | CH₃ | CH₂CH₂CO₂CH₃ |
| 261 | CH₃ | H | CH₃ | CH₂CH₂CO₂CH₂CH₃ |
| 262 | CH₃ | H | CH₃ | CH₂CH₂NH₂ |
| 263 | CH₃ | H | CH₃ | CH₂CH₂N(CH₃)₂ |
| 264 | CH₃ | H | CH₃ | CH₂CH₂N(CH₂CH₃)₂ |
| 265 | CH₃ | H | CH₃ | CH₂CH=CH₂ |
| 266 | CH₃ | H | CH₃ | C(CH₃)=CH₂ |
| 267 | CH₃ | H | CH₃ | CH₂CH=CHCH₃ |
| 268 | CH₃ | H | CH₃ | C(CH₃)CH=CHCH₃ |
| 269 | CH₃ | H | CH₃ | CH₂C≡CH |
| 270 | CH₃ | H | CH₃ | CH(CH₃)C≡CH |
| 271 | CH₃ | H | CH₃ | CH₂C≡CHCH₃ |
| 272 | CH₃ | H | CH₃ | Ph |
| 273 | CH₃ | H | CH₂CH₃ | H |
| 274 | CH₃ | H | CH₂CH₃ | CH₃ |
| 275 | CH₃ | H | CH₂CH₃ | CH₂CH₃ |
| 276 | CH₃ | H | CH₂CH₃ | CH₂CH₂CH₃ |
| 277 | CH₃ | H | CH₂CH₃ | CH₂CH₂CH₂CH₃ |
| 278 | CH₃ | H | CH₂CH₃ | CH(CH₃)₂ |
| 279 | CH₃ | H | CH₂CH₃ | CH₂CH(CH₃)₂ |
| 280 | CH₃ | H | CH₂CH₃ | CH(CH₃)CH₂CH₃ |
| 281 | CH₃ | H | CH₂CH₃ | C(CH₃)₃ |
| 282 | CH₃ | H | CH₂CH₃ | CH₂OCH₃ |
| 283 | CH₃ | H | CH₂CH₃ | CH₂CH₂OCH₃ |
| 284 | CH₃ | H | CH₂CH₃ | CH₂OCH₂CH₃ |
| 285 | CH₃ | H | CH₂CH₃ | CH₂CH₂OCH₂CH₃ |
| 286 | CH₃ | H | CH₂CH₃ | CH(CH₃)CH₂OCH₃ |
| 287 | CH₃ | H | CH₂CH₃ | CH₂CH₂Cl |
| 288 | CH₃ | H | CH₂CH₃ | CH₂CH₂SCH₃ |
| 289 | CH₃ | H | CH₂CH₃ | CH₂CH₂S(O)CH₃ |
| 290 | CH₃ | H | CH₂CH₃ | CH₂CH₂S(O)₂CH₃ |
| 291 | CH₃ | H | CH₂CH₃ | CH₂CH₂CN |
| 292 | CH₃ | H | CH₂CH₃ | CH₂CH₂CO₂CH₃ |
| 293 | CH₃ | H | CH₂CH₃ | CH₂CH₂CO₂CH₂CH₃ |
| 294 | CH₃ | H | CH₂CH₃ | CH₂CH₂NH₂ |
| 295 | CH₃ | H | CH₂CH₃ | CH₂CH₂N(CH₃)₂ |
| 296 | CH₃ | H | CH₂CH₃ | CH₂CH₂N(CH₂CH₃)₂ |
| 297 | CH₃ | H | CH₂CH₃ | CH₂CH=CH₂ |
| 298 | CH₃ | H | CH₂CH₃ | C(CH₃)=CH₂ |
| 299 | CH₃ | H | CH₂CH₃ | CH₂CH=CHCH₃ |
| 300 | CH₃ | H | CH₂CH₃ | C(CH₃)CH=CHCH₃ |
| 301 | CH₃ | H | CH₂CH₃ | CH₂C≡CH |
| 302 | CH₃ | H | CH₂CH₃ | CH(CH₃)C≡CH |
| 303 | CH₃ | H | CH₂CH₃ | CH₂C≡CHCH₃ |
| 304 | CH₃ | H | CH₂CH₃ | Ph |
| 305 | CH₃ | H | CH₂CH₂CH₃ | H |
| 306 | CH₃ | H | CH₂CH₂CH₃ | CH₃ |
| 307 | CH₃ | H | CH₂CH₂CH₃ | CH₂CH₃ |
| 308 | CH₃ | H | CH₂CH₂CH₃ | CH₂CH₂CH₃ |
| 309 | CH₃ | H | CH₂CH₂CH₃ | CH₂CH₂CH₂CH₃ |
| 310 | CH₃ | H | CH₂CH₂CH₃ | CH(CH₃)₂ |
| 311 | CH₃ | H | CH₂CH₂CH₃ | CH₂CH(CH₃)₂ |
| 312 | CH₃ | H | CH₂CH₂CH₃ | CH(CH₃)CH₂CH₃ |
| 313 | CH₃ | H | CH₂CH₂CH₃ | C(CH₃)₃ |
| 314 | CH₃ | H | CH₂CH₂CH₃ | CH₂OCH₃ |
| 315 | CH₃ | H | CH₂CH₂CH₃ | CH₂CH₂OCH₃ |
| 316 | CH₃ | H | CH₂CH₂CH₃ | CH₂OCH₂CH₃ |
| 317 | CH₃ | H | CH₂CH₂CH₃ | CH₂CH₂OCH₂CH₃ |
| 318 | CH₃ | H | CH₂CH₂CH₃ | CH(CH₃)CH₂OCH₃ |
| 319 | CH₃ | H | CH₂CH₂CH₃ | CH₂CH₂Cl |
| 320 | CH₃ | H | CH₂CH₂CH₃ | CH₂CH₂SCH₃ |
| 321 | CH₃ | H | CH₂CH₂CH₃ | CH₂CH₂S(O)CH₃ |
| 322 | CH₃ | H | CH₂CH₂CH₃ | CH₂CH₂S(O)₂CH₃ |
| 323 | CH₃ | H | CH₂CH₂CH₃ | CH₂CH₂CN |
| 324 | CH₃ | H | CH₂CH₂CH₃ | CH₂CH₂CO₂CH₃ |
| 325 | CH₃ | H | CH₂CH₂CH₃ | CH₂CH₂CO₂CH₂CH₃ |
| 326 | CH₃ | H | CH₂CH₂CH₃ | CH₂CH₂NH₂ |
| 327 | CH₃ | H | CH₂CH₂CH₃ | CH₂CH₂N(CH₃)₂ |
| 328 | CH₃ | H | CH₂CH₂CH₃ | CH₂CH₂N(CH₂CH₃)₂ |
| 329 | CH₃ | H | CH₂CH₂CH₃ | CH₂CH=CH₂ |
| 330 | CH₃ | H | CH₂CH₂CH₃ | C(CH₃)=CH₂ |
| 331 | CH₃ | H | CH₂CH₂CH₃ | CH₂CH=CHCH₃ |
| 332 | CH₃ | H | CH₂CH₂CH₃ | C(CH₃)CH=CHCH₃ |
| 333 | CH₃ | H | CH₂CH₂CH₃ | CH₂C≡CH |
| 334 | CH₃ | H | CH₂CH₂CH₃ | CH(CH₃)C≡CH |
| 335 | CH₃ | H | CH₂CH₂CH₃ | CH₂C≡CHCH₃ |
| 336 | CH₃ | H | CH₂CH₂CH₃ | Ph |
| 337 | CH₃ | H | CH(CH₃)₂ | H |
| 338 | CH₃ | H | CH(CH₃)₂ | CH₃ |
| 339 | CH₃ | H | CH(CH₃)₂ | CH₂CH₃ |
| 340 | CH₃ | H | CH(CH₃)₂ | CH₂CH₂CH₃ |
| 341 | CH₃ | H | CH(CH₃)₂ | CH₂CH₂CH₂CH₃ |
| 342 | CH₃ | H | CH(CH₃)₂ | CH(CH₃)₂ |
| 343 | CH₃ | H | CH(CH₃)₂ | CH₂CH(CH₃)₂ |
| 344 | CH₃ | H | CH(CH₃)₂ | CH(CH₃)CH₂CH₃ |
| 345 | CH₃ | H | CH(CH₃)₂ | C(CH₃)₃ |
| 346 | CH₃ | H | CH(CH₃)₂ | CH₂OCH₃ |
| 347 | CH₃ | H | CH(CH₃)₂ | CH₂CH₂OCH₃ |
| 348 | CH₃ | H | CH(CH₃)₂ | CH₂OCH₂CH₃ |
| 349 | CH₃ | H | CH(CH₃)₂ | CH₂CH₂OCH₂CH₃ |
| 350 | CH₃ | H | CH(CH₃)₂ | CH(CH₃)CH₂OCH₃ |
| 351 | CH₃ | H | CH(CH₃)₂ | CH₂CH₂Cl |
| 352 | CH₃ | H | CH(CH₃)₂ | CH₂CH₂SCH₃ |
| 353 | CH₃ | H | CH(CH₃)₂ | CH₂CH₂S(O)CH₃ |
| 354 | CH₃ | H | CH(CH₃)₂ | CH₂CH₂S(O)₂CH₃ |
| 355 | CH₃ | H | CH(CH₃)₂ | CH₂CH₂CN |
| 356 | CH₃ | H | CH(CH₃)₂ | CH₂CH₂CO₂CH₃ |
| 357 | CH₃ | H | CH(CH₃)₂ | CH₂CH₂CO₂CH₂CH₃ |
| 358 | CH₃ | H | CH(CH₃)₂ | CH₂CH₂NH₂ |
| 359 | CH₃ | H | CH(CH₃)₂ | CH₂CH₂N(CH₃)₂ |
| 360 | CH₃ | H | CH(CH₃)₂ | CH₂CH₂N(CH₂CH₃)₂ |
| 361 | CH₃ | H | CH(CH₃)₂ | CH₂CH=CH₂ |
| 362 | CH₃ | H | CH(CH₃)₂ | C(CH₃)=CH₂ |
| 363 | CH₃ | H | CH(CH₃)₂ | CH₂CH=CHCH₃ |
| 364 | CH₃ | H | CH(CH₃)₂ | C(CH₃)CH=CHCH₃ |
| 365 | CH₃ | H | CH(CH₃)₂ | CH₂C≡CH |
| 366 | CH₃ | H | CH(CH₃)₂ | CH(CH₃)C≡CH |
| 367 | CH₃ | H | CH(CH₃)₂ | CH₂C≡CHCH₃ |
| 368 | CH₃ | H | CH(CH₃)₂ | Ph |
| 369 | CH₃ | H | CH₂CH=CH₂ | H |
| 370 | CH₃ | H | CH₂CH=CH₂ | CH₃ |
| 371 | CH₃ | H | CH₂CH=CH₂ | CH₂CH₃ |
| 372 | CH₃ | H | CH₂CH=CH₂ | CH₂CH₂CH₃ |
| 373 | CH₃ | H | CH₂CH=CH₂ | CH₂CH₂CH₂CH₃ |
| 374 | CH₃ | H | CH₂CH=CH₂ | CH(CH₃)₂ |
| 375 | CH₃ | H | CH₂CH=CH₂ | CH₂CH(CH₃)₂ |
| 376 | CH₃ | H | CH₂CH=CH₂ | CH(CH₃)CH₂CH₃ |
| 377 | CH₃ | H | CH₂CH=CH₂ | C(CH₃)₃ |
| 378 | CH₃ | H | CH₂CH=CH₂ | CH₂OCH₃ |
| 379 | CH₃ | H | CH₂CH=CH₂ | CH₂CH₂OCH₃ |
| 380 | CH₃ | H | CH₂CH=CH₂ | CH₂OCH₂CH₃ |
| 381 | CH₃ | H | CH₂CH=CH₂ | CH₂CH₂OCH₂CH₃ |
| 382 | CH₃ | H | CH₂CH=CH₂ | CH(CH₃)CH₂OCH₃ |
| 383 | CH₃ | H | CH₂CH=CH₂ | CH₂CH₂Cl |
| 384 | CH₃ | H | CH₂CH=CH₂ | CH₂CH₂SCH₃ |
| 385 | CH₃ | H | CH₂CH=CH₂ | CH₂CH₂S(O)CH₃ |
| 386 | CH₃ | H | CH₂CH=CH₂ | CH₂CH₂S(O)₂CH₃ |
| 387 | CH₃ | H | CH₂CH=CH₂ | CH₂CH₂CN |
| 388 | CH₃ | H | CH₂CH=CH₂ | CH₂CH₂CO₂CH₃ |
| 389 | CH₃ | H | CH₂CH=CH₂ | CH₂CH₂CO₂CH₂CH₃ |
| 390 | CH₃ | H | CH₂CH=CH₂ | CH₂CH₂NH₂ |
| 391 | CH₃ | H | CH₂CH=CH₂ | CH₂CH₂N(CH₃)₂ |
| 392 | CH₃ | H | CH₂CH=CH₂ | CH₂CH₂N(CH₂CH₃)₂ |
| 393 | CH₃ | H | CH₂CH=CH₂ | CH₂CH=CH₂ |
| 394 | CH₃ | H | CH₂CH=CH₂ | C(CH₃)=CH₂ |
| 395 | CH₃ | H | CH₂CH=CH₂ | CH₂CH=CHCH₃ |
| 396 | CH₃ | H | CH₂CH=CH₂ | C(CH₃)CH=CHCH₃ |
| 397 | CH₃ | H | CH₂CH=CH₂ | CH₂C≡CH |
| 398 | CH₃ | H | CH₂CH=CH₂ | CH(CH₃)C≡CH |
| 399 | CH₃ | H | CH₂CH=CH₂ | CH₂C≡CHCH₃ |
| 400 | CH₃ | H | CH₂CH=CH₂ | Ph |
| 401 | CH₃ | CH₃ | H | H |
| 402 | CH₃ | CH₃ | H | CH₃ |
| 403 | CH₃ | CH₃ | H | CH₂CH₃ |
| 404 | CH₃ | CH₃ | H | CH₂CH₂CH₃ |
| 405 | CH₃ | CH₃ | H | CH₂CH₂CH₂CH₃ |
| 406 | CH₃ | CH₃ | H | CH(CH₃)₂ |
| 407 | CH₃ | CH₃ | H | CH₂CH(CH₃)₂ |
| 408 | CH₃ | CH₃ | H | CH(CH₃)CH₂CH₃ |
| 409 | CH₃ | CH₃ | H | C(CH₃)₃ |
| 410 | CH₃ | CH₃ | H | CH₂OCH₃ |
| 411 | CH₃ | CH₃ | H | CH₂CH₂OCH₃ |
| 412 | CH₃ | CH₃ | H | CH₂OCH₂CH₃ |
| 413 | CH₃ | CH₃ | H | CH₂CH₂OCH₂CH₃ |
| 414 | CH₃ | CH₃ | H | CH(CH₃)CH₂OCH₃ |
| 415 | CH₃ | CH₃ | H | CH₂CH₂Cl |
| 416 | CH₃ | CH₃ | H | CH₂CH₂SCH₃ |
| 417 | CH₃ | CH₃ | H | CH₂CH₂S(O)CH₃ |
| 418 | CH₃ | CH₃ | H | CH₂CH₂S(O)₂CH₃ |
| 419 | CH₃ | CH₃ | H | CH₂CH₂CN |
| 420 | CH₃ | CH₃ | H | CH₂CH₂CO₂CH₃ |
| 421 | CH₃ | CH₃ | H | CH₂CH₂CO₂CH₂CH₃ |
| 422 | CH₃ | CH₃ | H | CH₂CH₂NH₂ |
| 423 | CH₃ | CH₃ | H | CH₂CH₂N(CH₃)₂ |
| 424 | CH₃ | CH₃ | H | CH₂CH₂N(CH₂CH₃)₂ |
| 425 | CH₃ | CH₃ | H | CH₂CH=CH₂ |
| 426 | CH₃ | CH₃ | H | C(CH₃)=CH₂ |
| 427 | CH₃ | CH₃ | H | CH₂CH=CHCH₃ |
| 428 | CH₃ | CH₃ | H | C(CH₃)CH=CHCH₃ |
| 429 | CH₃ | CH₃ | H | CH₂C≡CH |
| 430 | CH₃ | CH₃ | H | CH(CH₃)C≡CH |
| 431 | CH₃ | CH₃ | H | CH₂C≡CHCH₃ |
| 432 | CH₃ | CH₃ | H | Ph |
| 433 | CH₃ | CH₃ | -(CH₂)₄- | |
| 434 | CH₃ | CH₃ | -(CH₂)₅- | |
| 435 | CH₃ | CH₃ | -(CH₂)₂NH(CH₂)₂- | |
| 436 | CH₃ | CH₃ | -(CH₂)₂NCH₃(CH₂)₂- | |
| 437 | CH₃ | CH₃ | -(CH₂)₂O(CH₂)₂- | |
| 438 | CH₃ | CH₃ | -CH₂CH=CHCH₂- | |
| 439 | CH₃ | CH₃ | -CH₂CH=CHCH₂CH₂- | |
| 440 | CH₃ | CH₃ | -CH=CHCH₂CH₂CH₂- | |
| 441 | CH₃ | CH₃ | CH₃ | H |
| 442 | CH₃ | CH₃ | CH₃ | CH₃ |
| 443 | CH₃ | CH₃ | CH₃ | CH₂CH₃ |
| 444 | CH₃ | CH₃ | CH₃ | CH₂CH₂CH₃ |
| 445 | CH₃ | CH₃ | CH₃ | CH₂CH₂CH₂CH₃ |
| 446 | CH₃ | CH₃ | CH₃ | CH(CH₃)₂ |
| 447 | CH₃ | CH₃ | CH₃ | CH₂CH(CH₃)₂ |
| 448 | CH₃ | CH₃ | CH₃ | CH(CH₃)CH₂CH₃ |
| 449 | CH₃ | CH₃ | CH₃ | C(CH₃)₃ |
| 450 | CH₃ | CH₃ | CH₃ | CH₂OCH₃ |
| 451 | CH₃ | CH₃ | CH₃ | CH₂CH₂OCH₃ |
| 452 | CH₃ | CH₃ | CH₃ | CH₂OCH₂CH |
| 453 | CH₃ | CH₃ | CH₃ | CH₂CH₂OCH₂CH₃ |
| 454 | CH₃ | CH₃ | CH₃ | CH(CH₃)CH₂OCH₃ |
| 455 | CH₃ | CH₃ | CH₃ | CH₂CH₂Cl |
| 456 | CH₃ | CH₃ | CH₃ | CH₂CH₂SCH₃ |
| 457 | CH₃ | CH₃ | CH₃ | CH₂CH₂S(O)CH₃ |
| 458 | CH₃ | CH₃ | CH₃ | CH₂CH₂S(O)₂CH₃ |
| 459 | CH₃ | CH₃ | CH₃ | CH₂CH₂CN |
| 460 | CH₃ | CH₃ | CH₃ | CH₂CH₂CO₂CH₃ |
| 461 | CH₃ | CH₃ | CH₃ | CH₂CH₂CO₂CH₂CH₃ |
| 462 | CH₃ | CH₃ | CH₃ | CH₂CH₂NH₂ |
| 463 | CH₃ | CH₃ | CH₃ | CH₂CH₂N(CH₃)₂ |
| 464 | CH₃ | CH₃ | CH₃ | CH₂CH₂N(CH₂CH₃)₂ |
| 465 | CH₃ | CH₃ | CH₃ | CH₂CH=CH₂ |
| 466 | CH₃ | CH₃ | CH₃ | C(CH₃)=CH₂ |
| 467 | CH₃ | CH₃ | CH₃ | CH₂CH=CHCH₃ |
| 468 | CH₃ | CH₃ | CH₃ | C(CH₃)CH=CHCH₃ |
| 469 | CH₃ | CH₃ | CH₃ | CH₂C≡CH |
| 470 | CH₃ | CH₃ | CH₃ | CH(CH₃)C≡CH |
| 471 | CH₃ | CH₃ | CH₃ | CH₂C≡CHCH₃ |
| 472 | CH₃ | CH₃ | CH₃ | Ph |
| 473 | CH₃ | CH₃ | CH₂CH₃ | H |
| 474 | CH₃ | CH₃ | CH₂CH₃ | CH₃ |
| 475 | CH₃ | CH₃ | CH₂CH₃ | CH₂CH₃ |
| 476 | CH₃ | CH₃ | CH₂CH₃ | CH₂CH₂CH₃ |
| 477 | CH₃ | CH₃ | CH₂CH₃ | CH₂CH₂CH₂CH₃ |
| 478 | CH₃ | CH₃ | CH₂CH₃ | CH(CH₃)₂ |
| 479 | CH₃ | CH₃ | CH₂CH₃ | CH₂CH(CH₃)₂ |
| 480 | CH₃ | CH₃ | CH₂CH₃ | CH(CH₃)CH₂CH₃ |
| 481 | CH₃ | CH₃ | CH₂CH₃ | C(CH₃)₃ |
| 482 | CH₃ | CH₃ | CH₂CH₃ | CH₂OCH₃ |
| 483 | CH₃ | CH₃ | CH₂CH₃ | CH₂CH₂OCH₃ |
| 484 | CH₃ | CH₃ | CH₂CH₃ | CH₂OCH₂CH₃ |
| 485 | CH₃ | CH₃ | CH₂CH₃ | CH₂CH₂OCH₂CH₃ |
| 486 | CH₃ | CH₃ | CH₂CH₃ | CH(CH₃)CH₂OCH₃ |
| 487 | CH₃ | CH₃ | CH₂CH₃ | CH₂CH₂Cl |
| 488 | CH₃ | CH₃ | CH₂CH₃ | CH₂CH₂SCH₃ |
| 489 | CH₃ | CH₃ | CH₂CH₃ | CH₂CH₂S(O)CH₃ |
| 490 | CH₃ | CH₃ | CH₂CH₃ | CH₂CH₂S(O)₂CH₃ |
| 491 | CH₃ | CH₃ | CH₂CH₃ | CH₂CH₂CN |
| 492 | CH₃ | CH₃ | CH₂CH₃ | CH₂CH₂CO₂CH₃ |
| 493 | CH₃ | CH₃ | CH₂CH₃ | CH₂CH₂CO₂CH₂CH₃ |
| 494 | CH₃ | CH₃ | CH₂CH₃ | CH₂CH₂NH₂ |
| 495 | CH₃ | CH₃ | CH₂CH₃ | CH₂CH₂N(CH₃)₂ |
| 496 | CH₃ | CH₃ | CH₂CH₃ | CH₂CH₂N(CH₂CH₃)₂ |
| 497 | CH₃ | CH₃ | CH₂CH₃ | CH₂CH=CH₂ |
| 498 | CH₃ | CH₃ | CH₂CH₃ | C(CH₃)=CH₂ |
| 499 | CH₃ | CH₃ | CH₂CH₃ | CH₂CH=CHCH₃ |
| 500 | CH₃ | CH₃ | CH₂CH₃ | C(CH₃)CH=CHCH₃ |
| 501 | CH₃ | CH₃ | CH₂CH₃ | CH₂C≡CH |
| 502 | CH₃ | CH₃ | CH₂CH₃ | CH(CH₃)C≡CH |
| 503 | CH₃ | CH₃ | CH₂CH₃ | CH₂C≡CHCH₃ |
| 504 | CH₃ | CH₃ | CH₂CH₃ | Ph |
| 505 | CH₃ | CH₃ | CH₂CH₂CH₃ | H |
| 506 | CH₃ | CH₃ | CH₂CH₂CH₃ | CH₃ |
| 507 | CH₃ | CH₃ | CH₂CH₂CH₃ | CH₂CH₃ |
| 508 | CH₃ | CH₃ | CH₂CH₂CH₃ | CH₂CH₂CH₃ |
| 509 | CH₃ | CH₃ | CH₂CH₂CH₃ | CH₂CH₂CH₂CH₃ |
| 510 | CH₃ | CH₃ | CH₂CH₂CH₃ | CH(CH₃)₂ |
| 511 | CH₃ | CH₃ | CH₂CH₂CH₃ | CH₂CH(CH₃)₂ |
| 512 | CH₃ | CH₃ | CH₂CH₂CH₃ | CH(CH₃)CH₂CH₃ |
| 513 | CH₃ | CH₃ | CH₂CH₂CH₃ | C(CH₃)₃ |
| 514 | CH₃ | CH₃ | CH₂CH₂CH₃ | CH₂OCH₃ |
| 515 | CH₃ | CH₃ | CH₂CH₂CH₃ | CH₂CH₂OCH₃ |
| 516 | CH₃ | CH₃ | CH₂CH₂CH₃ | CH₂OCH₂CH₃ |
| 517 | CH₃ | CH₃ | CH₂CH₂CH₃ | CH₂CH₂OCH₂CH₃ |
| 518 | CH₃ | CH₃ | CH₂CH₂CH₃ | CH(CH₃)CH₂OCH₃ |
| 519 | CH₃ | CH₃ | CH₂CH₂CH₃ | CH₂CH₂Cl |
| 520 | CH₃ | CH₃ | CH₂CH₂CH₃ | CH₂CH₂SCH₃ |
| 521 | CH₃ | CH₃ | CH₂CH₂CH₃ | CH₂CH₂S(O)CH₃ |
| 522 | CH₃ | CH₃ | CH₂CH₂CH₃ | CH₂CH₂S(O)₂CH₃ |
| 523 | CH₃ | CH₃ | CH₂CH₂CH₃ | CH₂CH₂CN |
| 524 | CH₃ | CH₃ | CH₂CH₂CH₃ | CH₂CH₂CO₂CH₃ |
| 525 | CH₃ | CH₃ | CH₂CH₂CH₃ | CH₂CH₂CO₂CH₂CH₃ |
| 526 | CH₃ | CH₃ | CH₂CH₂CH₃ | CH₂CH₂NH₂ |
| 527 | CH₃ | CH₃ | CH₂CH₂CH₃ | CH₂CH₂N(CH₃)₂ |
| 528 | CH₃ | CH₃ | CH₂CH₂CH₃ | CH₂CH₂N(CH₂CH₃)₂ |
| 529 | CH₃ | CH₃ | CH₂CH₂CH₃ | CH₂CH=CH₂ |
| 530 | CH₃ | CH₃ | CH₂CH₂CH₃ | C(CH₃)=CH₂ |
| 531 | CH₃ | CH₃ | CH₂CH₂CH₃ | CH₂CH=CHCH₃ |
| 532 | CH₃ | CH₃ | CH₂CH₂CH₃ | C(CH₃)CH=CHCH₃ |
| 533 | CH₃ | CH₃ | CH₂CH₂CH₃ | CH₂C≡CH |
| 534 | CH₃ | CH₃ | CH₂CH₂CH₃ | CH(CH₃)C≡CH |
| 535 | CH₃ | CH₃ | CH₂CH₂CH₃ | CH₂C≡CHCH₃ |
| 536 | CH₃ | CH₃ | CH₂CH₂CH₃ | Ph |
| 537 | CH₃ | CH₃ | CH(CH₃)₂ | H |
| 538 | CH₃ | CH₃ | CH(CH₃)₂ | CH₃ |
| 539 | CH₃ | CH₃ | CH(CH₃)₂ | CH₂CH₃ |
| 540 | CH₃ | CH₃ | CH(CH₃)₂ | CH₂CH₂CH₃ |
| 541 | CH₃ | CH₃ | CH(CH₃)₂ | CH₂CH₂CH₂CH₃ |
| 542 | CH₃ | CH₃ | CH(CH₃)₂ | CH(CH₃)₂ |
| 543 | CH₃ | CH₃ | CH(CH₃)₂ | CH₂CH(CH₃)₂ |
| 544 | CH₃ | CH₃ | CH(CH₃)₂ | CH(CH₃)CH₂CH₃ |
| 545 | CH₃ | CH₃ | CH(CH₃)₂ | C(CH₃)₃ |
| 546 | CH₃ | CH₃ | CH(CH₃)₂ | CH₂OCH₃ |
| 547 | CH₃ | CH₃ | CH(CH₃)₂ | CH₂CH₂OCH₃ |
| 548 | CH₃ | CH₃ | CH(CH₃)₂ | CH₂OCH₂CH₃ |
| 549 | CH₃ | CH₃ | CH(CH₃)₂ | CH₂CH₂OCH₂CH₃ |
| 550 | CH₃ | CH₃ | CH(CH₃)₂ | CH(CH₃)CH₂OCH₃ |
| 551 | CH₃ | CH₃ | CH(CH₃)₂ | CH₂CH₂Cl |
| 552 | CH₃ | CH₃ | CH(CH₃)₂ | CH₂CH₂SCH₃ |
| 553 | CH₃ | CH₃ | CH(CH₃)₂ | CH₂CH₂S(O)CH₃ |
| 554 | CH₃ | CH₃ | CH(CH₃)₂ | CH₂CH₂S(O)₂CH₃ |
| 555 | CH₃ | CH₃ | CH(CH₃)₂ | CH₂CH₂CN |
| 556 | CH₃ | CH₃ | CH(CH₃)₂ | CH₂CH₂CO₂CH₃ |
| 557 | CH₃ | CH₃ | CH(CH₃)₂ | CH₂CH₂CO₂CH₂CH₃ |
| 558 | CH₃ | CH₃ | CH(CH₃)₂ | CH₂CH₂NH₂ |
| 559 | CH₃ | CH₃ | CH(CH₃)₂ | CH₂CH₂N(CH₃)₂ |
| 560 | CH₃ | CH₃ | CH(CH₃)₂ | CH₂CH₂N(CH₂CH₃)₂ |
| 561 | CH₃ | CH₃ | CH(CH₃)₂ | CH₂CH=CH₂ |
| 562 | CH₃ | CH₃ | CH(CH₃)₂ | C(CH₃)=CH₂ |
| 563 | CH₃ | CH₃ | CH(CH₃)₂ | CH₂CH=CHCH₃ |
| 564 | CH₃ | CH₃ | CH(CH₃)₂ | C(CH₃)CH=CHCH₃ |
| 565 | CH₃ | CH₃ | CH(CH₃)₂ | CH₂C≡CH |
| 566 | CH₃ | CH₃ | CH(CH₃)₂ | CH(CH₃)C≡CH |
| 567 | CH₃ | CH₃ | CH(CH₃)₂ | CH₂C≡CHCH₃ |
| 568 | CH₃ | CH₃ | CH(CH₃)₂ | Ph |
| 569 | CH₃ | CH₃ | CH₂CH=CH₂ | H |
| 570 | CH₃ | CH₃ | CH₂CH=CH₂ | CH₃ |
| 571 | CH₃ | CH₃ | CH₂CH=CH₂ | CH₂CH₃ |
| 572 | CH₃ | CH₃ | CH₂CH=CH₂ | CH₂CH₂CH₃ |
| 573 | CH₃ | CH₃ | CH₂CH=CH₂ | CH₂CH₂CH₂CH₃ |
| 574 | CH₃ | CH₃ | CH₂CH=CH₂ | CH(CH₃)₂ |
| 575 | CH₃ | CH₃ | CH₂CH=CH₂ | CH₂CH(CH₃)₂ |
| 576 | CH₃ | CH₃ | CH₂CH=CH₂ | CH(CH₃)CH₂CH₃ |
| 577 | CH₃ | CH₃ | CH₂CH=CH₂ | C(CH₃)₃ |
| 578 | CH₃ | CH₃ | CH₂CH=CH₂ | CH₂OCH₃ |
| 579 | CH₃ | CH₃ | CH₂CH=CH₂ | CH₂CH₂OCH₃ |
| 580 | CH₃ | CH₃ | CH₂CH=CH₂ | CH₂OCH₂CH₃ |
| 581 | CH₃ | CH₃ | CH₂CH=CH₂ | CH₂CH₂OCH₂CH₃ |
| 582 | CH₃ | CH₃ | CH₂CH=CH₂ | CH(CH₃)CH₂OCH₃ |
| 583 | CH₃ | CH₃ | CH₂CH=CH₂ | CH₂CH₂Cl |
| 584 | CH₃ | CH₃ | CH₂CH=CH₂ | CH₂CH₂SCH₃ |
| 585 | CH₃ | CH₃ | CH₂CH=CH₂ | CH₂CH₂S(O)CH₃ |
| 586 | CH₃ | CH₃ | CH₂CH=CH₂ | CH₂CH₂S(O)₂CH₃ |
| 587 | CH₃ | CH₃ | CH₂CH=CH₂ | CH₂CH₂CN |
| 588 | CH₃ | CH₃ | CH₂CH=CH₂ | CH₂CH₂CO₂CH₃ |
| 589 | CH₃ | CH₃ | CH₂CH=CH₂ | CH₂CH₂CO₂CH₂CH₃ |
| 590 | CH₃ | CH₃ | CH₂CH=CH₂ | CH₂CH₂NH₂ |
| 591 | CH₃ | CH₃ | CH₂CH=CH₂ | CH₂CH₂N(CH₃)₂ |
| 592 | CH₃ | CH₃ | CH₂CH=CH₂ | CH₂CH₂N(CH₂CH₃)₂ |
| 593 | CH₃ | CH₃ | CH₂CH=CH₂ | CH₂CH=CH₂ |
| 594 | CH₃ | CH₃ | CH₂CH=CH₂ | C(CH₃)=CH₂ |
| 595 | CH₃ | CH₃ | CH₂CH=CH₂ | CH₂CH=CHCH₃ |
| 596 | CH₃ | CH₃ | CH₂CH=CH₂ | C(CH₃)CH=CHCH₃ |
| 597 | CH₃ | CH₃ | CH₂CH=CH₂ | CH₂C≡CH |
| 598 | CH₃ | CH₃ | CH₂CH=CH₂ | CH(CH₃)C≡CH |
| 599 | CH₃ | CH₃ | CH₂CH=CH₂ | CH₂C≡CHCH₃ |
| 600 | CH₃ | CH₃ | CH₂CH=CH₂ | Ph |

Ganz besonders bevorzugt sind auch die 3-Heterocyclyl substituierten Benzoesäure-Derivate der Formel I-Ab (≡ I mit R¹ = II-A, R¹⁰ = Methyl, R¹¹ = Trifluormethyl und R¹² = Wasserstoff, R² = H; R³ = Cl; R⁶ = H, X = O), worin R⁴, R⁵, R⁷ und R⁸ die oben genannten Bedeutungen, insbesondere die als bevorzugt genannten Bedeutungen, aufweisen. Beispiele für derartige Verbindungen sind die Verbindungen I-Ab.1 bis I-Ab.600, in denen die Variablen R⁴, R⁵, R⁷ und R⁸ gemeinsam die in einer Zeile der Tabelle 1 angegebenen Bedeutungen aufweisen.

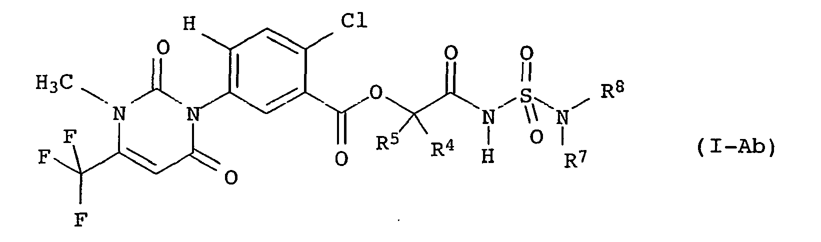

Ganz besonders bevorzugt sind auch die 3-Heterocyclyl substituierten Benzoesäure-Derivate der Formel I-Ac (≡ I mit R¹ = II-A, R¹⁰ = Amino, R¹¹ = Trifluormethyl und R¹² = Wasserstoff; R² = F; R³ = Cl; R⁶ = H, X = O), worin R⁴, R⁵, R⁷ und R⁸ die oben genannten Bedeutungen, insbesondere die als bevorzugt genannten Bedeutungen, aufweisen. Beispiele für derartige Verbindungen sind die Verbindungen I-Ac.1 bis I-Ac.600, in denen die Variablen R⁴, R⁵, R⁷ und R⁸ gemeinsam die in einer Zeile der Tabelle 1 angegebenen Bedeutungen aufweisen.

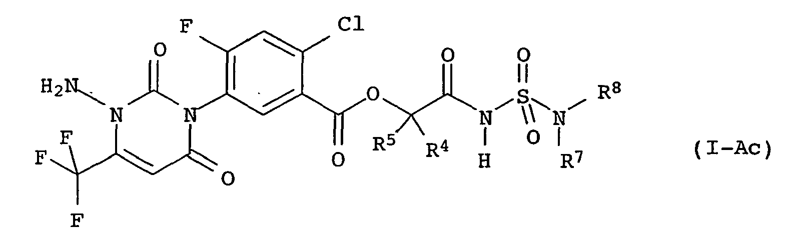

Ganz besonders bevorzugt sind auch die 3-Heterocyclyl substituierten Benzoesäure-Derivate der Formel I-Ad (≡ I mit R¹ = II-A, R¹⁰ = Amino, R¹¹ = Trifluormethyl und R¹² = Wasserstoff; R² = H; R³ = Cl; R⁶ = H, X = O), worin R⁴, R⁵, R⁷ und R⁸ die oben genannten Bedeutungen, insbesondere die als bevorzugt genannten Bedeutungen, aufweisen. Beispiele für derartige Verbindungen sind die Verbindungen I-Ad.1 bis I-Ad.600, in denen die Variablen R⁴, R⁵, R⁷ und R⁸ gemeinsam die in einer Zeile der Tabelle 1 angegebenen Bedeutungen aufweisen.

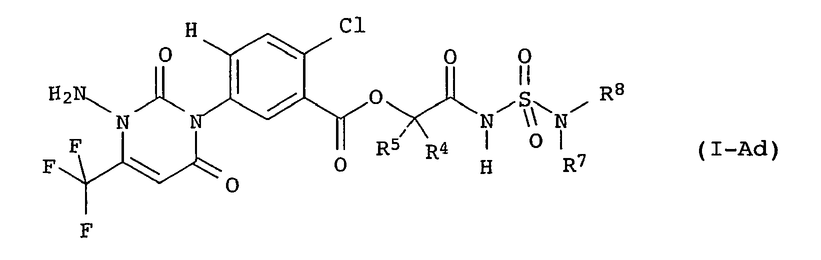

Unter den Verbindungen I-B sind die 3-Heterocyclyl substituierten Benzoesäure-Derivate der Formel I-Ba (≡ I mit R¹ = II-B, R^{13'}, R¹³ jeweils Methyl; R² = F; R³ = Cl; R⁶ = H, X = O) bevorzugt, worin R⁴, R⁵, R⁷ und R⁸ die oben genannten Bedeutungen, insbesondere die als bevorzugt angegebenen Bedeutungen, aufweisen. Beispiele für derartige Verbindungen sind die Verbindungen I-Ba.1 bis I-Ba.600, in denen die variablen R⁴, R⁵, R⁷ und R⁸ gemeinsam die in einer Zeile der Tabelle 1 angegebenen Bedeutungen aufweisen.

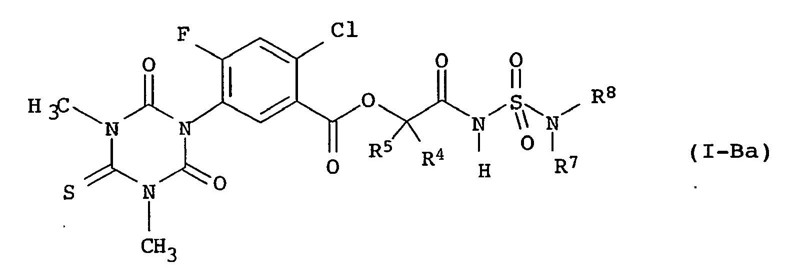

Unter den Verbindungen I-B sind die 3-Heterocyclyl substituierten Benzoesäure-Derivate der Formel I-Bb (≡ I mit R¹ = II-B, R^{13'}, R¹³ jeweils Methyl; R² = H; R³ = Cl; R⁶ = H, X = 0) bevorzugt, worin R⁴, R⁵, R⁷ und R⁸ die oben genannten Bedeutungen, insbesondere die als bevorzugt genannten Bedeutungen, aufweisen. Beispiele für derartige Verbindungen sind die Verbindungen I-Bb.1 bis I-Bb.600, in denen die Variablen R⁴, R⁵, R⁷ und R⁸ gemeinsam die in einer Zeile der Tabelle 1 angegebenen Bedeutungen aufweisen.

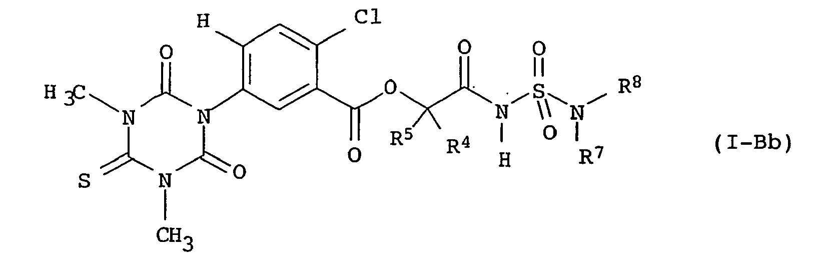

Unter den Verbindungen I-C sind die 3-Heterocyclyl substituierten Benzoesäure-Derivate der Formel I-Ca (≡ I mit R¹ = II-C, R¹⁴ = Chlor, R¹⁵, R¹⁷ = Wasserstoff, R¹⁶ = Trifluormethyl; R² = F; R³ = Cl; R⁶ = H, X = O) bevorzugt, worin R⁴, R⁵, R⁷ und R⁸ die oben genannten Bedeutungen, insbesondere die als bevorzugt genannten Bedeutungen, aufweisen. Beispiele für derartige Verbindungen sind die Verbindungen I-Ca.1 bis I-Ca.600, in denen die Variablen R⁴, R⁵, R⁷ und R⁸ gemeinsam die in einer Zeile der Tabelle 1 angegebenen Bedeutungen aufweisen.

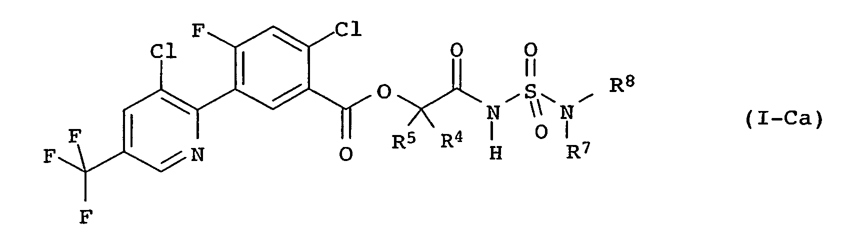

Unter den Verbindungen I-C sind die 3-Heterocyclyl substituierten Benzoesäure-Derivate der Formel I-Cb (≡ I mit R¹ = II-C, R¹⁴ = Chlor, R^{15,} R¹⁷ = Wasserstoff, R¹⁶ = Trifluormethyl; R² = H; R³ = Cl; R⁶ = H, X = O) bevorzugt, worin R⁴, R⁵, R⁷ und R⁸ die oben genannten Bedeutungen, insbesondere die als bevorzugt genannten Bedeutungen, aufweisen. Beispiele für derartige Verbindungen sind die Verbindungen I-Cb.1 bis I-Cb.600, in denen die Variablen R⁴, R⁵, R⁷ und R⁸ gemeinsam die in einer Zeile der Tabelle 1 angegebenen Bedeutungen aufweisen.

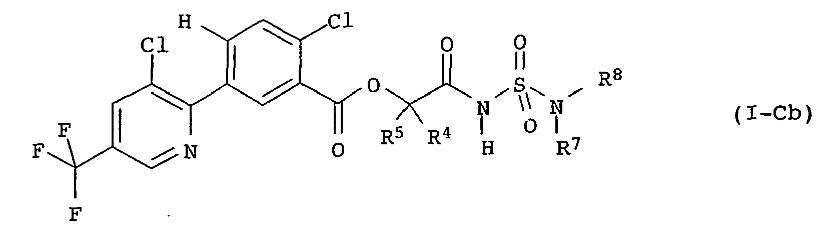

Unter den Verbindungen I-C sind die 3-Heterocyclyl substituierten Benzoesäure-Derivate der Formel I-Cc (≡ I mit R¹ = II-C, R¹⁴ = Chlor, R¹⁵, R¹⁷ = Wasserstoff, R¹⁶ = Methylsulfonyl; R² = F; R³ = Cl; R⁶ = H, X = O) bevorzugt, worin R⁴, R⁵, R⁷ und R⁸ die oben genannten Bedeutungen, insbesondere die als bevorzugt genannten Bedeutungen, aufweisen. Beispiele für derartige Verbindungen sind die Verbindungen I-Cc.1 bis I-Cc.600, in denen die Variablen R⁴, R⁵, R⁷ und R⁸ gemeinsam die in einer Zeile der Tabelle 1 angegebenen Bedeutungen aufweisen.

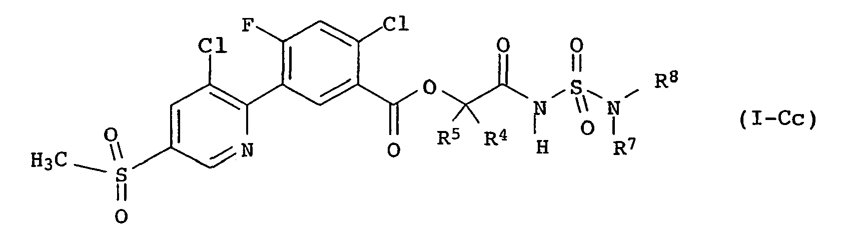

Unter den Verbindungen I-C sind die 3-Heterocyclyl substituierten Benzoesäure-Derivate der Formel I-Cd (≡ I mit R¹ = II-C, R¹⁴ = Chlor, R¹⁵, R¹⁷ = Wasserstoff, R¹⁶ = Methylsulfonyl; R² = H; R³ = Cl; R⁶ = H, X = O) bevorzugt, worin R⁴, R⁵, R⁷ und R⁸ die oben genannten Bedeutungen, insbesondere die als bevorzugt genannten Bedeutungen, aufweisen. Beispiele für derartige Verbindungen sind die Verbindungen I-Cd.1 bis I-Cd.600, in denen die Variablen R⁴, R⁵, R⁷ und R8 gemeinsam die in einer Zeile der Tabelle 1 angegebenen Bedeutungen aufweisen.

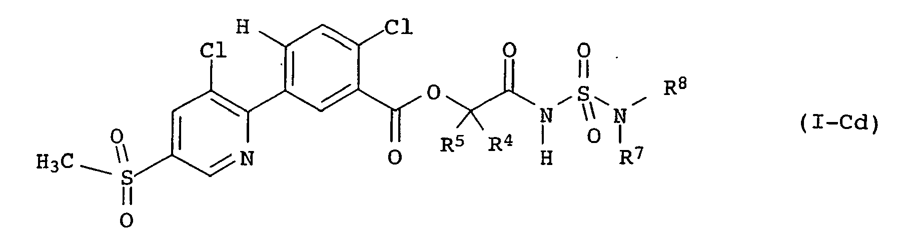

Unter den Verbindungen I-C sind die 3-Heterocyclyl substituierten Benzoesäure-Derivate der Formel I-Ce (≡ I mit R¹ = II-C, R¹⁴ = Chlor, R¹⁵, R¹⁷ = Wasserstoff, R¹⁶ = Methylsulfonyloxy; R² = F; R³ = Cl; R⁶ = H, X = 0) bevorzugt, worin R⁴, R⁵, R⁷ und R⁸ die oben genannten Bedeutungen, insbesondere die als bevorzugt genannten Bedeutungen, aufweisen. Beispiele für derartige Verbindungen sind die Verbindungen I-Ce.1 bis I-Ce.600, in denen die Variablen R⁴, R⁵, R⁷ und R⁸ gemeinsam die in einer Zeile der Tabelle 1 angegebenen Bedeutungen aufweisen.

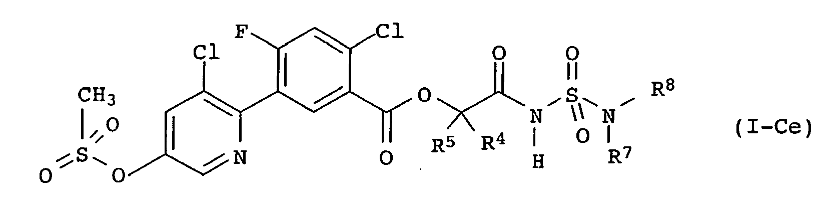

Unter den Verbindungen I-C sind die 3-Heterocyclyl substituierten Benzoesäure-Derivate der Formel I-Cf (≡ I mit R¹ = II-C, R¹⁴ = Chlor, R¹⁵, R¹⁷ = Wasserstoff, R¹⁶ = Methylsulfonyloxy; R² = H; R³ = Cl; R⁶ = H, X = O) bevorzugt, worin R⁴, R⁵, R⁷ und R⁸ die oben genannten Bedeutungen, insbesondere die als bevorzugt genannten Bedeutungen, aufweisen. Beispiele für derartige Verbindungen sind die Verbindungen I-Cf.1 bis I-Cf.600, in denen die Variablen R⁴, R⁵, R⁷ und R⁸ gemeinsam die in einer Zeile der Tabelle 1 angegebenen Bedeutungen aufweisen.

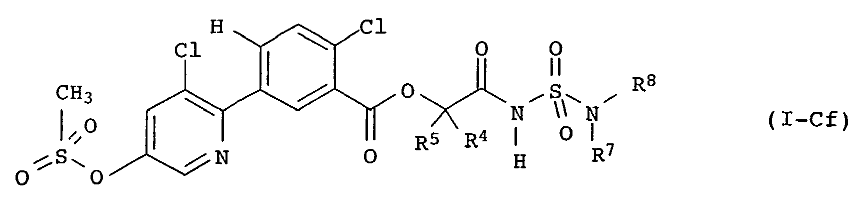

Unter den Verbindungen I-D sind die 3-Heterocyclyl substituierten Benzoesäure-Derivate der Formel I-Da (≡ I mit R¹ = II-D, R¹⁸, R²⁰ = Wasserstoff, R¹⁹ = Trifluormethyl; R² = F; R³ = Cl; R⁶ = H, X = O) bevorzugt, worin R⁴, R⁵, R⁷ und R⁸ die oben genannten Bedeutungen, insbesondere die als bevorzugt genannten Bedeutungen, aufweisen. Beispiele für derartige Verbindungen sind die Verbindungen I-Da.1 bis I-Da.600, in denen die Variablen R⁴, R⁵, R⁷ und R⁸ gemeinsam die in einer Zeile der Tabelle 1 angegebenen Bedeutungen aufweisen.

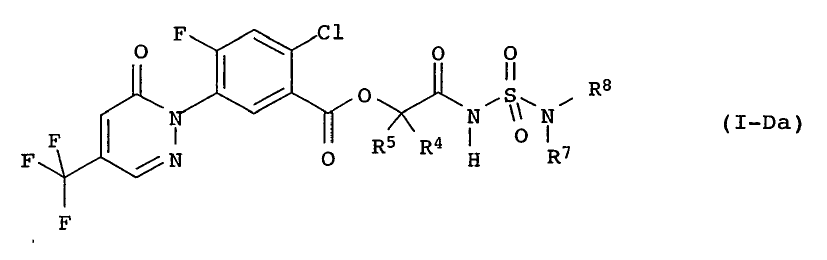

Unter den Verbindungen I-D sind die 3-Heterocyclyl substituierten Benzoesäure-Derivate der Formel I-Db (≡ I mit R¹ = II-D, R¹⁸, R²⁰ = Wasserstoff, R¹⁹ = Trifluormethyl; R² = H; R³ = Cl; R⁶ = H, X = O) bevorzugt, worin R⁴, R⁵, R⁷ und R⁸ die oben genannten Bedeutungen, insbesondere die als bevorzugt genannten Bedeutungen, aufweisen. Beispiele für derartige Verbindungen sind die Verbindungen I-Db.1 bis I-Db.600, in denen die Variablen R⁴, R⁵, R⁷ und R⁸ gemeinsam die in einer Zeile der Tabelle 1 angegebenen Bedeutungen aufweisen.

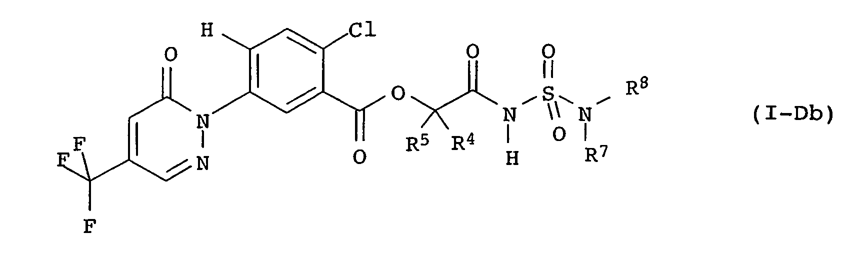

Unter den Verbindungen I-D sind die 3-Heterocyclyl substituierten Benzoesäure-Derivate der Formel I-Dc (≡ I mit R¹ = II-D, R¹⁸ = Methyl, R¹⁹ = Trifluormethyl, R²⁰ = Wasserstoff; R² = F; R³ = Cl; R⁶ = H, X = O) bevorzugt, worin R⁴, R⁵, R⁷ und R⁸ die oben genannten Bedeutungen, insbesondere die als bevorzugt genannten Bedeutungen, aufweisen. Beispiele für derartige Verbindungen sind die Verbindungen I-Dc.1 bis I-Dc.600, in denen die Variablen R⁴, R⁵, R⁷ und R⁸ gemeinsam die in einer Zeile der Tabelle 1 angegebenen Bedeutungen aufweisen.

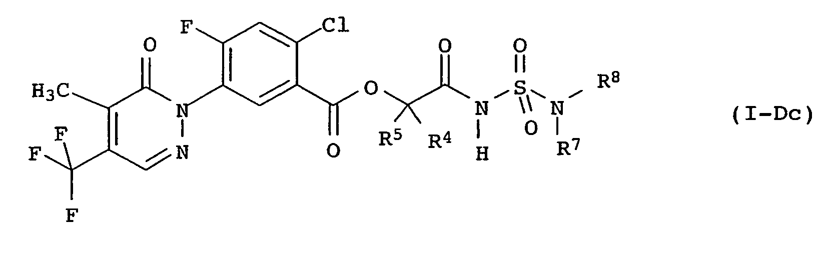

Unter den Verbindungen I-D sind die 3-Heterocyclyl substituierten Benzoesäure-Derivate der Formel I-Dd (≡ I mit R¹ = II-D, R¹⁸ = Methyl, R¹⁹ = Trifluormethyl, R²⁰ = Wasserstoff; R² = H; R³ = Cl; R⁶ = H, X = O) bevorzugt, worin R⁴, R⁵, R⁷ und R⁸ die oben genannten Bedeutungen, insbesondere die als bevorzugt genannten Bedeutungen, aufweisen. Beispiele für derartige Verbindungen sind die Verbindungen I-Dd.1 bis I-Dd.600, in denen die Variablen R⁴, R⁵, R⁷ und R⁸ gemeinsam die in einer Zeile der Tabelle 1 angegebenen Bedeutungen aufweisen.

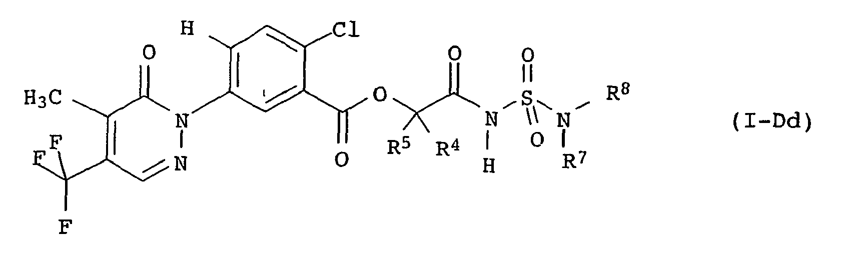

Unter den Verbindungen I-D sind die 3-Heterocyclyl substituierten Benzoesäure-Derivate der Formel I-De (≡ I mit R¹ = II-D, R¹⁸ = Amino, R¹⁹ = Methylsulfonyl, R²⁰ = Wasserstoff; R² = F; R³ = Cl; R⁶ = H, X = O) bevorzugt, worin R⁴, R⁵, R⁷ und R⁸ die oben genannten Bedeutungen, insbesondere die als bevorzugt genannten Bedeutungen, aufweisen. Beispiele für derartige Verbindungen sind die Verbindungen I-De.1 bis I-De.600, in denen die Variablen R⁴, R⁵, R⁷ und R⁸ gemeinsam die in einer Zeile der Tabelle 1 angegebenen Bedeutungen aufweisen.

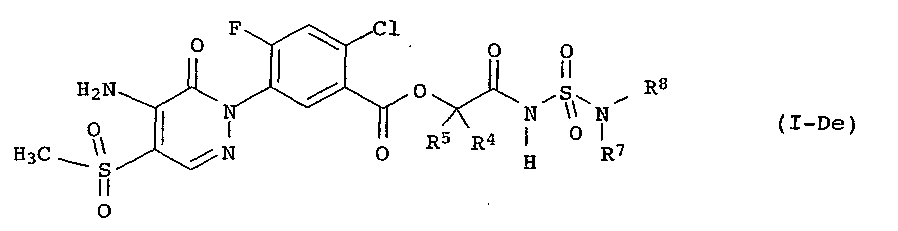

Unter den Verbindungen I-D sind die 3-Heterocyclyl substituierten Benzoesäure-Derivate der Formel I-Df (≡ I mit R¹ = II-D, R¹⁸ = Amino, R¹⁹ = Methylsulfonyl, R²⁰ = Wasserstoff; R² = H; R³ = Cl; R⁶ = H, X = 0) bevorzugt, worin R⁴, R⁵, R⁷ und R⁸ die oben genannten Bedeutungen, insbesondere die als bevorzugt genannten Bedeutungen, aufweisen. Beispiele für derartige Verbindungen sind die Verbindungen I-Df.1 bis I-Df.600, in denen die Variablen R⁴, R⁵, R⁷ und R⁸ gemeinsam die in einer Zeile der Tabelle 1 angegebenen Bedeutungen aufweisen.

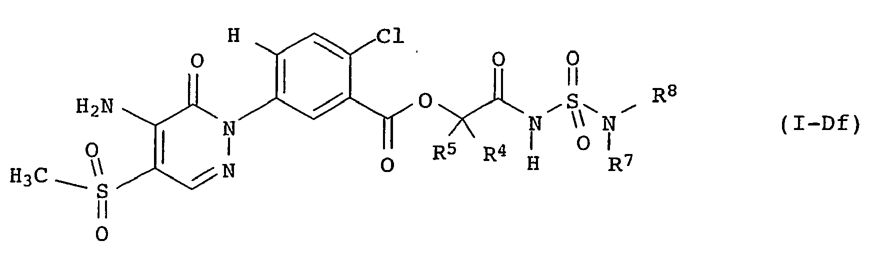

Unter den Verbindungen I-E sind die 3-Heterocyclyl substituierten Benzoesäure-Derivate der Formel I-Ea (≡ I mit R¹ = II-E, R²¹ = Chlor, R²² = Trifluormethyl, R²³ = Methyl; R² = F; R³ = Cl; R⁶ = H, X = O) bevorzugt, worin R⁴, R⁵, R⁷ und R⁸ die oben genannten Bedeutungen, insbesondere die als bevorzugt genannten Bedeutungen, aufweisen. Beispiele für derartige Verbindungen sind die Verbindungen I-Ea.1 bis I-Ea.600, in denen die Variablen R⁴, R⁵, R⁷ und R⁸ gemeinsam die in einer Zeile der Tabelle 1 angegebenen Bedeutungen aufweisen.

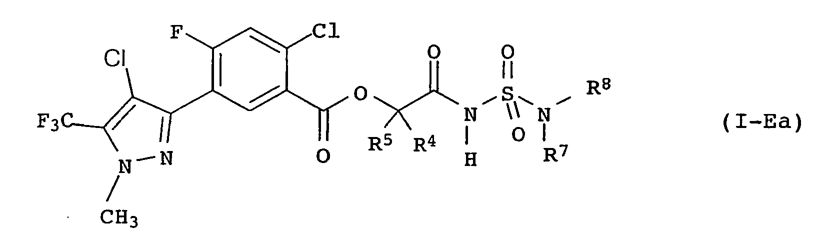

Unter den Verbindungen I-E sind die 3-Heterocyclyl substituierten Benzoesäure-Derivate der Formel I-Eb (≡ I mit R¹ = II-E, R²¹ = Brom, R²² = Trifluormethyl, R²³ = Methyl; R² = Cl; R³ = Cl; R⁶ = H, X = O) bevorzugt, worin R⁴, R⁵, R⁷ und R⁸ die oben genannten Bedeutungen, insbesondere die als bevorzugt genannten Bedeutungen, aufweisen. Beispiele für derartige Verbindungen sind die Verbindungen I-Eb.1 bis I-Eb.600, in denen die Variablen R⁴, R⁵, R⁷ und R⁸ gemeinsam die in einer Zeile der Tabelle 1 angegebenen Bedeutungen aufweisen.

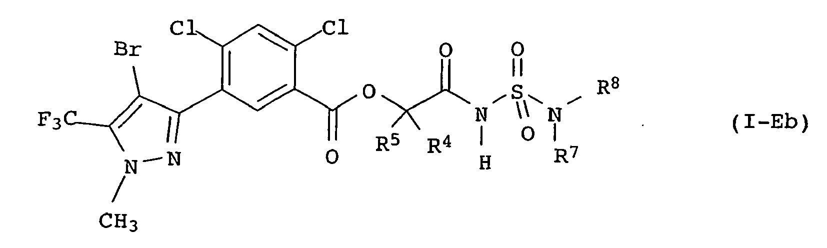

Unter den Verbindungen I-E sind die 3-Heterocyclyl substituierten Benzoesäure-Derivate der Formel I-Ec (≡ I mit R¹ = II-E, R²¹ = Chlor, R²² = Trifluormethyl, R²³ = Methyl; R² = H; R³ = Cl; R⁶ = H, X = O) bevorzugt, worin R⁴, R⁵, R⁷ und R⁸ die oben genannten Bedeutungen, insbesondere die als bevorzugt genannten Bedeutungen, aufweisen. Beispiele für derartige Verbindungen sind die Verbindungen I-Ec.1 bis I-Ec.600, in denen die Variablen R⁴, R⁵, R⁷ und R⁸ gemeinsam die in einer Zeile der Tabelle 1 angegebenen Bedeutungen aufweisen.

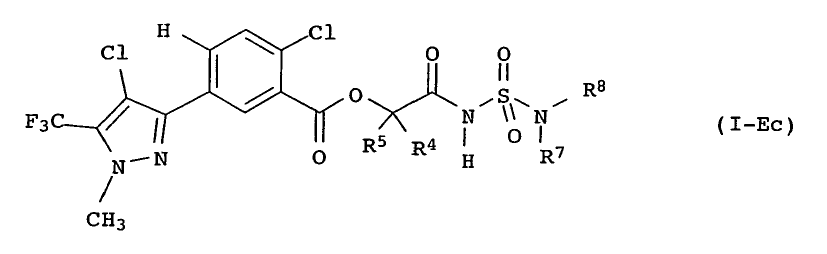

Unter den Verbindungen I-E sind die 3-Heterocyclyl substituierten Benzoesäure-Derivate der Formel I-Ed (≡ I mit R¹ = II-E, R²¹ = Chlor, R²² = Difluormethoxy, R²³ = Methyl; R² = F; R³ = Cl; R⁶ = H, X = O) bevorzugt, worin R⁴, R⁵, R⁷ und R⁸ die oben genannten Bedeutungen, insbesondere die als bevorzugt genannten Bedeutungen, aufweisen. Beispiele für derartige Verbindungen sind die Verbindungen I-Ed.1 bis I-Ed.600, in denen die Variablen R⁴, R⁵, R⁷ und R⁸ gemeinsam die in einer Zeile der Tabelle 1 angegebenen Bedeutungen aufweisen.

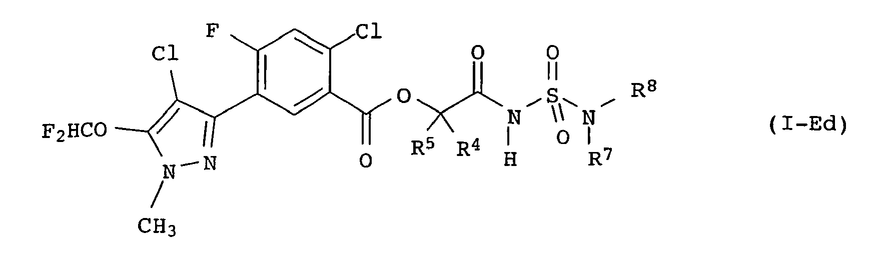

Unter den Verbindungen I-E sind die 3-Heterocyclyl substituierten Benzoesäure-Derivate der Formel I-Ee (≡ I mit R¹ = II-E, R²¹ = Chlor, R²² = Difluormethoxy, R²³ = Methyl; R² = H; R³ = Cl; R⁶ = H, X = O) bevorzugt, worin R⁴, R⁵, R⁷ und R⁸ die oben genannten Bedeutungen, insbesondere die als bevorzugt genannten Bedeutungen, aufweisen. Beispiele für derartige Verbindungen sind die Verbindungen I-Ee.1 bis I-Ee.600, in denen die Variablen R⁴, R⁵, R⁷ und R⁸ gemeinsam die in einer Zeile der Tabelle 1 angegebenen Bedeutungen aufweisen.

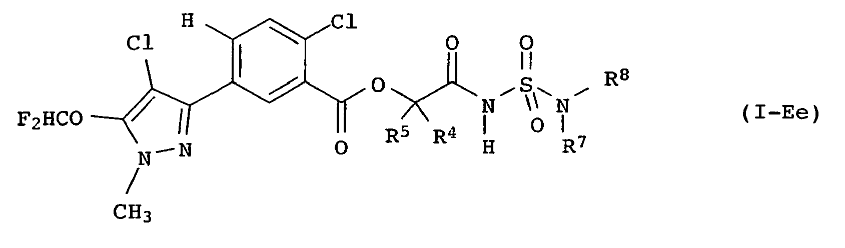

Unter den Verbindungen I-E sind die 3-Heterocyclyl substituierten Benzoesäure-Derivate der Formel I-Ef (≡ I mit R¹ = II-E, R²¹ = Brom, R²² = Difluormethoxy, R²³ = Methyl; R² = Cl; R³ = Cl; R⁶ = H, X = 0) bevorzugt, worin R⁴, R⁵, R⁷ und R⁸ die oben genannten Bedeutungen, insbesondere die als bevorzugt genannten Bedeutungen, aufweisen. Beispiele für derartige Verbindungen sind die Verbindungen I-Ef.1 bis I-Ef.600, in denen die Variablen R⁴, R⁵, R⁷ und R⁸ gemeinsam die in einer Zeile der Tabelle 1 angegebenen Bedeutungen aufweisen.

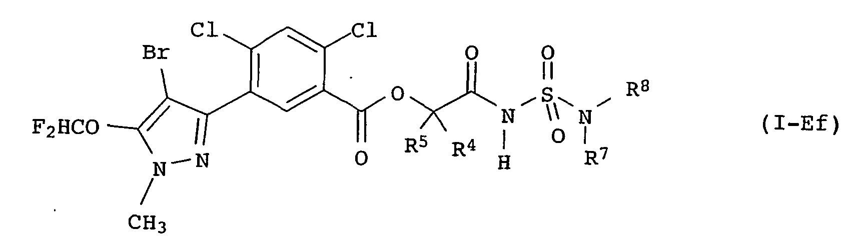

Unter den Verbindungen I-E sind die 3-Heterocyclyl substituierten Benzoesäure-Derivate der Formel I-Eg (≡ I mit R¹ = II-E, R²¹ = Chlor, R²² = Methylsulfonyl, R²³ = Methyl; R² = F; R³ = Cl; R⁶ = H, X = O) bevorzugt, worin R⁴, R⁵, R⁷ und R⁸ die oben genannten Bedeutungen, insbesondere die als bevorzugt genannten Bedeutungen, aufweisen. Beispiele für derartige Verbindungen sind die Verbindungen I-Eg.1 bis I-Eg.600, in denen die Variablen R⁴, R⁵, R⁷ und R⁸ gemeinsam die in einer Zeile der Tabelle 1 angegebenen Bedeutungen aufweisen.

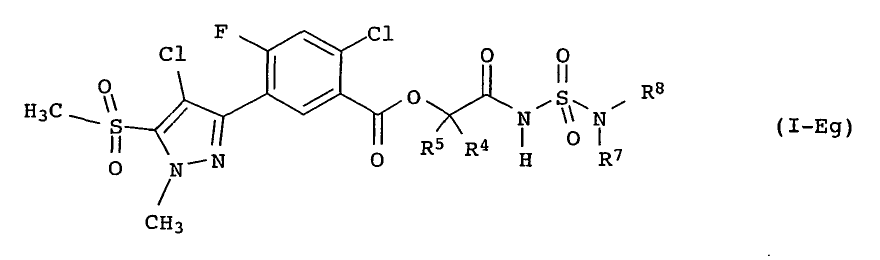

Unter den Verbindungen I-E sind die 3-Heterocyclyl substituierten Benzoesäure-Derivate der Formel I-Eh (≡ I mit R¹ = II-E, R²¹ = Brom, R²² = Methylsulfonyl, R²³ = Methyl; R² = Cl; R³ = Cl; R⁶ = H, X = O) bevorzugt, worin R⁴, R⁵, R⁷ und R⁸ die oben genannten Bedeutungen, insbesondere die als bevorzugt genannten Bedeutungen, aufweisen. Beispiele für derartige Verbindungen sind die Verbindungen I-Eh.1 bis I-Eh.600, in denen die Variablen R⁴, R⁵, R⁷ und R⁸ gemeinsam die in einer Zeile der Tabelle 1 angegebenen Bedeutungen aufweisen.

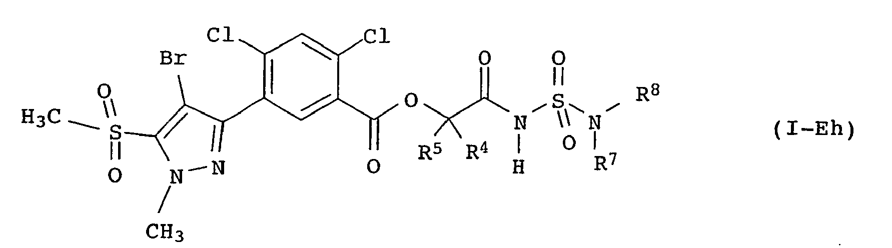

Unter den Verbindungen I-F sind die 3-Heterocyclyl substituierten Benzoesäure-Derivate der Formel I-Fa (≡ I mit R¹ = II-F, R²⁴ = Difluormethyl, R²⁵ = Methyl; R² = F; R³ = Cl; R⁶ = H, X = O) bevorzugt, worin R⁴, R⁵, R⁷ und R⁸ die oben genannten Bedeutungen, insbesondere die als bevorzugt genannten Bedeutungen, aufweisen. Beispiele für derartige Verbindungen sind die Verbindungen I-Fa.1 bis I-Fa.600, in denen die Variablen R⁴, R⁵, R⁷ und R⁸ gemeinsam die in einer Zeile der Tabelle 1 angegebenen Bedeutungen aufweisen.

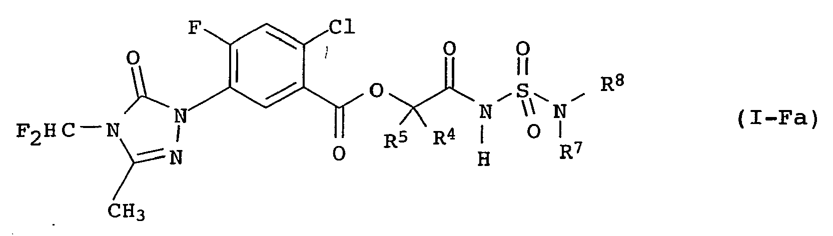

Unter den Verbindungen I-F sind die 3-Heterocyclyl substituierten Benzoesäure-Derivate der Formel I-Fb (≡ I mit R¹ = II-E, R²⁴ = Difluormethyl, R²⁵ = Methyl; R² = Cl; R³ = Cl; R⁶ = H, X = O) bevorzugt, worin R⁴, R⁵, R⁷ und R⁸ die oben genannten Bedeutungen, insbesondere die als bevorzugt genannten Bedeutungen, aufweisen. Beispiele für derartige Verbindungen sind die Verbindungen I-Fb.1 bis I-Fb.600, in denen die Variablen R⁴, R⁵, R⁷ und R⁸ gemeinsam die in einer Zeile der Tabelle 1 angegebenen Bedeutungen aufweisen.

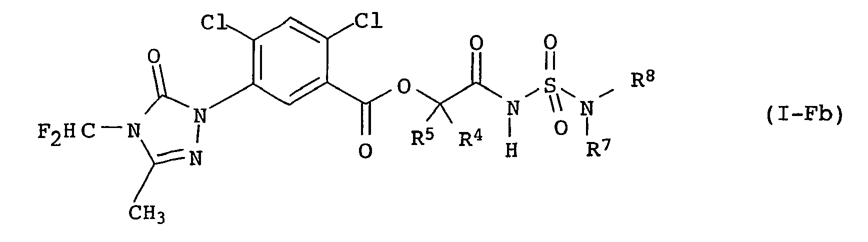

Unter den Verbindungen I-F sind die 3-Heterocyclyl substituierten Benzoesäure-Derivate der Formel I-Fc (≡ I mit R¹ = II-F, R²⁴, R²⁵ = (CH₂)₄; R² = F; R³ = Cl; R⁶ = H, X = 0) bevorzugt, worin R⁴, R⁵, R⁷ und R⁸ die oben genannten Bedeutungen, insbesondere die als bevorzugt genannten Bedeutungen, aufweisen. Beispiele für derartige Verbindungen sind die Verbindungen I-Fc.1 bis I-Fc.600, in denen die Variablen R⁴, R⁵, R⁷ und R⁸ gemeinsam die in einer Zeile der Tabelle 1 angegebenen Bedeutungen aufweisen.

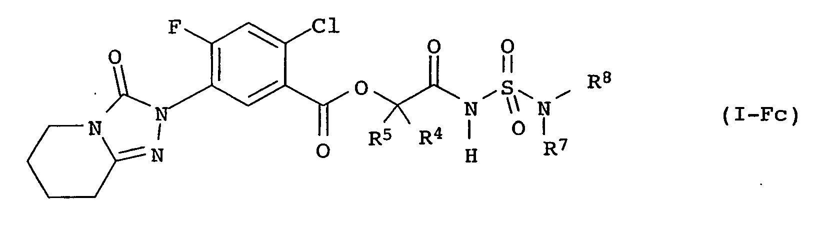

Unter den Verbindungen I-F sind die 3-Heterocyclyl substituierten Benzoesäure-Derivate der Formel I-Fd (≡ I mit R¹ = II-F, R²⁴, R²⁵ = (CH₂)₄; R² = Cl; R³ = Cl; R⁶ = H, X = O) bevorzugt, worin R⁴, R⁵, R⁷ und R⁸ die oben genannten Bedeutungen, insbesondere die als bevorzugt genannten Bedeutungen, aufweisen. Beispiele für derartige Verbindungen sind die Verbindungen I-Fd.1 bis I-Fd.600, in denen die Variablen R⁴, R⁵, R⁷ und R⁸ gemeinsam die in einer Zeile der Tabelle 1 angegebenen Bedeutungen aufweisen.

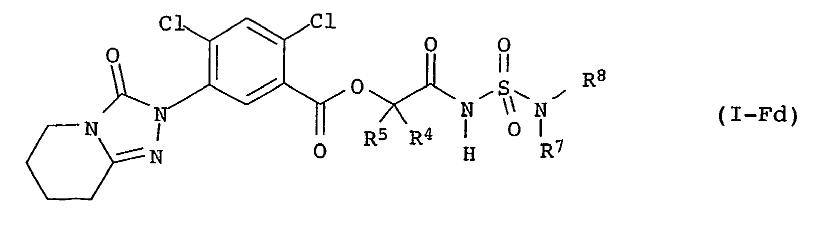

Unter den Verbindungen I-G sind die 3-Heterocyclyl substituierten Benzoesäure-Derivate der Formel I-Ga (≡ I mit R¹ = II-G, A¹, A² jeweils Sauerstoff; R² = F; R³ = Cl; R⁶ = H, X = 0) bevorzugt, worin R⁴, R⁵, R⁷ und R⁸ die oben genannten Bedeutungen, insbesondere die als bevorzugt genannten Bedeutungen, aufweisen. Beispiele für derartige Verbindungen sind die Verbindungen I-Ga.1 bis I-Ga.600, in denen die Variablen R⁴, R⁵, R⁷ und R⁸ gemeinsam die in einer Zeile der Tabelle 1 angegebenen Bedeutungen aufweisen.

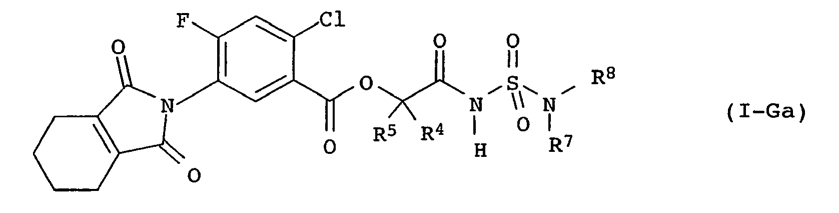

Unter den Verbindungen I-G sind die 3-Heterocyclyl substituierten Benzoesäure-Derivate der Formel I-Gb (≡ I mit R¹ = II-G, A¹, A² jeweils Sauerstoff; R² = H; R³ = Cl; R⁶ = H, X = O) bevorzugt, worin R⁴, R⁵, R⁷ und R⁸ die oben genannten Bedeutungen, insbesondere die als bevorzugt genannten Bedeutungen, aufweisen. Beispiele für derartige Verbindungen sind die Verbindungen I-Gb.1 bis I-Gb.600, in denen die Variablen R⁴, R⁵, R⁷ und R⁸ gemeinsam die in einer Zeile der Tabelle 1 angegebenen Bedeutungen aufweisen.

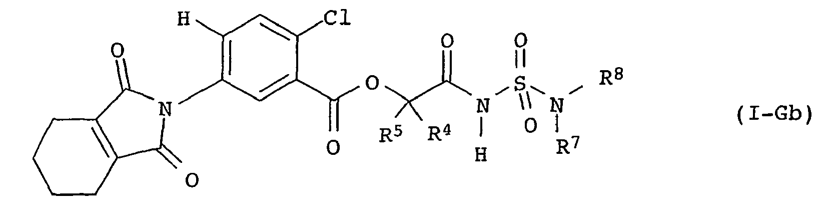

Unter den Verbindungen I-H sind die 3-Heterocyclyl substituierten Benzoesäure-Derivate der Formel I-Ha (≡ I mit R¹ = II-H, A³ und A⁴ jeweils Sauerstoff, R²⁶ = Difluormethyl, R²⁷ = Methyl; R² = F; R³ = Cl; R⁶ = H, X = 0) bevorzugt, worin R⁴, R⁵, R⁷ und R⁸ die oben genannten Bedeutungen, insbesondere die als bevorzugt genannten Bedeutungen, aufweisen. Beispiele für derartige Verbindungen sind die Verbindungen I-Ha.1 bis I-Ha.600, in denen die Variablen R⁴, R⁵, R⁷ und R⁸ gemeinsam die in einer Zeile der Tabelle 1 angegebenen Bedeutungen aufweisen.

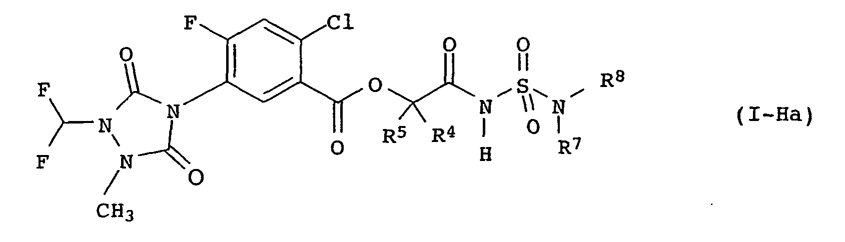

Unter den Verbindungen I-H sind die 3-Heterocyclyl substituierten Benzoesäure-Derivate der Formel I-Hb (≡ I mit R¹ = II-H, A³ und A⁴ jeweils Sauerstoff, R²⁶ und R²⁷ zusammen für Tetramethylen; R² = F; R³ = Cl; R⁶ = H, X = O) bevorzugt, worin R⁴, R⁵, R⁷ und R⁸ die oben genannten Bedeutungen, insbesondere die als bevorzugt genannten Bedeutungen, aufweisen. Beispiele für derartige Verbindungen sind die Verbindungen I-Hb.1 bis I-Hb.600, in denen die Variablen R⁴, R⁵, R⁷ und R⁸ gemeinsam die in einer Zeile der Tabelle 1 angegebenen Bedeutungen aufweisen.

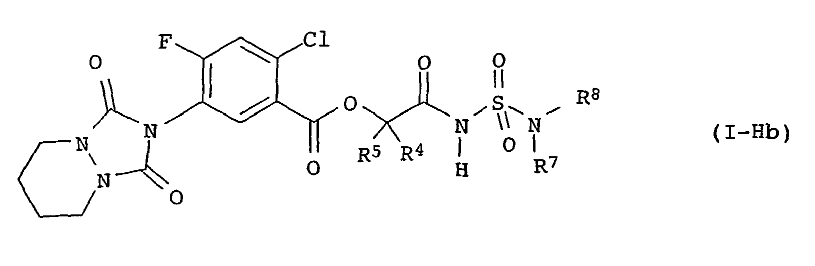

Unter den Verbindungen I-H sind die 3-Heterocyclyl substituierten Benzoesäure-Derivate der Formel I-Hc (≡ I mit R¹ = II-H, A³ und A⁴ jeweils Sauerstoff, R²⁶ und R²⁷ zusammen für Tetramethylen; R² = H; R³ = Cl; R⁶ = H, X = O) bevorzugt, worin R⁴, R⁵, R⁷ und R⁸ die oben genannten Bedeutungen, insbesondere die als bevorzugt genannten Bedeutungen, aufweisen. Beispiele für derartige Verbindungen sind die Verbindungen I-Hc.1 bis I-Hc.600, in denen die Variablen R⁴, R⁵, R⁷ und R⁸ gemeinsam die in einer Zeile der Tabelle 1 angegebenen Bedeutungen aufweisen.

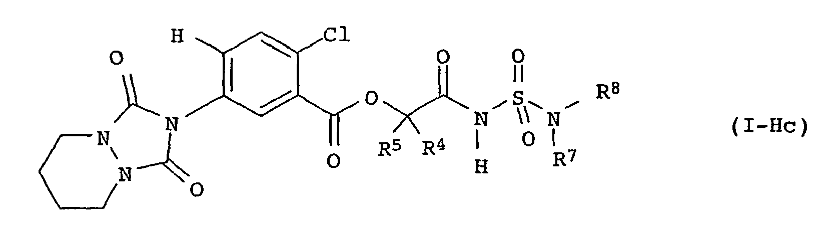

Die erfindungsgemäßen 3-Heterocyclyl substituierten Benzoesäure-Derivate können in Anlehnung an bekannte Verfahren hergestellt werden. Sofern keine gezielte Synthese zur Isolierung reiner Isomere durchgeführt wird, kann das Produkt als Isomerengemisch anfallen. Die Mischungen können gewünschtenfalls nach den hierfür üblichen Methoden wie Kristallisation oder Chromatographie, auch an einem optisch aktiven Adsorbat, in die weitgehend reinen Isomeren getrennt werden. Reine optisch aktive Isomere lassen sich beispielsweise auch aus den entsprechenden optisch aktiven Ausgangsmaterialien herstellen.

In der Regel stellt man die 3-Heterocyclyl substituierten Verbindungen der Formel I durch Umsetzung eines 3-Heterocyclyl substituiertes Benzoesäure-Derivat der allgemeinen Formel III, 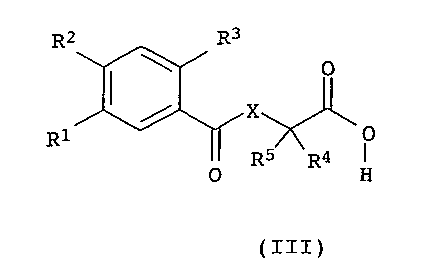 worin R¹, R², R³, R⁴, R⁵ und X die zuvor genannten Bedeutungen aufweisen, gegebenenfalls in Gegenwart eines Kupplungsmittels, oder das entsprechende Säurehalogenid zu III mit einem Sulfamid der Formel IV, 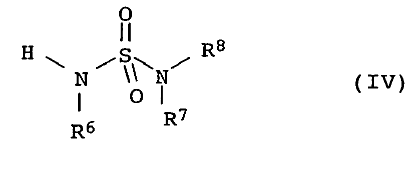 worin R⁶, R⁷ und R⁸ die zuvor genannten Bedeutungen aufweisen, her. Verfahren zur Aktivierung von Carbonsäuren sind beispielsweise aus Houben-Weyl, Methoden der organischen Chemie, Bd. E5 (1985), Teil 1, SS. 587 ff und Bd. E5 (1985), Teil II, SS. 934 ff bekannt. Die Umsetzung aktivierter Carbonsäuren III bzw. Carbonsäurehalogeniden von III kann in Analogie zu der in WO 01/83459 beschriebenen Herstellung von Carboxylsulfamiden erfolgen, z. B. nach der auf S. 31 f beschriebenen Weise.

Vorzugsweise aktiviert man zunächst die Carbonsäure III, indem man sie mit einem Kupplungsmittel umsetzt. Anschließend setzt man die aktivierte Carbonsäure III in der Regel ohne vorherige Isolierung mit dem Sulfamid IV um. Als Kupplungsmittel kommen beispielsweise N,N'-Carbonyldiimidazol oder Carbodiimide wie Dicyclohexylcarbodiimid in Betracht. Diese werden in der Regel wenigstens in äquimolarer Menge und bis zu einem vierfachen Überschuss, bezogen auf die Carbonsäure III, eingesetzt. Gegebenenfalls erwärmt man das erhaltene Reaktionsgemisch aus Carbonsäure III und Kupplungsmittel und lässt dann auf Raumtemperatur abkühlen. Üblicherweise führt man die Umsetzung in einem Lösungsmittel durch. Als Lösungsmittel kommen z. B. chlorierte Kohlenwasserstoffe wie Methylenchlorid, 1,2-Dichlorethan, Ether z. B. Dialkylether wie Diethylether, Methyl-tert.-butylether oder cyclische Ether wie Tetrahydrofuran oder Dioxan, Carbonsäureamide wie Dimethylformamid, N-Methyllactame wie N-Methylpyrrolidon, Nitrile wie Acetonitril, aromatische Kohlenwasserstoffe wie Toluol, aromatische Amine wie Pyridin oder Gemische hiervon in Betracht. Anschließend versetzt man mit dem Sulfamid IV. In der Regel löst man das Sulfamid IV in dem Lösungsmittel, das auch zur Aktivierung der Carbonsäure verwendet wurde.

Alternativ kann man auch die Carbonsäure III zunächst mit einem anorganischen Säurehalogenid, vorzugsweise einem Säurechlorid wie Thionylchlorid, Phosphorylchlorid, Phosphorpentachlorid, Oxalylchlorid oder Phosphortrichlorid in das entsprechende Säurehalogenid zu III überführen, gegebenenfalls das gebildete Säurehalogenid isolieren, und anschließend mit dem Sulfamid IV umsetzen. Gegebenenfalls steigert man die Reaktivität des Thionylchlorids durch Zusatz katalytischer Mengen Dimethylformamid. Üblicherweise setzt man das Halogenierungsmittel wenigstens in äquimolarer Menge, bezogen auf die Carbonsäure, ein. Der Reaktionspartner Thionylchlorid, Phosphortrichlorid oder Phosphorylchlorid kann gleichzeitig als Lösungsmittel fungieren. Geeignete Lösungsmittel sind ferner unter den Reaktionsbedingungen inerte Lösungsmittel beispielsweise chlorierte Kohlenwasserstoffe wie Methylenchlorid, 1,2-Dichlorethan, aromatische Kohlenwasserstoffe wie Benzol oder Toluol, aliphatische und cycloaliphatische Kohlenwasserstoffe wie Hexan, Petrolether, Cyclohexan und deren Gemische. Die Reaktionstemperatur liegt in der Regel zwischen Raumtemperatur und dem Siedepunkt des Lösungsmittels. Nach beendeter Umsetzung entfernt man in der Regel den Überschuss an Halogenierungsmittel. Anschließend versetzt man das so erhaltene Säurechlorid von III mit dem Sulfamid IV. In der Regel löst man das Sulfamid IV in dem Lösungsmittel, das auch zur Herstellung des Carbonsäurehalogenids verwendet wurde, sofern es sich bei dem Lösungsmittel nicht um eines der vorgenannten Säurehalogenide handelt.

Selbstverständlich können auch andere Verfahren zur Aktivierung der Carbonsäure verwendet werden. Solche Verfahren sind im Stand der Technik beschrieben.

Das molare Verhältnis von Carbonsäure III bzw. aktivierter Carbonsäure zu III bzw. des entsprechenden Säurechlorids zu III zu Sulfamid IV beträgt in der Regel wenigstens 0,9:1, vorzugsweise wenigstens 1:1. Gegebenenfalls kann es auch vorteilhaft sein, das Sulfamid IV in einem geringen Überschuss, beispielsweise in einem bis zu 30%igem Überschuss, bezogen auf die Carbonsäure III, einzusetzen.

Üblicherweise führt man die Umsetzung in Gegenwart einer Base, die vorzugsweise in äquimolarer Menge oder bis zu einem vierfachen Überschuss, bezogen auf Carbonsäure III, eingesetzt wird, durch. Geeignete Basen sind beispielsweise Amine wie 1,5-Dizabicyclo[4.3.0]non-5-en (DBN), 1,8-Diazabicyclo[5.4.0]undec-7-en (DBU), Pyridin oder Triethylamin. Gegebenenfalls kann es von Vorteil sein, die Umsetzung in Gegenwart einer katalytischen Menge 4-Dimethylaminopyridin (DMAP) durchzuführen. Der Zusatz an Base beträgt in der Regel 5 bis 10 mol-%, bezogen auf aktivierte Carbonsäure III.

In der Regel liegt die Reaktionstemperatur im Bereich von 0 °C bis zur Höhe des Siedepunktes des Reaktionsgemisches. Die Aufarbeitung kann in an sich bekannter Weise erfolgen.

Die Verbindungen der Formel IV sind nach an sich bekannten Verfahren erhältlich, zum Beispiel nach von G. Hamprecht in Angew. Chem. 93, 151 - 163 (1981) beschriebenen Verfahren oder nach den Verfahren, wie sie in WO 01/83459, DE 102 21 910.9 oder in Houben-Weyl, Bd. E11 (1985), S. 1019 beschrieben werden.

3-Heterocyclyl substituierte Benzoesäure-Derivate der allgemeinen Formel III sind im Stand der Technik bekannt oder lassen sich in Anlehnung an bekannte Verfahren, häufig ausgehend von den entsprechenden Estern zu III, herstellen.

Die Ester werden dann nach bekannten Verfahren durch Hydrolyse im sauren Milieu unter Verwendung von starken Mineralsäuren wie konzentrierte Salzsäure oder Schwefelsäure oder organischen Säuren wie Eisessig oder Gemischen davon in die entsprechenden Carbonsäuren III überführt. Alternativ lassen sich Ester auch im alkalischen Milieu unter Verwendung von Basen wie Alkalihydroxid, beispielsweise Natriumhydroxid oder Kaliumhydroxid in Gegenwart von Wasser hydrolysieren.

Als Lösungsmittel sowohl für die säure- als auch für die basenkatalysierte Hydrolyse von Estern kommen beispielsweise auch chlorierte aliphatische oder alicyclische Kohlenwasserstoffe wie Methylenchlorid oder 1,2-Dichlorethan oder Alkohole in Betracht. Bei der säurekatalysierten Hydrolyse ist üblicherweise der Reaktionspartner gleichzeitig auch das Lösungsmittel und wird daher im Überschuss, bezogen auf den Ester eingesetzt. Die Reaktionstemperatur liegt üblicherweise zwischen Raumtemperatur und dem Siedepunkt des Lösungsmittels.

Ester der Carbonsäure III, worin R¹ für einen heterocyclischen Rest der Formel II-A steht, sind beispielsweise aus US 6,207,830 und DE 197 41 411 bekannt. Ester von Carbonsäuren der Formel III, worin R¹ für einen heterocyclischen Rest der Formel II-C steht, sind aus WO 97/11059 bekannt. Ester von Carbonsäuren der Formel III, worin R¹ für einen Rest II-E steht, sind beispielsweise aus WO 92/06962 und JP 09059113 bekannt. Ester von Carbonsäuren der Formel II-F sind z. B. aus JP 61069776 bekannt. Verbindungen III, die in diesen Schriften nicht explizit beschrieben sind, lassen sich in Anlehnung an diese Verfahren herstellen.

Soweit die Ester von Carbonsäuren der Formel III nicht bekannt sind, kann man sie beispielsweise durch Umsetzung einer 3-Heterocyclyl substituierten Benzoesäure der Formel V, 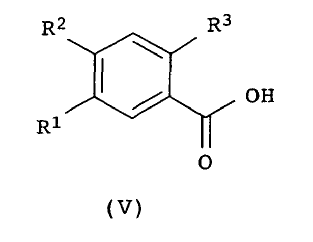 worin R¹, R² und R³ die zuvor genannten Bedeutungen aufweisen, mit einem α-Aminocarbonsäureester oder einem α-Hydroxycarbonsäureester der Formel VI,

HXC(R⁴)(R⁵)COOR' (VI)

worin X, R⁴ und R⁵ die zuvor genannten Bedeutungen aufweisen und R' für Niederalkyl steht, in Gegenwart eines wasserentziehenden Mittels wie N,N'-Carbonyldümidazol oder Dicyclohexylcarbodiimid herstellen. Alternativ kann man auch die Benzoesäure der Formel V zunächst in ihr Säurehalogenid überführen und anschließend mit der Verbindung der Formel VI umsetzen.

Die Reaktionsbedingungen entsprechen im Wesentlichen den zuvor für die Umsetzung von III mit IV genannten Bedingungen. Üblicherweise führt man die Umsetzung in einem Lösungsmittel durch. Geeignete Lösungsmittel sind chlorierte Kohlenwasserstoffe, wie Methylenchlorid, 1,2-Dichlorethan, Ether, wie Diethylether, Methyl-tert.-butylether, Tetrahydrofuran, Dioxan oder Gemische hiervon. Die Umsetzung mit einer Verbindung der Formel VI erfolgt üblicherweise bei einer Temperatur zwischen Raumtemperatur und dem Siedepunkt des Lösungsmittels. Abschließend hydrolysiert man den erhaltenen Ester von III, wobei man die gewünschte 3-Heterocyclyl substituierte Carbonsäure III erhält. Bezüglich der Durchführung der Hydrolyse sei auf das zuvor Gesagte verwiesen.

Die Carbonsäure V lässt sich beispielsweise nach WO 01/083459 oder dem darin zitierten Stand der Technik oder in Anlehnung an die darin beschriebenen Verfahren herstellen. Gegebenenfalls muss der im Stand der Technik beschriebene Ester nach bekannten Verfahren in die Carbonsäure V überführt werden. Bezüglich der säure- oder basenkatalysierten Hydrolyse von Estern sei auf das zuvor Gesagte verwiesen. Ausdrücklich verwiesen sei auf folgende Schriften:
WO 88/10254, WO 89/02891, WO 89/03825, WO 91/00278 (Verbindungen der Formel V beziehungsweise deren Ester, worin R¹ für einen heterocyclischen Rest der Formel II-A steht), EP 0 584 655, WO 00/050409 (die Ester der Verbindungen der Formel V, worin R¹ für einen heterocyclischen Rest der Formel II-B steht), WO 96/39392, WO 97/07104 (Verbindungen der Formel V und/oder der entsprechenden Ester zu V, worin R¹ für einen heterocyclischen Rest der Formel II-D steht), WO 92/06962 (Verbindungen der Formel V, worin R¹ für einen heterocyclischen Rest der Formel II-E).

Die Verbindungen I und deren landwirtschaftlich brauchbaren Salze eignen sich - sowohl als Isomerengemische als auch in Form der reinen Isomeren - als Herbizide. Die I enthaltenden herbiziden Mittel bekämpfen Pflanzenwuchs auf Nichtkulturflächen sehr gut. In Kulturen wie Weizen, Reis, Mais, Soja und Baumwolle wirken sie gegen Unkräuter und Schadgräser, ohne die Kulturpflanzen nennenswert zu schädigen. Dieser Effekt tritt vor allem bei niedrigen Aufwandmengen auf.

In Abhängigkeit von der jeweiligen Applikationsmethode können die Verbindungen I bzw. sie enthaltenden herbiziden Mittel noch in einer weiteren Zahl von Kulturpflanzen zur Beseitigung unerwünschter Pflanzen eingesetzt werden. In Betracht kommen beispielsweise folgende Kulturen:
Allium cepa, Ananas comosus, Arachis hypogaea, Asparagus officinalis, Beta vulgaris spec. altissima, Beta vulgaris spec. rapa, Brassica napus var. napus, Brassica napus var. napobrassica, Brassica rapa var. silvestris, Camellia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum, (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spec., Manihot esculenta, Medicago sativa, Musa spec., Nicotiana tabacum (N.rustica), Olea europaea, Oryza sativa, Phaseolus lunatus, Phaseolus vulgaris, Picea abies, Pinus spec., Pisum sativum, Prunus avium, Prunus persica, Pyrus communis, Ribes sylvestre, Ricinus communis, Saccharum officinarum, Secale cereale, Solanum tuberosum, Sorghum bicolor (s. vulgare), Theobroma cacao, Trifolium pratense, Triticum aestivum, Triticum durum, Vicia faba, Vitis vinifera und Zea mays.

Darüber hinaus können die Verbindungen I auch in Kulturen, die durch Züchtung einschließlich gentechnischer Methoden gegen die Wirkung von Herbiziden tolerant sind, verwandt werden.

Des Weiteren eignen sich die Verbindungen I und deren landwirtschaftlich brauchbaren Salze auch zur Desikkation und/oder Defoliation von Pflanzen.

Als Desikkantien eignen sie sich insbesondere zur Austrocknung der oberirdischen Teile von Kulturpflanzen wie Kartoffel, Raps, Sonnenblume und Sojabohnen. Damit wird ein vollständig mechanisches Beernten dieser wichtigen Kulturpflanzen ermöglicht.

Von wirtschaftlichem Interesse ist auch
- das zeitlich konzentrierte Abfallen von Früchten oder das Vermindern ihrer Haftfestigkeit an der Pflanze, beispielsweise bei Zitrusfrüchten, Oliven oder anderen Arten und Sorten von Kern-, Stein- und Schalenobst, da hierdurch die Ernte dieser Früchte erleichtert wird, sowie
- das kontrollierte Entblättern von Nutzpflanzen, insbesondere Baumwolle (Defoliation).

Das durch die Anwendung von erfindungsgemäßen Wirkstoffen der Formel I geförderte Abfallen beruht auf der Ausbildung von Trenngewebe zwischen Frucht- oder Blatt- und Sproßteil der Pflanzen.

Die Baumwolldefoliation ist von ganz besonderem wirtschaftlichem Interesse, da sie die Ernte erleichtert. Gleichzeitig führt die Verkürzung des Zeitintervalls, in dem die einzelnen Pflanzen reif werden, zu einer erhöhten Qualität des geernteten Fasermaterials.

Die Verbindungen I bzw. die sie enthaltenden herbiziden Mittel können beispielsweise in Form von direkt versprühbaren wässrigen Lösungen, Pulvern, Suspensionen, auch hochprozentigen wässrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen, Gießen oder Behandlung des Saatgutes bzw. Mischen mit dem Saatgut angewendet werden. Die Anwendungsformen richten sich nach den Verwendungszwecken; sie sollten in jedem Fall möglichst die feinste Verteilung der erfindungsgemäßen Wirkstoffe gewährleisten.

Die herbiziden Mittel enthalten eine herbizid wirksame Menge mindestens eines Wirkstoffes der Formel I und für die Formulierung von Pflanzenschutzmitteln übliche Hilfsstoffe.

Als inerte Hilfsstoffe kommen im Wesentlichen in Betracht:
Mineralölfraktionen von mittlerem bis hohem Siedepunkt wie Kerosin und Dieselöl, ferner Kohlenteeröle sowie Öle pflanzlichen oder tierischen Ursprungs, aliphatische, cyclische und aromatische Kohlenwasserstoffe, z.B. Paraffine, Tetrahydronaphthalin, alkylierte Naphthaline und deren Derivate, alkylierte Benzole und deren Derivate, Alkohole wie Methanol, Ethanol, Propanol, Butanol und Cyclohexanol, Ketone wie Cyclohexanon, stark polare Lösungsmittel, z.B. Amine wie N-Methylpyrrolidon und Wasser.

Wässrige Anwendungsformen können aus Emulsionskonzentraten, Suspensionen, Pasten, netzbaren Pulvern oder wasserdispergierbaren Granulaten durch Zusatz von Wasser bereitet werden. Zur Herstellung von Emulsionen, Pasten oder Öldispersionen können die 3-Heterocyclyl substituierten Benzoesäure-Derivate I als solche oder in einem öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenyl-, Tributylphenylpolyglykolether, Alkylarylpolyetheralkohole, Isotridecylalkohol, Fettalkoholethylenoxid-Kondensate, ethoxyliertes Rizinusöl, Polyoxyethylen- oder Polyoxypropylenalkylether, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löss, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte wie Getreidemehl, Baumrinden-, Holz- und Nußschalenmehl, Cellulosepulver oder andere feste Trägerstoffe.

Die Konzentrationen der Wirkstoffe I in den anwendungsfertigen Zubereitungen können in weiten Bereichen variiert werden. Die Formulierungen enthalten im allgemeinen etwa 0,001 bis 98 Gew.-%, vorzugsweise 0,01 bis 95 Gew.-%, mindestens eines Wirkstoffs I. Die Wirkstoffe werden dabei in einer Reinheit von 90 % bis 100 %, vorzugsweise 95 % bis 100 % (nach NMR-Spektrum) eingesetzt.

Die erfindungsgemäßen Verbindungen I können beispielsweise wie folgt formuliert werden:
I. 20 Gewichtsteile einer Verbindung I werden in einer Mischung gelöst, die aus 80 Gewichtsteilen alkyliertem Benzol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Rizinusöl besteht. Durch Ausgießen und feines Verteilen der Lösung in 100000 Gewichtsteilen Wasser erhält man eine wäßrige Dispersion, die 0,02 Gew.-% des Wirkstoffs enthält.
II. 20 Gewichtsteile einer Verbindung I werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Rizinusöl besteht. Durch Eingießen und feines Verteilen der Lösung in 100000 Gewichtsteilen Wasser erhält man eine wäßrige Dispersion, die 0,02 Gew.-% des Wirkstoffs enthält.
III. 20 Gewichtsteile einer Verbindung I werden in einer Mischung gelöst, die aus 25 Gewichtsteilen Cyclohexanon, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Rizinusöl besteht. Durch Eingießen und feines Verteilen der Lösung in 100000 Gewichtsteilen Wasser erhält man eine wäßrige Dispersion, die 0,02 Gew.-% des Wirkstoffs enthält.
IV. 20 Gewichtsteile einer Verbindung I werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser enthält man eine Spritzbrühe, die 0,1 Gew.-% des Wirkstoffs enthält.
V. 3 Gewichtsteile einer Verbindung I werden mit 97 Gewichtsteilen feinteiligem Kaolin vermischt. Man erhält auf diese Weise ein Stäubemittel, das 3 Gew.-% des Wirkstoffs enthält.
VI. 20 Gewichtsteile einer Verbindung I werden mit 2 Gewichtsteilen Calciumsalz der Dodecylbenzolsulfonsäure, 8 Gewichtsteilen Fettalkoholpolyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.
VII. 1 Gewichtsteil einer Verbindung I wird in einer Mischung gelöst, die aus 70 Gewichtsteilen Cyclohexanon, 20 Gewichtsteilen ethoxyliertem Isooctylphenol und 10 Gewichtsteilen ethoxyliertem Rizinusöl besteht. Man erhält ein stabiles Emulsionskonzentrat.
VIII. 1 Gewichtsteil einer Verbindung I wird in einer Mischung gelöst, die aus 80 Gewichtsteilen Cyclohexanon und 20 Gewichtsteilen Wettol® EM 31 (= nichtionischer Emulgator auf der Basis von ethoxyliertem Rizinusöl; BASF AG) besteht. Man erhält ein stabiles Emulsionskonzentrat.

Die Applikation der Wirkstoffe I bzw. der herbiziden Mittel kann im Vorauflauf- oder im Nachauflaufverfahren erfolgen. Es besteht auch die Möglichkeit, die herbiziden Mittel bzw. Wirkstoffe dadurch zu applizieren, dass mit den herbiziden Mitteln bzw. Wirkstoffen vorbehandeltes Saatgut einer Kulturpflanze ausgebracht wird. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, dass die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff I betragen je nach Bekämpfungsziel, Jahreszeit, Zielpflanzen und Wachstumsstadium 0,001 bis 3,0, vorzugsweise 0,01 bis 1,0 kg/ha aktive Substanz (a.S.).

Zur Verbreiterung des Wirkungsspektrums und zur Erzielung synergistischer Effekte können die 3-Heterocyclyl substituierte Benzoesäure-Derivate I mit zahlreichen Vertretern anderer herbizider oder wachstumsregulierender Wirkstoffgruppen gemischt und gemeinsam ausgebracht werden.

Beispielsweise kommen als Mischungspartner 1,2,4-Thiadiazole, 1,3,4-Thiadiazole, Amide, Aminophosphorsäure und deren Derivate, Aminotriazole, Anilide, Aryloxy-/Heteroaryloxyalkansäuren und deren Derivate, Benzoesäure und deren Derivate, Benzothiadiazinone, 2-(Hetaroyl/Aroyl)-1,3-cyclohexandione, Heteroaryl-Aryl-Ketone, Benzylisoxazolidinone, meta-CF₃-Phenylderivate, Carbamate, Chinolincarbonsäure und deren Derivate, Chloracetanilide, Cyclohexan-1,3-dionderivate, Diazine, Dichlorpropionsäure und deren Derivate, Dihydrobenzofurane, Dihydrofuran-3-one, Dinitroaniline, Dinitrophenole, Diphenylether, Dipyridyle, Halogencarbonsäuren und deren Derivate, Harnstoffe, 3-Phenyluracile, Imidazole, Imidazolinone, N-Phenyl-3,4,5,6-tetrahydrophthalimide, Oxadiazole, Oxirane, Phenole, Aryloxy- und Heteroaryloxyphenoxypropionsäureester, Phenylessigsäure und deren Derivate, 2-Phenylpropionsäure und deren Derivate, Pyrazole, Phenylpyrazole, Pyridazine, Pyridincarbonsäure und deren Derivate, Pyrimidylether, Sulfonamide, Sulfonylharnstoffe, Triazine, Triazinone, Triazolinone, Triazolcarboxamide und Uracile in Betracht.

Außerdem kann es von Nutzen sein, die Verbindungen I allein oder in Kombination mit anderen Herbiziden auch noch mit weiteren Pflanzenschutzmitteln gemischt, gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

Die folgenden Beispiele sollen die Erfindung erläutern, ohne sie einzuschränken.

### Herstellungsbeispiele

### Beispiel 1: (S)-2-[2-Chlor-5-(3-methyl-2,6-dioxo-4-trifluormethyl-3,6-dihydro-2H-pyrimidin-1-yl)-4-fluorbenzoyloxy]-propionsäure-N,N-dimethylsulfamid (S-Enantiomer von Verbindung I-Aa. 242)

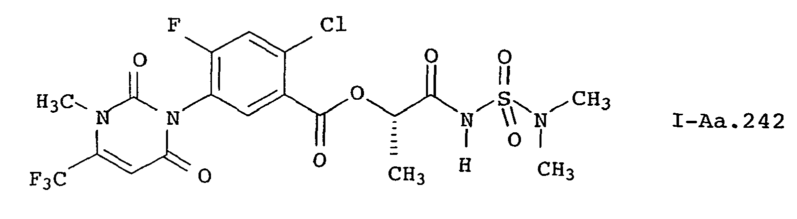

### 1.1: 2-Chlor-4-fluor-5-(3-methyl-2,6-dioxo-4-trifluormethyl-3,6-dihydro-2H-pyrimidin-1-yl)benzoesäure

Man löste 13,9 g (34 mmol) 2-Chlor-4-fluor-5-(3-methyl-2,6-dioxo-4-trifluormethyl-3,6-dihydro-2H-pyrimidin-1-yl)benzoesäureisopropylester (CAS-Nr. 105756-82-9, US 5,176,735, US 4,943,309, WO 88/10254) in 100 ml Eisessig und 100 ml konz. HCl und erhitzte 15 Stunden auf 70 °C. Man entfernte die Essigsäure im Vakuum, nahm den Rückstand in Wasser auf und saugte den ausgefallenen Niederschlag. Nach dem Trocknen erhielt man 11,3 g der Uracilcarbonsäure, die ohne weitere Reinigung im nächsten Schritt eingesetzt wurde.

¹H-NMR (DMSO-d₆) δ (ppm) = 8,1 (d, 1 H), 7,8 (d, 1 H), 6,6 (s, 1 H), 3,4 (s, 3 H).

### 1.2: (S)-2-[2-Chlor-5-(3-methyl-2,6-dioxo-4-trifluormethyl-3,6-dihydro-2H-pyrimidin-1-yl)-4-fluorbenzoyl]-propionsäuremethylester

Man erhitzte 5,0 g (13,64 mmol) Uracilcarbonsäure aus 1.1 in 50 ml Thionylchlorid 3 h zum Rückfluss und entfernte anschließend nicht umgesetztes Thionylchlorid im Vakuum. Danach löste man das erhaltene Säurechlorid in 50 ml Methylenchlorid und tropfte unter Rühren die erhaltene Lösung bei 0-5 °C zu einer Lösung von 1,6 g (15,01 mmol) (S)-Milchsäuremethylester, 0,2 g (1,36 mmol) 4-Dimethylaminopyridin (DMAP) und 1,7 g (16,37 mmol) Triethylamin in 80 ml CH₂Cl₂. Man ließ auf Raumtemperatur erwärmen und rührte 16 Stunden bei Raumtemperatur nach. Anschließend engte man das Reaktionsgemisch ein und chromatographierte an Kieselgel mit Cyclohexan/Essigester 70/30. Man verdampfte das Lösungsmittel unter vermindertem Druck und erhielt 5,85 g des Esters.

¹H-NMR (DMSO-d₆) δ (ppm) = 8,0 (d, 1 H), 7,4 (d, 1 H), 6,4 (s, 1 H), 5,4 (q, 1 H), 4,8 (s, 3 H), 3,6 (s, 3 H), 1,5 (d, 3 H).

### 1.3: (S)-2-[2-Chlor-5-(3-methyl-2,6-dioxo-4-trifluormethyl-3,6-dihydro-2H-pyrimidin-1-yl)-4-fluorbenzoyl]-propionsäure

Man versetzte 3,6 g (8 mmol) Ester aus 1.2 mit 25 ml Eisessig und 25 ml conz. HCl, erwärmte 4 Stunden auf 60 °C und rührte 8 Stunden bei Raumtemperatur nach. Man entfernte die Essigsäure im Vakuum, verdünnte das Reaktionsgemisch mit Wasser und extrahierte dreimal mit je etwa 150 ml Essigsäureethylester. Anschließend trocknete man die vereinigten organischen Phasen über Na₂SO₄ und engte im Vakuum ein, wobei man 3,3 g Säure erhielt.

¹H-NMR (DMSO-d₆) δ (ppm) = 8,0 (d, 1 H), 7,4 (d, 1 H), 6,4 (s, 1 H), 5,4 (q, 1 H), 3,5 (s, 3 H), 1,6 (d, 3 H).

### 1.4: (S)-2-[2-Chlor-5-(3-methyl-2,6-dioxo-4-trifluormethyl-3,6-dihydro-2H-pyrimidin-1-yl)-4-fluorbenzoyloxy]-propionsäure-N,N-dimethylsulfamid

Man erhitzte 0,45 g (1,03 mmol) Säure aus 1.3 in 10 ml Thionylchlorid 3 Stunden zum Rückfluss, entfernte anschließend überschüssiges Thionylchlorid im Vakuum und löste das erhaltene Säurechlorid in etwa 5 ml CH₂Cl₂. Diese Lösung wurde bei etwa 5 °C zu einer Lösung von 0,13 g (1,03 mmol) N,N-Dimethylsulfamid, 0,23 g (2,23 mmol) Triethylamin und einer katalytischen Menge DMAP in 20 ml CH₂Cl₂ getropft. Man rührte 14 Stunden bei Raumtemperatur, engte das Reaktionsgemisch im Vakuum ein. Man nahm den Rückstand in Essigsäureethylester auf und wusch mit etwa 200 ml 10%-iger Salzsäure. Die Chromatographie an Kieselgel mit Cyclohexan/Essigester 70/30 ergab 0,16 g der Titelverbindung mit Schmp. 207-208 °C.

¹H-NMR: siehe Tabelle 2

### Beispiel 2: 2-[2-Chlor-5-(3-methyl-2,6-dioxo-4-trifluormethyl-3,6-dihydro-2H-pyrimidin-1-yl)benzoyloxy]-2-methylpropionsäure-N-methyl-N-allylsulfamid (Verbindung I-Ab.465)

Man löste 0,33 g (2,2 mmol) N-Methyl-N-allylsulfamid, 0,27 g 4-Dimethylaminopyridin und 0,64 ml Triethylamin in 10 ml Dichlormethan. Danach tropfte man eine Lösung von 0,98 g (2,2 mmol) 2-[2-Chlor-5-(3-methyl-2,6-dioxo-4-trifluormethyl-3,6-dihydro-2H-pyrimidin-1-yl)benzoyloxy]-2-methylpropionsäurechlorid (CAS-Nr. 160152-72-7) in Dichlormethan zu. Man rührte die Lösung 3 Tage, engte ein und nahm den Rückstand in Ethylacetat auf. Die organische Phase wusch man mit 10 %iger Salzsäure und Wasser, trocknete über Natriumsulfat, filtrierte das Trockenmittel ab und engte ein. Die Säulenchromatographie an Kieselgel (Eluens: Cyclohexan/Ethylacetat 2:1) ergab 0,21 g der Titelverbindung mit einem Schmelzpunkt von 161-164 °C.

### Beispiel 3: (S)-2-[2-Chlor-5-(3-methyl-2,6-dioxo-4-trifluormethyl-3,6-dihydro-2H-pyrimidin-1-yl)benzoyloxy]propionsäure-N-methyl-N-allylsulfamid (S-Enantiomer von Verbindung I-Ab.265)

### 3.1: (S)-2-[2-Chlor-5-(3-methyl-2,6-dioxo-4-trifluormethyl-3,6-dihydro-2H-pyrimidin-1-yl)benzoyloxy]propionsäuremethylester

Man löste 7,0 g (20 mmol) 2-[2-Chlor-5-(3-methyl-2,6-dioxo-4-trifluormethyl-3,6-dihydro-2H-pyrimidin-1-yl)benzoesäure (CAS-Nr. 120890-58-6) in 50 ml Thionylchlorid. Danach erhitzte man das Reaktionsgemisch 3 Stunden am Rückfluss und engte die so erhaltene Lösung des Säurechlorids ein. Anschließend löste man 2,3 g (22 mmol) (S)-Milchsäuremethylester, 2,46 g (20 mmol) 4-Dimethylaminopyridin und 2,44 g (20 mmol) Triethylamin in 50 ml Dichlormethan und tropfte bei 0 °C eine Lösung des zuvor erhaltenen Säurechlorids in Dichlormethan zu. Das Reaktionsgemisch rührte man 16 Stunden bei Raumtemperatur und engte danach die Lösung ein. Die Säulenchromatographie an Kieselgel (Eluens: Cyclohexan/Ethylacetat 2:1) ergab 7,0 g (S)-2-[2-Chlor-5-(3-methyl-2,6-dioxo-4-trifluormethyl-3,6-dihydro-2H-pyrimidin-1-yl)benzoyloxy]propionsäuremethylester mit einem Schmelzpunkt von 59-60 °C.

### 3.2: (S)-2-[2-Chlor-5-(3-methyl-2,6-dioxo-4-trifluormethyl-3,6-dihydro-2H-pyrimidin-1-yl)benzoyloxy]propionsäure

Man löste 7,0 g (16 mmol) (S)-2-[2-Chlor-5-(3-methyl-2,6-dioxo-4-trifluormethyl-3,6-dihydro-2H-pyrimidin-1-yl)benzoyloxy]propionsäuremethylester aus Beispiel 3.1 in 50 ml Essigsäure, versetzte mit 50 ml konz. Salzsäure und erhitzte die Lösung 4 Stunden am Rückfluss. Die Essigsäure destillierte man weitgehend ab und goss die verbleibende Lösung auf Eiswasser. Die wässrige Phase extrahierte man dreimal mit Ethylacetat, trocknete die organische Phase über Natriumsulfat, filtrierte das Trockenmittel ab und engte bis zur Trockne ein, wobei man 5,7 g (S)-2-[2-Chlor-5-(3-methyl-2,6-dioxo-4-trifluormethyl-3,6-dihydro-2H-pyrimidin-1-yl)benzoyloxy]propionsäure erhielt.

### 3.3: (S)-2-[2-Chlor-5-(3-methyl-2,6-dioxo-4-trifluormethyl-3,6-dihydro-2H-pyrimidin-1-yl)benzoyloxy]propionsäurechlorid

Man löste 5,7 g (14 mmol) (S)-2-[2-Chlor-5-(3-methyl-2,6-dioxo-4-trifluormethyl-3,6-dihydro-2H-pyrimidin-1-yl)benzoyloxy]propionsäure aus Beispiel 3.2 in 50 ml Thionylchlorid und erhitzte das Reaktionsgemisch 3,5 Stunden am Rückfluss. Man ließ abkühlen und engte die Lösung ein, wobei man 5,9 g (S)-2-[2-Chlor-5-(3-methyl-2,6-dioxo-4-trifluormethyl-3,6-dihydro-2H-pyrimidin-1-yl)benzoyloxy]propionsäurechlorid erhielt.

### 3.4: (S)-2-[2-Chlor-5-(3-methyl-2,6-dioxo-4-trifluormethyl-3,6-dihydro-2H-pyrimidin-1-yl)benzoyloxy]propionsäure-N-methyl-N-allylsulfamid

Man löste 0,33 g (2,2 mmol) N-Methyl-N-allylsulfamid, 0,27 g 4-Dimethylaminopyridin und 0,67 ml Triethylamin in 10 ml Dichlormethan und tropfte eine Lösung von 0,98 g (2,2 mmol) (S)-2-[2-Chlor-5-(3-methyl-2,6-dioxo-4-trifluormethyl-3,6-dihydro-2H-pyrimidin-1-yl)benzoyloxy]propionsäurechlorid aus Beispiel 3.3 in 10 ml Dichlormethan zu. Die Lösung rührte man 16 Stunden, engte danach ein und löste den erhaltenen Rückstand in Ethylacetat. Die organische Phase wusch man mit 10%iger Salzsäure und Wasser, trocknete die organische Phase über Natriumsulfat, filtrierte vom Trockenmittel ab und engte ein. Die Säulenchromatographie an Kieselgel ergab 0,26 g der Titelverbindung.

### Beispiel 4: 2-[2-Chlor-5-(3-methyl-2,6-dioxo-4-trifluormethyl-3,6-dihydro-2H-pyrimidin-1-yl)-4-fluorbenzoyloxy]-2-methylpropionsäure-N-methyl-N-allylsulfamid (Verbindung I-Aa.442)

### 4.1: 2-[2-Chlor-5-(3-methyl-2,6-dioxo-4-trifluormethyl-3,6-dihydro-2H-pyrimidin-1-yl)-4-fluorbenzoyloxy]-2-methylpropionsäuremethylester

Man löste 0,61 g (5,2 mmol) 2-Hydroxy-2-methylpropionsäuremethylester, 70 mg (0,5 mmol) 4-N-Pyrrolidinopyridin und 0,87 ml (6,2 mmol) Triethylamin in 50 ml Tetrahydrofuran und tropfte danach eine Lösung von 2 g (5,2 mmol) des Säurechlorids aus Beispiel 1.2 in 50 ml Tetrahydrofuran zu. Man rührte die Lösung 16 Stunden, engte ein und löste danach den Rückstand in Ethylacetat. Die organische Phase wusch man mit 10%iger Zitronensäure und Wasser, trocknete die organische Phase über Natriumsulfat, filtrierte das Trockenmittel ab und engte ein. Die Säulenchromatographie an Kieselgel (Eluens: Cyclohexan/Ethylacetat 2:1) ergab 1,0 g 2-[2-Chlor-5-(3-methyl-2,6-dioxo-4-trifluormethyl-3,6-dihydro-2H-pyrimidin-1-yl)-4-fluorbenzoyloxy]-2-methylpropionsäuremethylester.

### 4.2: 2-[2-Chlor-5-(3-methyl-2,6-dioxo-4-trifluormethyl-3,6-dihydro-2H-pyrimidin-1-yl)-4-fluorbenzoyloxy]-2-methylpropionsäure

Man löste 1,0 g (2,1 mmol) 2-[2-Chlor-5-(3-methyl-2,6-dioxo-4-trifluormethyl-3,6-dihydro-2H-pyrimidin-1-yl)-4-fluorbenzoyloxy]-2-methylpropionsäuremethylester aus Beispiel 4.1 in 50 ml Essigsäure, gab 50 ml konz. Salzsäure zu und erhitzte das Reaktionsgemisch 5 Stunden am Rückfluss. Die Essigsäure destillierte man weitgehend ab und goss die verbleibende Lösung in Eiswasser. Man filtrierte den ausgefallenen Niederschlag ab und trocknete, wobei man 0,65 g 2-[2-Chlor-5-(3-methyl-2,6-dioxo-4-trifluormethyl-3,6-dihydro-2H-pyrimidin-1-yl)-4-fluorbenzoyloxy]-2-methylpropionsäure erhielt.

### 4.3: 2-[2-Chlor-5-(3-methyl-2,6-dioxo-4-trifluormethyl-3,6-dihydro-2H-pyrimidin-1-yl)-4-fluorbenzoyloxy]-2-methylpropionsäure-N,N-dimethylsulfamid

Man löste 0,65 g (1,4 mmol) 2-[2-Chlor-5-(3-methyl-2,6-dioxo-4-trifluormethyl-3,6-dihydro-2H-pyrimidin-1-yl)-4-fluorbenzoyloxy]-2-methylpropionsäure aus Beispiel 4.2 in 30 ml Thionylchlorid, erhitzte das Reaktionsgemisch 3 Stunden am Rückfluss und engte danach die erhaltene Lösung des Säurechlorids ein. 0,18 g (1,4 mmol) N,N-Dimethylsulfamid, 0,07 g (1,4 mmol) 4-Dimethylaminopyridin und 0,35 ml (3,5 mmol) Triethylamin löste man in 10 ml Dichlormethan und tropfte danach eine Lösung des zuvor hergestellten Säurechlorids in 10 ml Dichlormethan zu. Man rührte die Lösung 16 Stunden, engte anschließend ein und löste danach den Rückstand in Ethylacetat. Die organische Phase wusch man mit 10%iger Salzsäure und Wasser, trocknete die organische Phase über Natriumsulfat, filtrierte das Trockenmittel ab und engte ein. Die Säulenchromatographie an Kieselgel (Eluens: Cyclohexan/Ethylacetat 2:1) ergab 0,30 g der Titelverbindung mit einem Schmelzpunkt von 211-213 °C.

In der nachfolgenden Tabelle 2 sind neben den vorstehend beschriebenen Benzoesäure-Derivaten der Formel I noch weitere Verbindungen der Formel I aufgeführt, die auf analoge Weise hergestellt wurden.

**Tabelle 2:**

| Nr. | ¹H-NMR δ [ppm], | Schmp. [°C] |
|---|---|---|
| S-Enantiomer der Verbindung I-Aa.242 | (CDCl₃): 8,7 (br., 1 H), 8,0 m, 1 H), 7,5 (d, 1 H), 6,4 (s, 1 H), 5,4 (m, 1 H), 3,5 (s, 3 H), 2,9 (s, 6 H), 1,6 (d, 3 H). | 207 - 208 |
| S-Enantiomer der Verbindung I-Aa.243 | (CDCl₃): 8,7 (br., 1 H), 8,0 m, 1 H), 7,5 (d, 1 H), 6,4 (s, 1 H), 5,4 (m, 1 H), 3,5 (s, 3 H), 3,4 (q, 2 H), 2,9 (s, 3 H), 1,6 (d, 3 H9, 1,3 (t, 3 H), | 170 - 171 |
| S-Enantiomer der Verbindung I-Aa.246 | (CDCl₃): 8,7 (br., 1 H), 8,0 m, 1 H), 7,5 (d, 1 H), 6,4 (s, 1 H), 5,4 (m, 1 H), 4,2 (m, 1 H), 3,5 (s, 3 H), 2,9 (s, 3 H), 1,6 (d, 3 H), 1,3 (d, 6 H). | 164 - 165 |
| S-Enantiomer der Verbindung bindung | (CDCl₃) : 8,7 ( br., 1 H), 8,0 m, 1 H), 7,5 (d, 1 H), 6,4 (s, 1 H), 5,4 (m, 1 H), 3,6-3,4 (m, 7 H), 3,3 (s, 3 H), 2,9 (s, 3 H), 1,6 (d, 3 H). | 132 - 134 132 - 134 |
| S-Enantiomer der Verbindung bindung | (CDCl₃): 8,7 (br., 1 H), 8,0 m, 1 H), 7,5 (d, 1H), 6,4 (s, 1 H), 5,9-5,8 (m, 1 H), 5,5-5;1 (m, 3 H), 3,9 d, 1 H), 3,5 (s, 3 5, 5 - 5 ; 1 (m, 3 H), 3,9 d, 1 H), 3, 5 (s, 3 H), 2,9 (s, 3 H), 1,6 (d, 3 H). | 129 - 130 129 - 130 |
| S-Enander Verbindung bindung | (CDCl₃) : 8,7 (br., 1 H), 8,0 m, 1 H), 7,5 (d, 1 H), 6,4 (s, 1 H), 5,4 (m, 1 H), 4,2 (m, 2 H), 3,5 (s, 3 H), 2,9 (s, 3 H), 2,3 (m, 1 H), 1,6 (d, 3 H). | |
| I-Ab.465 | (DMSO-d₆): 11,7 (br. s, 1H), 7,9 (m, 1H), 7,7 (m, 1H), 7,6 (m, 1H), 6,6 (s, 1H), 5,8 (m, 1H), 5,3 - 5,2 (m, 2H), 3,8 (d, 2H), 3,4 (s, 3H), 2,8 (s, 3H), 1,6 (s, 6H). | 161 - 164 |
| I-Ab.469 | (DMSO-d₆): 11,7 (br. s, 1H), 7,9 (m, 1H), 7,7 (m, 1H), 7,6 (m, 1H), 6,6 (s, 1H), 4,1 (d, 2H), 3,4 (s, 3H), 3,3 (t, 3H), 2,8 (s, 3H), 1,6 (s, 6H). | |
| I-Ab.442 | (DMSO-d₆): 11,7 (br. s, 1H), 7,9 (m, 1H), 7,7 (m, 1H), 7,6 (m, 1H), 6,6 (s, 1H), 4,0 (m, 1H), 3,4 (s, 3H), 2,8 (s, 6H), 1,6 (s, 6H). | |
| S-Enantiomer von Verbindung Nr. I-Ab.248 | | Öl |
| S-Enantiomer von Verbindung I-Ab.242 | | Öl |
| S-Enantiomer von Verbindung I-Ab.243 | | Öl |
| S-Enantiomer von Verbindung I-Ab.265 | | Öl |
| I-Ab.443 | | 193-194 |
| I-Ab.446 | | 177-179 |
| I-Ab.448 | | 140-142 |
| I-Aa.442 | (DMSO-d₆): 11,63 (s, 1 H), 8,13 (d, 1 H), 7,91 (d, 1 H), 6,63 (s, 1 H), 3,42 (s, 3 H), 2,84 (s, 6 H), 1,61 (s, 6 H) | 211-213 |

### Anwendungsbeispiele

Die herbizide Wirkung der 3-Heterocyclyl substituierten Benzoesäure-Derivate I ließ sich durch die folgenden Gewächshausversuche zeigen:

Als Kulturgefäße dienten Plastikblumentöpfe mit lehmigem Sand mit etwa 3,0 % Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern, und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zweck der Nachauflaufbehandlung wurden die Testpflanzen je nach Wuchsform erst bis zu einer Wuchshöhe von 3 bis 15 cm angezogen und erst dann mit den in Wasser suspendierten oder emulgierten Wirkstoffen behandelt. Die Testpflanzen wurden dafür entweder direkt gesät und in den gleichen Gefäßen aufgezogen oder sie wurden erst als Keimpflanzen getrennt angezogen und einige Tage vor der Behandlung in die Versuchsgefäße verpflanzt. Die Aufwandmenge für die Nachauflaufbehandlung betrug 7,8 oder 3,9 g/ha a.S. (aktive Substanz).

Die Pflanzen wurden artenspezifisch bei Temperaturen von 10 - 25°C bzw. 20 - 35°C gehalten. Die Versuchsperiode erstreckte sich über 2 bis 4 Wochen. Während dieser Zeit wurden die Pflanzen gepflegt, und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| Lateinischer Name | Deutscher Name | Englischer Name |
|---|---|---|
| Amaranthus retrof lexus | Zurückgekrümmter Fuchsschwanz | redroot pigweed |
| Chenopodium album | Weißer Gänsefuß | lambsquarters (goosefoot) |

Das S-Enantiomer der Verbindung I-Aa.246 zeigte im Nachauflaufverfahren eine hervorragende herbizide Wirkung gegen die o. g. Pflanzen.

### Anwendungsbeispiele (desikkante/defoliante Wirksamkeit)

Als Testpflanzen dienten junge, 4-blättrige (ohne Keimblätter) Baumwollpflanzen, die unter Gewächshausbedingungen angezogen wurden (rel. Luftfeuchtigkeit 50 bis 70 %; Tag-/Nachttemperatur 27/20°C).

Die jungen Baumwollpflanzen wurden tropfnass mit wässrigen Aufbereitungen der Wirkstoffe (unter Zusatz von 0,15 Gew.-% des Fettalkoholalkoxylats Plurafac® LF 700¹⁾, bezogen auf die Spritzbrühe) blattbehandelt. Die ausgebrachte Wassermenge betrug umgerechnet 1000 1/ha. Nach 13 Tagen wurde die Anzahl der abgeworfenen Blätter und der Grad der Entblätterung in % bestimmt.
¹⁾ ein schaumarmes, nichtionisches Tensid der BASF AG

Bei den unbehandelten Kontrollpflanzen trat kein Blattfall auf.

## Patentansprüche

1. 3-Heterocyclyl substituierte Benzoesäure-Derivate der allgemeinen Formel I, 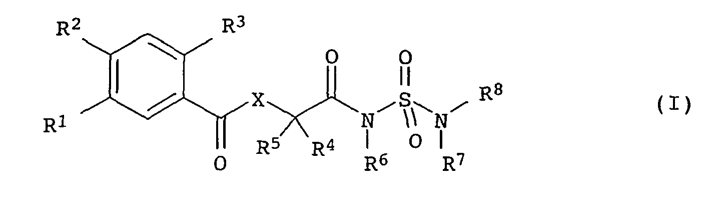 worin die Variablen die folgenden Bedeutungen haben:
X Sauerstoff oder NR⁹,
R¹ heterocylischer Rest der allgemeinen Formeln II-A bis II-H, 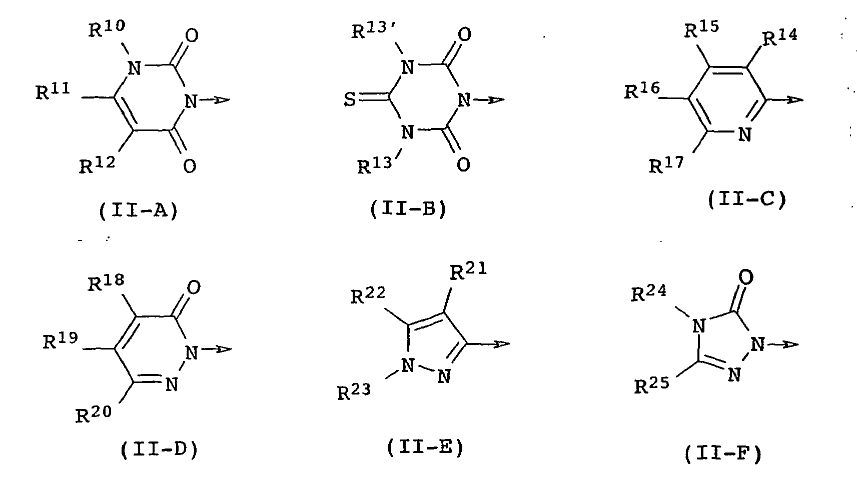 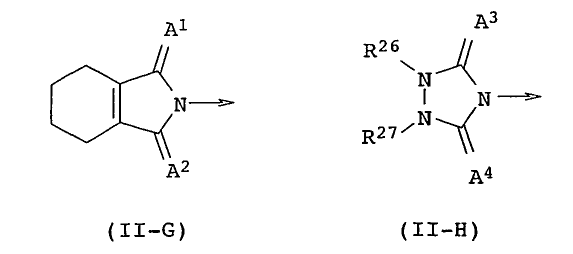
R² Wasserstoff oder Halogen,
R³ Halogen oder Cyano,
R⁴, R⁵ unabhängig voneinander Wasserstoff, C₁-C₄-Alkyl oder C₁-C₄-Alkoxy, oder R⁴ und R⁵ stehen gemeinsam für eine Gruppe =CH₂,
R⁶ Wasserstoff, C₁-C₄-Alkyl oder C₁-C₄-Alkoxy,
R⁷, R⁸ unabhängig voneinander Wasserstoff, C₁-C₆-Alkyl, C₃-C₆-Alkenyl, C₃-C₆-Alkinyl, C₁-C₄-Halogenalkyl, C₁-C₄-Alkoxy-C₁-C₄-alkyl, C₁-C₄-Alkylthio-C₁-C₄-alkyl, C₁-C₄-Alkylsulfinyl-C₁-C₄-alkyl, C₁-C₄-Alkylsulfonyl-C₁-C₄-alkyl, Cyano-C₁-C₄-alkyl, C₁-C₄-Alkoxycarbonyl-C₁-C₄-alkyl, Amino-C₁-C₄-alkyl, C₁-C₄-Alkylamino-C₁-C₄-alkyl, Di(C₁-C₄-alkyl)amino-C₁-C₄-alkyl, Aminocarbonyl-C₁-C₄-alkyl, (C₁-C₄-Alkylamino)carbonyl-C₁-C₄-alkyl, Di(C₁-C₄-alkyl)aminocarbonyl-C₁-C₄-alkyl, Phenyl oder C₁-C₄-Alkylphenyl oder
R⁷ und R⁸ bilden gemeinsam mit dem Stickstoff-Atom, an das sie gebunden sind, einen gesättigten oder ungesättigten 3-, 4-, 5-, 6- oder 7-gliedrigen Stickstoffheterocyclus, der gegebenenfalls ein oder zwei weitere Heteroatome, ausgewählt unter Stickstoff, Schwefel und Sauerstoff, als Ringglieder enthalten kann, der 1 oder 2 Carbonyl- und/ oder Thiocarbonylgruppen als Ringglied enthalten kann, und/oder durch ein, zwei oder drei Substituenten, ausgewählt unter C₁-C₄-Alkyl und Halogen, substituiert sein kann,
R⁹ Wasserstoff, Hydroxy, C₁-C₄-Alkyl, C₁-C₄-Alkoxy, Phenyl, Phenyl-C₁-C₄-alkyl, C₃-C₆-Alkenyl oder C₃-C₆-Alkinyl,
R¹⁰ Wasserstoff, C₁-C₄-Alkyl oder Amino,
R¹¹ C₁-C₄-Alkyl oder C₁-C₄-Halogenalkyl,
R¹² Wasserstoff oder C₁-C₄-Alkyl,
R¹³, R^{13'} unabhängig voneinander Wasserstoff oder C₁-C₄-Alkyl,
R¹⁴ Halogen,
R¹⁵ Wasserstoff oder C₁-C₄-Alkyl,
R¹⁶ C₁-C₄-Halogenalkyl, C₁-C₄-Alkylthio, C₁-C₄-Alkylsulfonyl oder C₁-C₄-Alkylsulfonyloxy,
R¹⁷ Wasserstoff oder C₁-C₄-Alkyl,
R¹⁸ Wasserstoff, C₁-C₄-Alkyl oder Amino,
R¹⁹ C₁-C₄-Halogenalkyl, C₁-C₄-Alkylthio oder C₁-C₄-Alkylsulfonyl,
R²⁰ Wasserstoff oder C₁-C₄-Alkyl,
R²¹ Wasserstoff, Halogen oder C₁-C₄-Alkyl,
R²² C₁-C₄-Alkyl, C₁-C₄-Halogenalkyl, C₁-C₄-Halogenalkoxy, C₁-C₄-Alkylthio oder C₁-C₄-Alkylsulfonyl,
R²³ Wasserstoff oder C₁-C₄-Alkyl,
oder
R²² und R²³ bilden gemeinsam mit den Atomen, an die sie gebunden sind, einen 5-, 6- oder 7-gliedrigen, gesättigten oder ungesättigten Ring, der ein Heteroatom, das ausgewählt ist unter Sauerstoff und Stickstoff, als ringbildendes Atom enthalten kann, und/oder der durch ein, zwei oder drei Reste, ausgewählt unter C₁-C₄-Alkyl und Halogen, substituiert sein kann,
R²⁴ Wasserstoff, C₁-C₄-Alkyl oder C₁-C₄-Halogenalkyl,
R²⁵ C₁-C₄-Alkyl oder C₁-C₄-Halogenalkyl,
oder
R²⁴ und R²⁵ bilden gemeinsam mit den Atomen, an die sie gebunden sind, einen 5-, 6- oder 7-gliedrigen, gesättigten oder ungesättigten Ring, der gegebenenfalls ein Sauerstoffatom als ringbildendes Atom enthält, und/oder der durch ein, zwei oder drei Reste, ausgewählt unter C₁-C₄₋Alkyl und Halogen, substituiert sein kann,
R²⁶ Wasserstoff, C₁-C₄-Alkyl oder C₁-C₄-Halogenalkyl,
R²⁷ Wasserstoff, C₁-C₄-Alkyl oder C₁-C₄-Halogenalkyl,
oder
R²⁶ und R²⁷ bilden gemeinsam mit den Atomen, an die sie gebunden sind, einen 5-, 6- oder 7-gliedrigen, gesättigten oder ungesättigten Ring, der gegebenenfalls ein Sauerstoffatom als ringbildendes Atom enthält, und/oder der durch ein, zwei oder drei Reste, ausgewählt unter C₁-C₄₋Alkyl und Halogen, substituiert sein kann,
A¹, A², A³, A⁴ jeweils unabhängig voneinander Sauerstoff oder Schwefel,
sowie deren landwirtschaftlich brauchbaren Salze.

2. Benzoesäure-Derivate nach Anspruch 1, worin R² für Fluor, Chlor oder Wasserstoff steht.

3. Benzoesäure-Derivate nach Anspruch 1 oder 2, worin R³ für Chlor oder Cyano steht.

4. Benzoesäure-Derivate nach einem der vorhergehenden Ansprüche, worin X für Sauerstoff steht.

5. Benzoesäure-Derivate nach einem der vorhergehenden Ansprüche, worin R⁶ für Wasserstoff steht.

6. Benzoesäure-Derivate nach einem der Ansprüche 1 bis 5, worin R¹ einen heterocyclischen Rest der Formel II-A bedeutet, in der R¹⁰ für C₁-C₄-Alkyl oder Amino, R¹¹ für C₁-C₄-Halogenalkyl und R¹² für Wasserstoff stehen.

7. Benzoesäure-Derivate nach einem der Ansprüche 1 bis 5, worin R¹ einen heterocyclischen Rest der Formel II-B bedeutet, in der R¹³ und R^{13'} jeweils unabhängig voneinander für C₁-C₄-Alkyl stehen.

8. Benzoesäure-Derivate nach einem der Ansprüche 1 bis 5, worin R¹ einen heterocyclischen Rest der Formel II-C bedeutet, in der R¹⁴ für Fluor oder Chlor, R¹⁵ für Wasserstoff und R¹⁶ für C₁-C₄-Halogenalkyl, C₁-C₄-Alkylsulfonyl oder C₁-C₄-Alkylsulfonyloxy stehen.

9. Benzoesäure-Derivate nach einem der Ansprüche 1 bis 5, worin R¹ einen heterocyclischen Rest der Formel II-D bedeutet, in der R¹⁸ für Wasserstoff, Methyl oder Amino, R¹⁹ für C₁-C₄-Halogenalkyl oder C₁-C₄-Alkylsulfonyl und R²⁰ für Wasserstoff stehen.

10. Benzoesäure-Derivate nach einem der Ansprüche 1 bis 5, worin R¹ einen heterocyclischen Rest der Formel II-E bedeutet, in der R²¹ für Halogen oder C₁-C₄-Alkyl, R²² für C₁-C₄-Halogenalkyl, C₁-C₄-Halogenalkoxy oder C₁-C₄-Alkylsulfonyl und R²³ für C₁-C₄-Alkyl stehen.

11. Benzoesäure-Derivate nach einem der Ansprüche 1 bis 5, worin R¹ einen heterocyclischen Rest der Formel II-F bedeutet, in der R²⁴ für Wasserstoff, Methyl, Difluormethyl oder Trifluormethyl, R²⁵ für Methyl oder Trifluormethyl oder R²⁴ zusammen mit R²⁵ für eine Kette der Formel -(CH₂)₄- stehen.

12. Benzoesäure-Derivate nach einem der Ansprüche 1 bis 5, worin R¹ einen heterocyclischen Rest der Formel II-G bedeutet, in der A¹ und A² jeweils für Sauerstoff stehen.

13. Benzoesäure-Derivate nach einem der Ansprüche 1 bis 5, worin R¹ einen heterocyclischen Rest der Formel II-H bedeutet, in der R²⁶ und R²⁷ jeweils unabhängig voneinander für C₁-C₄-Alkyl oder C₁-C₄-Halogenalkyl stehen oder R²⁶ zusammen mit R²⁷ für eine Kette der Formeln: -CH₂-O-(CH₂)₂- oder -(CH₂)₄- stehen.

14. Benzoesäure-Derivate nach einem der Ansprüche 1 bis 13,
worin
R² für Wasserstoff, Chlor oder Fluor steht,
R³ für Chlor oder Cyano steht,
R⁶ für Wasserstoff steht und
X für Sauerstoff steht.

15. Benzoesäure-Derivate nach einem der Ansprüche 1 bis 14, worin R⁴ oder R⁵ für Wasserstoff und der andere Rest R⁴ oder R⁵ für C₁-C₄-Alkyl oder R⁴, R⁵ jeweils für Methyl stehen.

16. Mittel, enthaltend eine herbizid wirksame Menge mindestens eines 3-Heterocyclyl substituierten Benzoesäure-Derivats der allgemeinen Formel I oder ein landwirtschaftlich brauchbares Salz von I gemäß einem der Ansprüche 1 bis 15, und mindestens einen inerten flüssigen und/oder festen Trägerstoff sowie gewünschtenfalls mindestens einen oberflächenaktiven Stoff.

17. Mittel zur Desikkation/Defoliation von Pflanzen, enthaltend eine desikkant/defoliant wirksame Menge mindestens eines 3-Heterocyclyl substituierten Benzoesäure-Derivats der allgemeinen Formel I oder ein landwirtschaftlich brauchbares Salz von I gemäß einem der Ansprüche 1 bis 15, und mindestens einen inerten flüssigen und/oder festen Trägerstoff sowie gewünschtenfalls mindestens einen oberflächenaktiven Stoff.

18. Verfahren zur Bekämpfung von unerwünschtem Pflanzenwuchs, **dadurch gekennzeichnet, dass** man eine herbizid wirksame Menge mindestens eines 3-Heterocyclyl substituierten Benzoesäure-Derivates der allgemeinen Formel I oder ein landwirtschaftlich brauchbares Salz von I gemäß einem der Ansprüche 1 bis 15, auf Pflanzen, deren Lebensraum und/oder auf Saatgut einwirken lässt.

19. Verfahren zur Desikkation/Defoliation von Pflanzen, **dadurch gekennzeichnet, dass** man eine desikkant/defoliant wirksame Menge mindestens eines 3-Heterocyclyl substituierten Benzoesäure-Derivates der allgemeinen Formel I oder ein landwirtschaftlich brauchbares Salz von I gemäß einem der Ansprüche 1 bis 15, auf Pflanzen einwirken lässt.

20. Verwendung von 3-Heterocyclyl substituierten Benzoesäure-Derivaten der allgemeinen Formel I oder deren landwirtschaftlich brauchbaren Salzen gemäß einem der Ansprüche 1 bis 15 als Herbizide oder zur Desikkation/Defoliation von Pflanzen.

## Claims

1. A 3-heterocyclyl-substituted benzoic acid derivative of the formula I 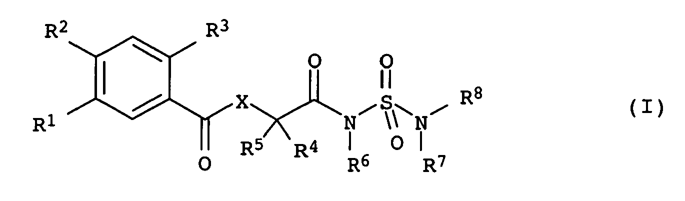 where:
X is oxygen or NR⁹,
R¹ is a heterocyclic radical of the formulae II-A to II-H, 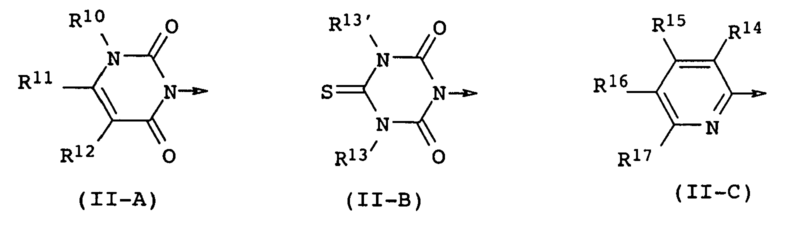 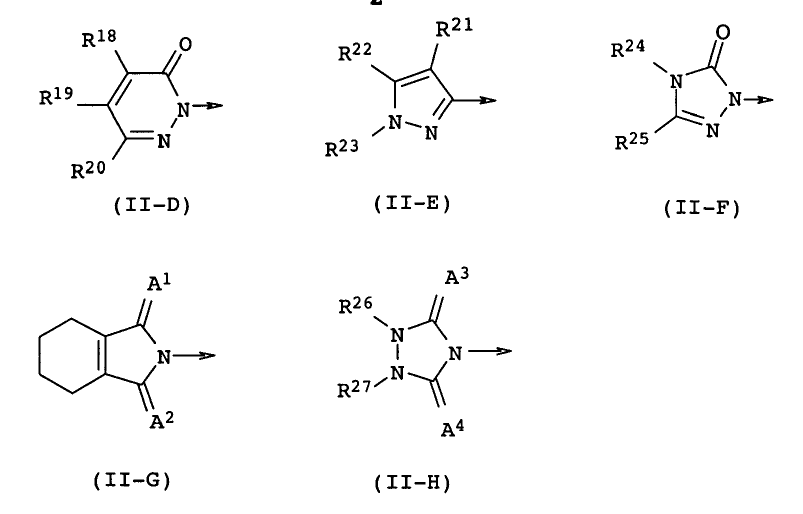
R² is hydrogen or halogen,
R³ is halogen or cyano,
R⁴, R⁵ independently of one another are hydrogen, C₁-C₄-alkyl or C₁-C₄-alkoxy, or R⁴ and R⁵ together are a group =CH₂,
R⁶ is hydrogen, C₁-C₄-alkyl or C₁-C₄-alkoxy,
R⁷, R⁸ independently of one another are hydrogen, C₁-C₆-alkyl, C₃-C₆-alkenyl, C₃-C₆-alkynyl, C₁-C₄-haloalkyl, C₁-C₄-alkoxy-C₁-C₄-alkyl, C₁-C₄-alkylthio-C₁-C₄-alkyl, C₁-C₄-alkylsulfinyl-C₁-C₄-alkyl, C₁-C₄-alkylsulfonyl-C₁-C₄-alkyl, cyano-C₁-C₄-alkyl, C₁-C₄-alkoxycarbonyl-C₁-C₄-alkyl, amino-C₁-C₄-alkyl, C₁-C₄-alkylamino-C₁-C₄-alkyl, di(C₁-C₄-alkyl)amino-C₁-C₄-alkyl, aminocarbonyl-C₁-C₄-alkyl, (C₁-C₄-alkylamino)carbonyl-C₁-C₄-alkyl, di(C₁-C₄-alkyl)aminocarbonyl-C₁-C₄-alkyl, phenyl or C₁-C₄-alkylphenyl or
R⁷ and R⁸ together with the nitrogen atom to which they are attached form a saturated or unsaturated 3-, 4-, 5-, 6- or 7-membered nitrogen heterocycle which may optionally contain one or two further heteroatoms selected from the group consisting of nitrogen, sulfur and oxygen as ring members, which may contain 1 or 2 carbonyl and/or thiocarbonyl groups as ring members, and/or may be substituted by one, two or three substituents selected from the group consisting of C₁-C₄-alkyl and halogen,
R⁹ is hydrogen, hydroxyl, C₁-C₄-alkyl, C₁-C₄-alkoxy, phenyl, phenyl-C₁-C₄-alkyl, C₃-C₆-alkenyl or C₃-C₆-alkynyl,
R¹⁰ is hydrogen, C₁-C₄-alkyl or amino,
R¹¹ is C₁-C₄-alkyl or C₁-C₄-haloalkyl,
R¹² is hydrogen or C₁-C₄-alkyl,
R¹³, R^{13'} independently of one another are hydrogen or C₁-C₄-alkyl,
R¹⁴ is halogen,
R¹⁵ is hydrogen or C₁-C₄-alkyl,
R¹⁶ is C₁-C₄-haloalkyl, C₁-C₄-alkylthio, C₁-C₄-alkylsulfonyl or C₁-C₄-alkylsulfonyloxy,
R¹⁷ is hydrogen or C₁-C₄-alkyl,
R¹⁸ is hydrogen, C₁-C₄-alkyl or amino,
R¹⁹ is C₁-C₄-haloalkyl, C₁-C₄-alkylthio or C₁-C₄-alkylsulfonyl,
R²⁰ is hydrogen or C₁-C₄-alkyl,
R²¹ is hydrogen, halogen or C₁-C₄-alkyl,
R²² is C₁-C₄-alkyl, C₁-C₄-haloalkyl, C₁-C₄-haloalkoxy, C₁-C₄-alkylthio or C₁-C₄-alkylsulfonyl,
R²³ is hydrogen or C₁-C₄-alkyl,
or
R²² and R²³ together with the atoms to which they are attached form a 5-, 6- or 7-membered saturated or unsaturated ring which may contain a heteroatom selected from the group consisting of oxygen and nitrogen as ring-forming atom and/or which may be substituted by one, two or three radicals selected from the group consisting of C₁-C₄-alkyl and halogen,
R²⁴ is hydrogen, C₁-C₄-alkyl or C₁-C₄-haloalkyl,
R²⁵ is C₁-C₄-alkyl or C₁-C₄-haloalkyl,
or
R²⁴ and R²⁵ together with the atoms to which they are attached form a 5-, 6- or 7-membered saturated or unsaturated ring which optionally contains an oxygen atom as ring-forming atom and/or which may be substituted by one, two or three radicals selected from the group consisting of C₁-C₄-alkyl and halogen,
R²⁶ is hydrogen, C₁-C₄-alkyl or C₁-C₄-haloalkyl,
R²⁷ is hydrogen, C₁-C₄-alkyl or C₁-C₄-haloalkyl,
or
R²⁶ and R²⁷ together with the atoms to which they are attached form a 5-, 6- or 7-membered saturated or unsaturated ring which optionally contains an oxygen atom as ring-forming atom and/or which may be substituted by one, two or three radicals selected from the group consisting of C₁-C₄-alkyl and halogen,
A¹, A², A³, A⁴ are each independently of one another oxygen or sulfur,
and its agriculturally useful salts.

2. A benzoic acid derivative according to claim 1 where R² is fluorine, chlorine or hydrogen.

3. A benzoic acid derivative according to claim 1 or 2 where R³ is chlorine or cyano.

4. A benzoic acid derivative according to any of the preceding claims where X is oxygen.

5. A benzoic acid derivative according to any of the preceding claims where R⁶ is hydrogen.

6. A benzoic acid derivative according to any of claims 1 to 5 where R¹ is a heterocyclic radical of the formula II-A in which R¹⁰ is C₁-C₄-alkyl or amino, R¹¹ is C₁-C₄-haloalkyl and R¹² is hydrogen.

7. A benzoic acid derivative according to any of claims 1 to 5 where R¹ is a heterocyclic radical of the formula II-B in which R¹³ and R¹³' are each independently of one another C₁-C₄-alkyl.

8. A benzoic acid derivative according to any of claims 1 to 5 where R¹ is a heterocyclic radical of the formula II-C in which R¹⁴ is fluorine or chlorine, R¹⁵ is hydrogen and R¹⁶ is C₁-C₄-haloalkyl, C₁-C₄-alkylsulfonyl or C₁-C₄-alkylsulfonyloxy.

9. A benzoic acid derivative according to any of claims 1 to 5 where R¹ is a heterocyclic radical of the formula II-D in which R¹⁸ is hydrogen, methyl or amino, R¹⁹ is C₁-C₄-haloalkyl or C₁-C₄-alkylsulfonyl and R²⁰ is hydrogen.

10. A benzoic acid derivative according to any of claims 1 to 5 where R¹ is a heterocyclic radical of the formula II-E in which R²¹ is halogen or C₁-C₄-alkyl, R²² is C₁-C₄-haloalkyl, C₁-C₄-haloalkoxy or C₁-C₄-alkylsulfonyl and R²³ is C₁-C₄-alkyl.

11. A benzoic acid derivative according to any of claims 1 to 5 where R¹ is a heterocyclic radical of the formula II-F in which R²⁴ is hydrogen, methyl, difluoromethyl or trifluoromethyl, R²⁵ is methyl or trifluoromethyl or R²⁴ together with R²⁵ are a chain of the formula -(CH₂)₄-.

12. A benzoic acid derivative according to any of claims 1 to 5 where R¹ is a heterocyclic radical of the formula II-G in which A¹ and A² are each oxygen.

13. A benzoic acid derivative according to any of claims 1 to 5 where R¹ is a heterocyclic radical of the formula II-H in which R²⁶ and R²⁷ are each independently of one another C₁-C₄-alkyl or C₁-C₄-haloalkyl or R²⁶ together with R²⁷ are a chain of the formulae: -CH₂-O-(CH₂)₂- or -(CH₂)₄-.

14. A benzoic acid derivative according to any of claims 1 to 13 where
R² is hydrogen, chlorine or fluorine,
R³ is chlorine or cyano,
R⁶ is hydrogen and
X is oxygen.

15. A benzoic acid derivative according to any of claims 1 to 14 where R⁴ or R⁵ is hydrogen and the other radical R⁴ or R⁵ is C₁-C₄-alkyl or R⁴, R⁵ are each methyl.

16. A composition comprising a herbicidally effective amount of at least one 3-heterocyclyl-substituted benzoic acid derivative of the formula I or an agriculturally useful salt of I according to any of claims 1 to 15 and at least one inert liquid and/or solid carrier and, if desired, at least one surfactant.

17. A composition for the desiccation/defoliation of plants, comprising an amount which is effective as a desiccant/defoliant of at least one 3-heterocyclyl-substituted benzoic acid derivative of the formula I or an agriculturally useful salt of I according to any of claims 1 to 15 and at least one inert liquid and/or solid carrier and, if desired, at least one surfactant.

18. A method for controlling unwanted vegetation, which comprises allowing a herbicidally effective amount of at least one 3-heterocyclyl-substituted benzoic acid derivative of the formula I or an agriculturally useful salt of I according to any of claims 1 to 15 to act on plants, their habitat and/or on seed.

19. A method for the desiccation/defoliation of plants, which comprises allowing an amount which is effective as a desiccant/defoliant of at least one 3-heterocyclyl-substituted benzoic acid derivative of the formula I or an agriculturally useful salt of I according to any of claims 1 to 15 to act on plants.

20. The use of 3-heterocyclyl-substituted benzoic acid derivatives of the formula I or their agriculturally useful salts according to any of claims 1 to 15 as herbicides or for the desiccation/defoliation of plants.

## Revendications

1. Dérivés d'acide benzoïque substitués par un groupe 3-hétérocyclyle de la formule générale I : 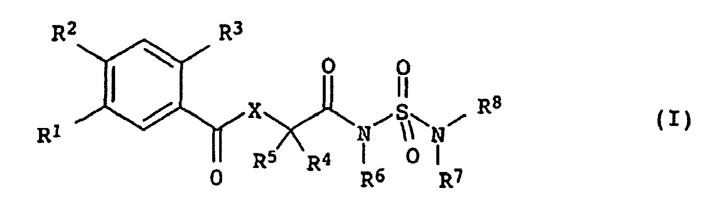 dans laquelle les variables ont les significations suivantes :
X représente de l'oxygène ou NR⁹,
R¹ représente un radical hétérocyclique des formules générales II-A à II-H : 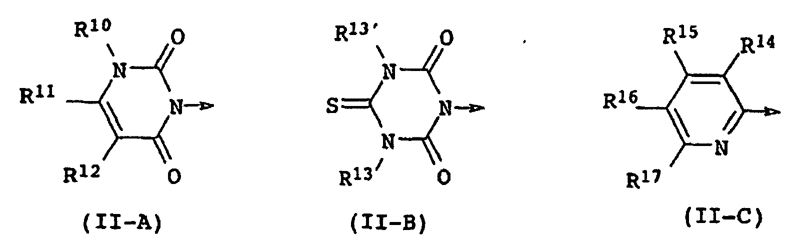 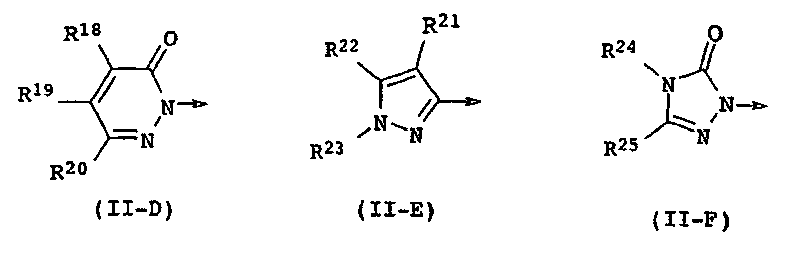 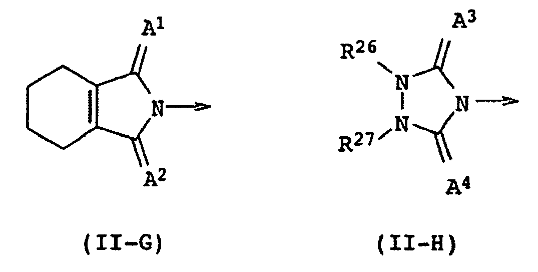
R² représente de l'hydrogène ou un halogène,
R³ représente un halogène ou du cyano,
R⁴, R⁵ représentent, indépendamment l'un de l'autre, de l'hydrogène ou un groupe C₁-C₄-alkyle ou C₁-C₄-alcoxy ou R⁴ et R⁵ représentent conjointement un groupe =CH₂,
R⁶ représente de l'hydrogène ou un groupe C₁-C₄-alkyle ou C₁-C₄-alcoxy,
R⁷ R⁸ représentent, indépendamment l'un de l'autre, de l'hydrogène ou un groupe C₁-C₆₋alkyle, C₃-C₆-alcényle, C₃-C₆-alcynyle, C₁-C₄₋halogénoalkyle, C₁-C₄-alcoxy-C₁-C₄-alkyle, C₁- C₄-alkylthio-C₁-C₄-alkyle, C₁-C₄-alkylsulfinyl-C₁-C₄-alkyle, C₁-C₄-alkylsulfonyl-C₁-C₄-alkyle, cyano-C₁-C₄-alkyle, C₁-C₄-alcoxycarbonyl-C₁-C₄₋alkyle, amino-C₁-C₄-alkyle, C₁-C₄-alkylamino-C₁-C₄-alkyle, di (C₁-C₄-alkyl) amino-C₁-C₄₋alkyle, aminocarbonyl-C₁-C₄-alkyle, (C₁-C₄₋alkylamino) carbonyl-C₁-C₄-alkyle, di (C₁-C₄₋alkyl)aminocarbonyl-C₁-C₄-alkyle, phényle ou C₁-C₄-alkylphényle ou
R⁷ et R⁸ forment conjointement avec l'atome d'azote sur lequel ils sont fixés un hétérocycle azoté saturé ou insaturé, à 3, 4, 5, 6 ou 7 termes, qui peut éventuellement contenir un ou deux autres hétéroatomes, choisis parmi de l'azote, du soufre et de l'oxygène, en tant que termes cycliques, qui peut contenir 1 ou 2 groupes carbonyle et/ou thiocarbonyle, comme termes cycliques, et/ou peut être substitué par un, deux ou trois substituants, choisis parmi un groupe C₁-C₄-alkyle et de l'halogène,
R⁹ représente de l'hydrogène ou un groupe hydroxy, C₁-C₄-alkyle, C₁-C₄-alcoxy, phényle, phényl-C₁-C₄-alkyle, C₃-C₆-alcényle ou C₃-C₆₋alcynyle,
R¹⁰ représente de l'hydrogène ou un groupe C₁-C₄₋alkyle ou amino,
R¹¹ représente un groupe C₁-C₄-alkyle ou C₁-C₄₋halogénoalkyle,
R¹² représente de l'hydrogène ou un groupe C₁-C₄₋alkyle,
R¹³ , R¹³ représentent, indépendamment l'un de l'autre, de l'hydrogène ou un groupe C₁-C₄₋alkyle,
R¹⁴ représente de l'halogène,
R¹⁵ représente de l'hydrogène ou un groupe C₁-C₄₋alkyle,
R¹⁶ représente un groupe C₁-C₄-halogénoalkyle, C₁₋C₄-alkylthio, C₁-C₄-alkylsulfonyle ou C₁-C₄₋alkylsulfonyloxy,
R¹⁷ représente de l'hydrogène ou un groupe C₁-C₄₋alkyle,
R¹⁸ représente de l'hydrogène ou un groupe C₁-C₄₋alkyle ou amino,
R¹⁹ représente un groupe C₁-C₄-halogénoalkyle, C₁₋C₄-alkylthio ou C₁-C₄-alkylsulfonyle,
R²⁰ représente de l'hydrogène ou un groupe C₁-C₄₋alkyle,
R²¹ représente de l'hydrogène, de l'halogène ou un groupe C₁-C₄-alkyle,
R²² représente un groupe C₁-C₄-alkyle, C₁-C₄₋halogénoalkyle, C₁-C₄-halogénoalcoxy, C₁-C₄₋alkylthio ou C₁-C₄-alkylsulfonyle,
R²³ représente de l'hydrogène ou un groupe C₁-C₄₋alkyle,
ou
R²² et R²³ forment conjointement avec les atomes sur lesquels ils sont fixés un noyau à 5, 6 ou 7 termes, saturé ou insaturé, qui peut contenir un hétéroatome choisi parmi de l'oxygène et de l'azote, comme atome cyclisant et/ou qui peut être substitué par un, deux ou trois radicaux, choisis parmi un groupe C₁-C₄-alkyle et de l'halogène,
R²⁴ représente de l'hydrogène ou un groupe C₁-C₄₋alkyle ou C₁-C₄-halogénoalkyle,
R²⁵ représente un groupe C₁-C₄-alkyle ou C₁-C₄₋halogénoalkyle,
ou
R²⁴ et R²⁵ forment conjointement avec les atomes sur lesquels ils sont fixés un noyau à 5, 6 ou 7 termes, saturé ou insaturé, qui contient éventuellement un atome d'oxygène comme atome cyclisant et/ou peut être substitué par un, deux ou trois radicaux choisis parmi un groupe C₁-C₄-alkyle et de l'halogène,
R²⁶ représente de l'hydrogène ou un groupe C₁-C₄₋alkyle ou C₁-C₄-halogénoalkyle,
R²⁷ représente de l'hydrogène ou un groupe C₁-C₄₋alkyle ou C₁-C₄-halogénoalkyle,
ou
R²⁶ et R²⁷ forment conjointement avec les atomes sur lesquels il sont fixés un noyau à 5, 6 ou 7 termes, saturé ou insaturé, qui contient éventuellement un atome d'oxygène comme atome cyclisant et/ou peut être substitué par un, deux ou trois radicaux choisis parmi un groupe C₁-C₄-alkyle et de l'halogène,
A¹, A², A³, A⁴ représentent chacun indépendamment l'un de l'autre de l'oxygène ou du soufre,
ainsi que leurs sels utilisables dans l'agriculture.

2. Dérivés d'acide benzoïque suivant la revendication 1, dans lesquels R² représente du fluor, du chlore ou de l'hydrogène.

3. Dérivés d'acide benzoïque suivant la revendication 1 ou 2, dans lesquels R³ représente du chlore ou du cyano.

4. Dérivés d'acide benzoïque suivant l'une des revendications précédentes, dans lesquels X représente de l'oxygène.

5. Dérivés d'acide benzoïque suivant l'une des revendications précédentes, dans lesquels R⁶ représente de l'hydrogène.

6. Dérivés d'acide benzoïque suivant l'une des revendications 1 à 5, dans lesquels R¹ représente un radical hétérocyclique de la formule II-A, dans laquelle R¹⁰ représente un groupe C₁-C₄-alkyle ou amino, R¹¹ un groupe C₁-C₄-halogénoalkyle et R¹² de l'hydrogène.

7. Dérivés d'acide benzoïque suivant l'une des revendications 1 à 5, dans lesquels R¹ représente un radical hétérocyclique de la formule II-B, dans laquelle R¹³ et R¹³' représentent chacun, indépendamment l'un de l'autre, un groupe C₁-C₄-alkyle.

8. Dérivés d'acide benzoïque suivant l'une des revendications 1 à 5, dans lesquels R¹ représente un radical hétérocyclique de la formule II-C, dans laquelle R¹⁴ représente du fluor ou du chlore, R¹⁵ de l'hydrogène et R¹⁶ un groupe C₁-C₄₋halogénoalkyle, C₁-C₄-alkylsulfonyle ou C₁-C₄₋alkylsulfonyloxy.

9. Dérivés d'acide benzoïque suivant l'une des revendications 1 à 5, dans lesquels R¹ représente un radical hétérocyclique de la formule II-D, dans laquelle R¹⁸ représente de l'hydrogène ou un groupe méthyle ou amino, R¹⁹ un groupe C₁-C₄₋halogénoalkyle ou C₁-C₄-alkylsulfonyle et R²⁰ de l'hydrogène.

10. Dérivés d'acide benzoïque suivant l'une des revendications 1 à 5, dans lesquels R¹ représente un radical hétérocyclique de la formule II-E, dans laquelle R²¹ représente de l'halogène ou un groupe C₁-C₄-alkyle, R²² un groupe C₁-C₄-halogénoalkyle, C₁₋C₄-halogénoalcoxy ou C₁-C₄-alkylsulfonyle et R²³ un groupe C₁-C₄-alkyle.

11. Dérivés d'acide benzoïque suivant l'une des revendications 1 à 5, dans lesquels R¹ représente un radical hétérocyclique de la formule II-F, dans laquelle R²⁴ représente de l'hydrogène ou un groupe méthyle, difluorométhyle ou trifluorométhyle, R²⁵ un groupe méthyle ou trifluorométhyle ou R²⁴ forme conjointement avec R²⁵ une chaîne de la formule -(CH₂)₄-.

12. Dérivés d'acide benzoïque suivant l'une des revendications 1 à 5, dans lesquels R¹ représente un radical hétérocyclique de la formule II-G, dans laquelle A¹ et A² représentent chacun de l'oxygène.

13. Dérivés d'acide benzoïque suivant l'une des revendications 1 à 5, dans lesquels R¹ représente un radical hétérocyclique de la formule II-H, dans laquelle R²⁶ et R²⁷ représentent chacun, indépendamment l'un de l'autre, un groupe C₁-C₄-alkyle ou C₁-C₄-halogénoalkyle ou R²⁶ forme conjointement avec R²⁷ une chaîne des formules -CH₂-O-(CH₂)₂- ou -(CH₂)₄- .

14. Dérivés d'acide benzoïque suivant l'une des revendications 1 à 13, dans lesquels
R² représente de l'hydrogène, du chlore ou du fluor,
R³ représente du chlore ou du cyano,
R⁶ représente de l'hydrogène, et
X représente de l'oxygène.

15. Dérivés d'acide benzoïque suivant l'une des revendications 1 à 14, dans lesquels R⁴ ou R⁵ représente de l'hydrogène et l'autre radical R⁴ ou R⁵ un groupe C₁-C₄-alkyle ou R⁴, R⁵ représentent chacun du méthyle.

16. Produit, contenant une quantité efficace du point de vue herbicide d'au moins un dérivé d'acide benzoïque substitué par un groupe 3-hétérocyclyle de la formule générale I ou un sel utilisable en agriculture de I suivant l'une des revendications 1 à 15, et au moins une matière de support inerte liquide et/ou solide ainsi qu'éventuellement au moins une matière tensioactive.

17. Produit de dessiccation/défoliation de plantes, contenant une quantité dessiccante/défoliante efficace d'au moins un dérivé d'acide benzoïque substitué par un groupe 3-hétérocyclyle de la formule générale I ou un sel utilisable en agriculture de I suivant l'une des revendications 1 à 15, et au moins une matière de support inerte liquide et/ou solide ainsi qu'éventuellement au moins une matière tensioactive.

18. Procédé pour lutter contre une croissance non désirée de plantes, **caractérisé en ce qu'**on fait agir sur des plantes, leur environnement et/ou des semences une quantité efficace du point de vue herbicide d'au moins un dérivé d'acide benzoïque substitué par un groupe 3-hétérocyclyle de la formule générale I ou un sel utilisable en agriculture de I suivant l'une des revendications 1 à 15.

19. Procédé de dessiccation/défoliation de plantes, **caractérisé en ce qu'**on fait agir sur des plantes une quantité dessiccante/défoliante efficace d'au moins un dérivé d'acide benzoïque substitué par un groupe 3-hétérocyclyle de la formule générale I ou un sel utilisable en agriculture de I suivant l'une des revendications 1 à 15.

20. Utilisation de dérivés d'acide benzoïque substitués par un groupe 3-hétérocyclyle de la formule générale I ou de leurs sels utilisables en agriculture suivant l'une des revendications 1 à 15, comme herbicides ou pour la dessiccation/défoliation de plantes.
